(19) Europäisches Patentamt
European Patent Office
Office européen des brevets

(11) EP 4 480 498 A1

(12) **EUROPEAN PATENT APPLICATION**
published in accordance with Art. 153(4) EPC

(43) Date of publication:
**25.12.2024 Bulletin 2024/52**

(21) Application number: **22927389.1**

(22) Date of filing: **17.02.2022**

(51) International Patent Classification (IPC):
***A61K 47/68*** *(2017.01)* ***A61K 38/05*** *(2006.01)*
***A61K 31/437*** *(2006.01)* ***A61P 35/00*** *(2006.01)*
***C07K 16/28*** *(2006.01)*

(52) Cooperative Patent Classification (CPC):
**A61K 31/437; A61K 38/05; A61K 47/68; A61P 35/00; C07K 16/28**

(86) International application number:
**PCT/KR2022/002358**

(87) International publication number:
**WO 2023/157989 (24.08.2023 Gazette 2023/34)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **NOVELTY NOBILITY Inc.**
**Gyeonggi-do, 13477 (KR)**

(72) Inventors:
- **PARK, Sang Gyu**
**Seongnam-si Gyeonggi-do 13477 (KR)**
- **KIM, Jin-Ock**
**Seongnam-si Gyeonggi-do 13477 (KR)**

(74) Representative: **SONN Patentanwälte GmbH & Co KG**
**Riemergasse 14**
**1010 Wien (AT)**

Remarks:
The complete document including Reference Table(s) and the Sequence Listing(s) can be downloaded from the EPO website

# (54) ANTIBODY-DRUG CONJUGATE

(57) The present invention relates to an antibody-drug conjugate targeting c-Kit and a use thereof.

EP 4 480 498 A1

Figure 5c

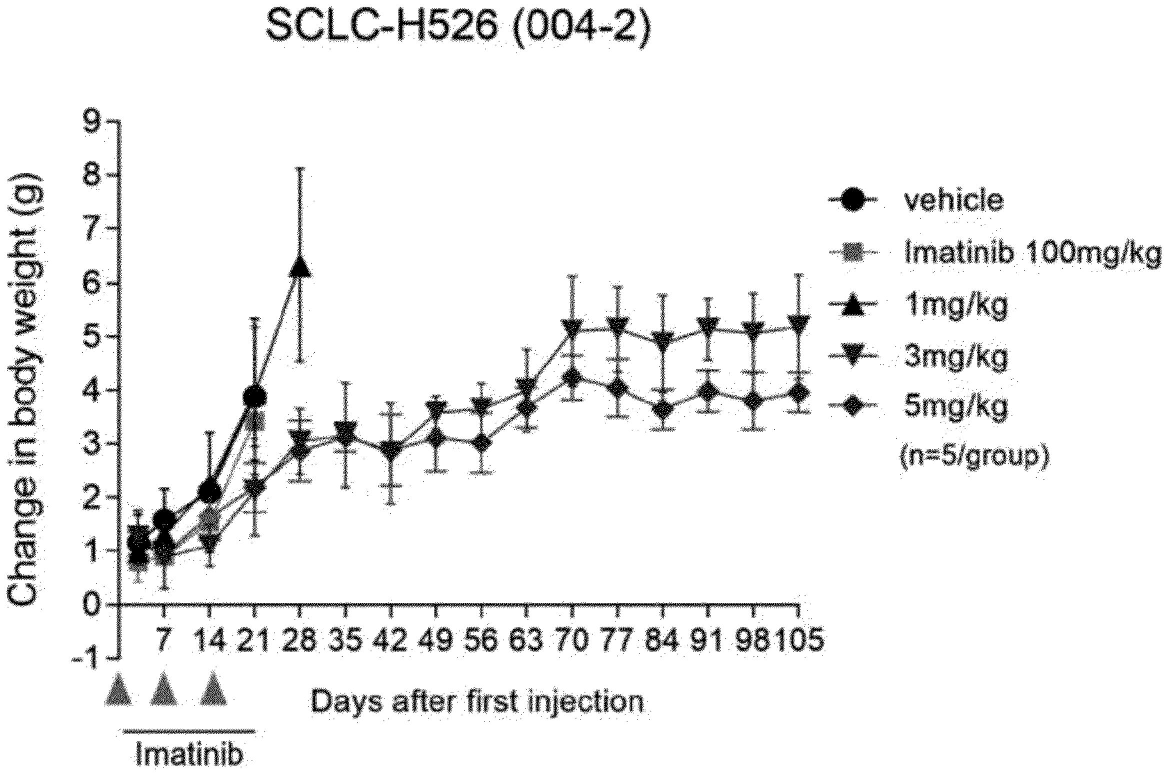
SCLC-H526 (004-2)
Change in body weight (g)
9
8
7
6
5
4
3
2
1
0
-1
7 14 21 28 35 42 49 56 63 70 77 84 91 98 105
Days after first injection
Imatinib
vehicle
Imatinib 100mg/kg
1mg/kg
3mg/kg
5mg/kg
(n=5/group)

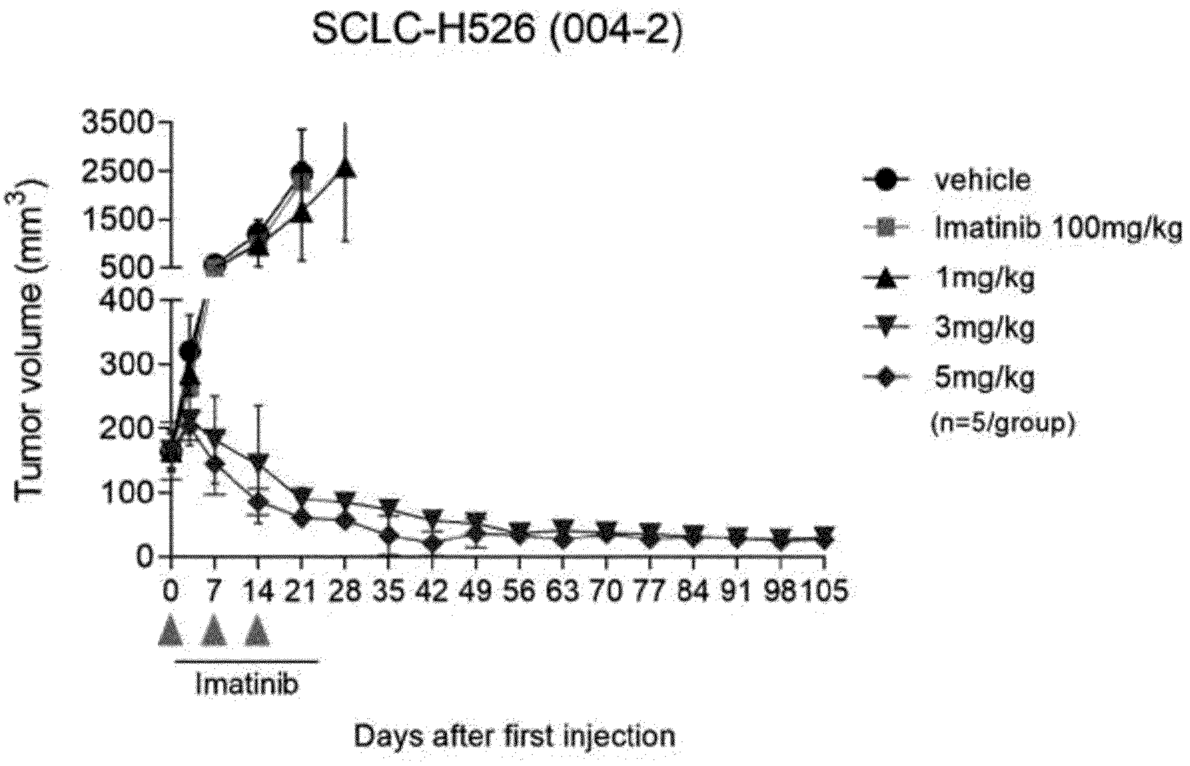
SCLC-H526 (004-2)
Tumor volume (mm3)
3500
2500
1500
500
400
300
200
100
0
0 7 14 21 28 35 42 49 56 63 70 77 84 91 98 105
Imatinib
Days after first injection
vehicle
Imatinib 100mg/kg
1mg/kg
3mg/kg
5mg/kg
(n=5/group)

## Description

### Technical Field

The present invention relates to an antibody-drug conjugate targeting c-Kit and a use thereof.

### Background Art

Binding of stem cell factor (SCF) to c-Kit (also known as CD117) activates several signaling pathways, including phosphoinositide 3 kinase, phospholipase C-gamma, Src kinase, Janus kinase-signal transducer and transcriptional activator (Yasuda A et al., Dig Dis Sci 52, 2292-2300. (2007); Sun J, Pedersen M & Ronnstrand L, J Biol Chem 284, 11039-11047. (2009)).

Previous studies have reported that expression of c-Kit with oncogenic mutations is dysregulated or upregulated in various cancers, resulting in SCF-independent c-Kit activation and cell proliferation. Overexpression of c-Kit is induced by hypoxia as well as activation of various transcription factors including AP-2, ETS, SP1, MYB, and MITF. Currently, more than 500 c-Kit mutations have been identified in human tumors (Sanger Institute Catalog of Somatic Mutations in Cancer, https://cancer.sanger.ac.uk). SCF-independent spontaneously activating c-Kit mutations are detected in approximately 85%, 30%, 25%, 25%, and 90% of gastrointestinal stromal tumor (GIST), acute myeloid leukemia, acral melanoma, testicular carcinoma, and systemic mastocytoma (SM) (Lennartsson J & Ronnstrand L, Physiol Rev 92, 1619-1649. (2012)).

The efficacy of small molecules such as imatinib to eliminate cancer stem cells harboring constitutively activated mutations of c-Kit is limited in tumor cells harboringmutations at different sites. In order to overcome c-Kit mutation-induced treatmentresistance, various small molecules such as dasatinib, sunitinib, and axitinib have been developed (Abbaspour Babaei M et al., Drug Des Devel Ther 10, 2443-2459. (2016)). However, the rate of grade 3/4 adverse reactions in patients treated with these small molecules is higher than in patients treated with imatinib, limiting their effective application in cancer treatment (Kantarjian HM et al., Blood 119, 1123-1129. (2012)).

Antibody-drug conjugates (ADCs) enable the delivery of potent cytotoxic agents by exploiting the specificity of a monoclonal antibody against cancer cells. Recently, various ADCs targeting CD30 (brentuximab vedotin), CD22 (inotuzumab ozogamicin), CD79b (polatuzumab vedotin), and HER2 (trastuzumab emtansine and trastuzumab deruxtecan) have been approved by the US Food and Drug Administration (FDA) (Leung D et al., Antibodies (Basel) 9, 2. (2020)). Since these ADCs are antibody-based drugs that bind to the extracellular domain of the target molecule they do not have mutations that mainly occur in the intracellular domain and have the relative advantage of being applicable regardless of various mutations.

## Detailed Description of Invention

### Technical Problem

An object of the present invention is to provide an antibody-drug conjugate of the following formula:

$$Ab(L_1)_m\text{-}(S)_n\text{-}(L_2)_p\text{-}(D)_q$$

in the formula,

Ab is an anti-c-Kit antibody or an antigen-binding fragment thereof that specifically binds to the epitope of human c-Kit in SEQ ID NO: 9 and SEQ ID NO: 10;
$L_1$ is a linker connecting Ab and S or Ab and $L_2$;
S is a spacer in a form in which a polymer is bound or not bound;
$L_2$ is a cleavable linker;
D is a drug moiety;
m is an integer from 0 to 8;
n is an integer from 0 to 8;
p is an integer from 1 to 8; and
q is an integer from 1 to 8.

Another object of the present invention is to provide an antibody-drug conjugate of the following formula:

$$Ab\text{-}(L)_x\text{-}(D)_y$$

in the formula,

Ab is an anti-c-Kit antibody or an antigen-binding fragment thereof that specifically binds to the epitope of human c-Kit in SEQ ID NO: 9 and SEQ ID NO: 10;
L is a linker comprising a cleavable linker;
D is a drug moiety;
x is an integer from 1 to 8; and
y is an integer from 1 to 8.

Another object of the present invention is to provide a composition comprising the antibody-drug conjugate.

Another object of the present invention is to provide a method for preventing or treating cancer, comprising administering a pharmaceutically effective amount of the composition to a subject in need thereof.

Another object of the present invention is to provide a use of the composition for use in therapy.

Another object of the present invention is to provide a pharmaceutical composition for preventing or treating cancer, comprising the antibody-drug conjugate.

Another object of the present invention is to provide a use of the pharmaceutical composition for manufacture of a medicament.

Another object of the present invention is to provide a composition for diagnosing cancer, comprising the antibody-drug conjugate.

Another object of the present invention is to provide a method of providing information for the diagnosis of cancer, comprising: treating a sample isolated from a subject with the antibody-drug conjugate.

Another object of the present invention is to provide a method for producing an anti-c-Kit antibody-drug conjugate, comprising: conjugating $(L_1)_m$-$(S)_n$-$(L_2)_p$-$(D)_q$ to an antibody or an antigen-binding fragment thereof (Ab) that specifically binds to human c-Kit:
in the formula,

$L_1$ is a linker connecting Ab and S or Ab and $L_2$;
S is a spacer in a form in which a polymer is bound or not bound;
$L_2$ is a cleavable linker;
D is a drug moiety;
m is an integer from 0 to 8;
n is an integer from 0 to 8;
p is an integer from 1 to 8; and
q is an integer from 1 to 8.

Another object of the present invention is to provide a method for producing an anti-c-Kit antibody-drug conjugate, comprising: conjugating a linker (L)-drug (D) to an antibody or an antigen-binding fragment thereof comprising the heavy chain CDR1 of SEQ ID NO: 1, the heavy chain CDR2 of SEQ ID NO: 2, the heavy chain CDR3 of SEQ ID NO: 3, the light chain CDR1 of SEQ ID NO: 4, the light chain CDR2 of SEQ ID NO: 5, and the light chain CDR3 of SEQ ID NO: 6, wherein L is a linker comprising a cleavable linker, and D is a drug moiety.

Other objects and advantages of the present invention will become more apparent from the detailed description of the invention, the claims and the drawings which follow.

## Solution to Problem

The present invention relates to an antibody-drug conjugate and a use thereof. The present invention demonstrates that cancer may be controlled, treated, alleviated, or reduced, or recurrence of cancer may be inhibited, by administration of an antibody-drug conjugate comprising, for example, an antibody or an antigen-binding fragment thereof that specifically binds to human c-Kit.

In order to facilitate understanding of the present invention, a number of terms and phrases are defined. Additional definitions are presented throughout the detailed description.

### I. Definition

As used herein, the term "about" is used to mean approximately, roughly, around, or within the region of .... When the term "about" is used with a numerical range, it modifies that range by extending the boundaries above and below the numerical value presented. In general, the term "about" may modify a numerical value, for example up or down (higher or

lower), up to a change of 10 percent, above or below the stated value.

In addition, "and/or" should be construed to mean that each of the two specified features or ingredients is specifically disclosed either in conjunction with the other or alone. Therefore, the term "and/or" as used in phrases such as "A and/or B" is intended to include "A and B", "A or B", "A" (alone), and "B" (alone). Likewise, the term "and/or" used in phrases such as "A, B and/or C" is intended to include aspects of A, B and C; A, B or C; A or C; A or B; B or C; A and C; A and B; B and C; A (alone); B (alone); and C (alone), respectively.

As used herein, the terms "treat," "treating," and "treatment" refer to any kind of intervention or process performed on a subject, or the administration of an active agent to a subject for the purpose of reversing, ameliorating, alleviating, inhibiting or delaying the progression, occurrence, severity or recurrence of symptoms, complications, conditions or biochemical indicators associated with a disease. Treatment may be directed to a subject with the disease or to a subject without the disease (for example, prophylaxis).

As used herein, the term "administration" refers to the physical introduction of a therapeutic agent or a composition comprising a therapeutic agent into a subject using any of a variety of methods and delivery systems known to those of ordinary skill in the art. Different routes of administration for the antibody-drug conjugate disclosed herein include intravenous, intraperitoneal, intramuscular, subcutaneous, spinal, or other parenteral routes of administration, for example, by injection or infusion. As used herein, the term "parenteral administration" generally refers to modes of administration other than enteral and topical administration by injection, including, but not limited to, intravenous, intraperitoneal, intramuscular, intraarterial, intrathecal, intralymphatic, intralesional, intracapsular, intraorbital, intracardiac, intradermal, transtracheal, intratracheal, pulmonary, subcutaneous, subepithelial, intraarticular, subcapsular, subarachnoid, intraventricular, intravitreal, epidural, and substernal injections and infusions, as well as *in vivo* electroporation. Alternatively, the antibody-drug conjugate disclosed herein may be administered via a parenteral route, such as via a topical, epithelial or mucosal route of administration, such as via an intranasal, oral, vaginal, rectal, sublingual or topical route. In addition, administration may be performed over an extended period of time, for example, once, multiple times, and/or more than once.

As used herein, the term "therapeutically effective amount" refers to an amount of a drug that is effective, alone or in combination with other therapeutic agents, "to treat" a disease or disorder in a subject or to reduce the risk, potential, likelihood or occurrence of the disease or disorder (for example, cancer). A "therapeutically effective amount" includes an amount of a drug or therapeutic agent that provides some improvement or benefit to a subject having or at risk of having a disease or disorder (for example, cancer). Therefore, a "therapeutically effective amount" is an amount that reduces the risk, potential, likelihood or occurrence of a disease or disorder, or provides some alleviation and mitigation, and/or reduces at least one indicator (for example, cancer), and/or reduces at least one clinical symptom of the disease or disorder.

As used herein, the term "cancer" refers to a broad group of various diseases characterized by the uncontrolled growth of abnormal cells in the body. "Cancer" or "cancer tissue" may include a tumor. Uncontrolled cell division and growth leads to the formation of malignant tumors that invade neighboring tissues and may also be metastasized to distant parts of the body through the lymphatic system or bloodstream. After metastasis, distal tumors may be said to be "derived from" the pre-metastatic tumor. For example, a tumor "derived from melanoma" refers to a tumor that is the result of metastatic melanoma. Since a distant tumor is derived from a pre-metastatic tumor, The phrase "derived from" a tumor may also include a pre-metastatic tumor, for example, a tumor derived from a melanoma may include a melanoma.

As used herein, the phrase "inhibits tumor growth" includes any measurable reduction in tumor growth, for example, inhibition of tumor growth by at least about 10%, at least about 20%, at least about 30%, at least about 40%, at least about 50%, at least about 60%, at least about 70%, at least about 80%, at least about 90%, at least about 99%, or up to 100%.

The term "effective amount", "pharmaceutically effective amount" or "effective dosage" are defined as an amount sufficient to achieve, or at least partially achieve, the desired effect. A "therapeutically effective amount" or "therapeutically effective dosage" of a drug or therapeutic agent, when used alone or in combination with other therapeutic agents, refers to an amount of a drug that promotes disease regression as evidenced by a reduction in the severity of disease symptoms, an increase in the frequency and duration of disease symptom-free periods, or the prevention of disability or injury due to the disease. A therapeutically effective amount or effective dosage of a drug includes a "prophylactically effective amount" or "prophylactically effective dosage", which refers to an amount of a drug that inhibits the development or recurrence of a disease when administered alone or in combination with other therapeutic agents to a subject at risk of developing the disease or suffering a recurrence of the disease. The efficacy of a therapeutic agent to promote the regression of a disease or to inhibit the development or recurrence of a disease may be assessed using a variety of methods known to those of ordinary skill in the art, and may be assessed, for example, by analyzing the activity of the agent in human subjects during clinical trials, in animal model systems predicting efficacy in humans, or in *in vitro* assays.

As an example, an anticancer agent is a drug that promotes cancer regression in a subject. In some embodiments, a therapeutically effective amount of the drug promotes cancer regression until the cancer is eliminated. "Promotion of cancer regression" means that administering a pharmaceutically effective amount of a drug, alone or in

combination with an antineoplastic agent, results in a decrease in growth or size of a tumor, necrosis of a tumor, a decrease in the severity of at least one disease symptom, an increase in the frequency and duration of disease symptom-free periods, prevention of disability or injury due to the disease, or other improvement in the patient's disease symptoms. In addition, the terms "effective" and "effectiveness" include both pharmacological effectiveness and physiological safety in the context of treatment. Pharmacological effectiveness refers to the efficacy of a drug that may promote the regression of a patient's cancer. Physiological safety refers to the level of toxicity or other negative physiological effects (side effects) at the cellular, organ and/or organism level that result from administration of a drug.

As an example, in the case of tumor treatment, a therapeutically effective amount or therapeutically effective dosage of a drug inhibits cell growth or tumor growth by at least about 20%, at least about 40%, at least about 60%, or at least about 80% compared to an untreated subject. In some embodiments, a therapeutically effective amount or therapeutically effective dosage of a drug completely inhibits cell growth or tumor growth, i.e., inhibits cell growth or tumor growth by up to 100%. The ability of an inhibitor to inhibit tumor growth may be assessed using the assay described below. Alternatively, this property of the composition may be assessed by testing the ability of the inhibitor to inhibit cell growth, and such inhibition may be measured *in vitro* by assays known to those of ordinary skill in the art. In other embodiments disclosed herein, tumor regression may be observed and may last for at least about 20 days, at least about 40 days, or at least about 60 days.

Some embodiments of the present invention relate to diagnosing cancer in a subject.

As used herein, the term "diagnosis" refers to a method that may be used to determine or predict whether a patient is suffering from a given disease or condition. Those of ordinary skill in the art may make a diagnosis based on one or more diagnostic markers (for example, expression level of c-Kit), wherein the presence, absence, amount, or change in the amount of the diagnostic marker indicates the presence, severity, or absence of the condition. In some embodiments, increased expression of c-Kit in a biological sample from a subject is indicative of a tumor. The term "diagnosis" does not refer to the ability to determine the presence or absence of a particular disease with 100% accuracy, or even that a given course or outcome is more likely to occur than not. Instead, those of ordinary skill in the art will understand the term "diagnosis" to refer to an increased probability that a particular disease is present in a subject.

The term "diagnostic marker" (for example, expression of c-Kit) refers to a substance that may be used to separate and diagnose tumors from normal cells, and includes organic biomolecules such as polypeptides, or nucleic acids (for example, mRNA), lipids, glycolipids, glycoproteins, and sugars (monosaccharides, disaccharides, oligosaccharides, etc.), which are increased or decreased in the tumor cells. The diagnostic marker disclosed herein for cancer may be a protein expressed from the c-Kit gene whose expression is increased in tumor cells.

A composition for diagnosing cancer includes an agent for measuring the expression level of mRNA of the c-Kit gene or the amount of expressed protein. These agents include an oligonucleotide having a sequence complementary to c-Kit mRNA, a primer or nucleic acid probe that specifically binds to c-Kit mRNA, and an antibody or an antigen-binding fragment thereof (hereinafter referred to as an anti-c-Kit antibody) that specifically binds to c-Kit protein, or an antibody-drug conjugate in which an anti-c-Kit antibody is linked to a drug by a linker.

As used herein, the term "subject" includes any human or non-human animal. The term "non-human animal" includes all vertebrates, including mammals and non-mammals, such as non-human primates, sheep, dogs, cattle, chickens, amphibians, reptiles, and the like.

The term "c-Kit" belongs to class III of receptor tyrosine kinase (RTK) and is also known as the receptor for SCF.

"c-Kit" includes any variant or isoform of c-Kit that is naturally expressed by a cell. Therefore, the anti-c-Kit antibody or antibody-drug conjugate disclosed herein may cross-react with different isoforms of the same species (for example, different isoforms of human c-Kit) or may cross-react with c-Kit from a species other than human (for example, mouse c-Kit). Alternatively, the anti-c-Kit antibody may be specific for human c-Kit and may not show cross-reactivity with other species. c-Kit, or any variants and isoforms thereof, may be isolated from cells or tissues that naturally express them or may be produced recombinantly. The sequence of domains 1 to 3 (Q26 to D309) of human c-Kit, excluding 25 amino acids of signal peptide, may be SEQ ID NO: 12.

The term "antibody" is a term in the art and may be used interchangeably herein, and refers to a molecule that has an antigen binding site that specifically binds to an antigen. As used herein, the term includes a whole antibody and any antigen-binding fragment (i.e., "antigen-binding portion") or a single chain thereof. In one embodiment, an "antibody" refers to a glycoprotein, or an antigen-binding portion thereof, comprising at least two heavy (H) and two light (L) chains interconnected by disulfide bonds. In other embodiments, an "antibody" refers to a single chain antibody comprising a single variable domain, such as a VHH domain. Each heavy chain consists of a heavy chain variable region (abbreviated as VH) and a heavy chain constant region. In certain naturally-occurring antibodies, the heavy chain constant region consists of three domains CH1, CH2, and CH3. In certain naturally-occurring antibodies, each light chain consists of a light chain variable region (abbreviated as VL) and a light chain constant region. The light chain constant region consists of one domain CL.

The VH and VL regions may be further subdivided into hypervariable regions, referred to as complementarity determining regions (CDR), interspersed with more conserved regions, referred to as framework regions (FR). Each VH

and VL consists of three CDRs and four FRs, which are arranged from amino-terminus to carboxy-terminus in the following order: FR1, CDR1, FR2, CDR2, FR3, CDR3, and FR4. The variable regions of the heavy and light chains contain binding domains that interact with antigen. The constant region of the antibody may mediate the binding of the immunoglobulin to host tissues or factors, including various cells of the immune system (for example, effector cells) and the first component of the classical complement system (C1q).

Antibodies may be of any type (for example, IgG, IgE, IgM, IgD, IgA, or IgY), any type (for example, IgD, IgG2, IgG3, IgG4, IgA1, or IgA2), or any subtype (for example, IgG1, IgG2, IgG3, and IgG4 in humans; and IgG1, IgG2a, IgG2b, and IgG3 in mice) of immunoglobulin molecule. Immunoglobulins, such as IgG1, exist in several allotypes, which differ from each other by up to several amino acids. The antibodies disclosed herein may be derived from any of the commonly known isotypes, types, subtypes, or allotypes. In certain embodiments, the antibodies disclosed herein are of the IgG1, IgG2, IgG3, or IgG4 subtype or any hybrid thereof. In certain embodiments, the antibodies are of the human IgG1 subtype or the human IgG2 or human IgG4 subtype.

As an example, an "antibody" includes naturally-occurring and non-naturally-occurring antibodies; monoclonal and polyclonal antibodies; chimeric and humanized antibodies; human and non-human antibodies, fully synthetic antibodies; single chain antibodies; monospecific antibodies; multispecific antibodies (including bispecific antibodies); tetrameric antibodies comprising two heavy and two light chain molecules; antibody light chain monomer; antibody heavy chain monomer; antibody light chain dimer; antibody heavy chain dimer; antibody light chain-antibody heavy chain pair; intrabodies; heteroconjugate antibodies; monovalent antibodies; camelized antibodies; affibodies; anti-idiotypic (anti-Id) antibodies (including, for example, anti-anti-Id antibodies); a single-domain antibody (sdAb) comprising a binding molecule consisting of a single monomeric variable antibody domain (for example, a VH domain or a VL domain) sufficiently capable of binding to an antigen (Harmen M. M. and Haard H. J. Appl Microbiol Biotechnol. 77(1): 13-22 (2007)).

As used herein, the term "antigen-binding portion" of an antibody, i.e., "antigen-binding fragment," refers to one or more fragments of an antibody that possesses the ability to specifically bind to an antigen (for example, domain 2/3 of human c-Kit). These "fragments" may be, for example, from about 8 to about 1500 amino acids in length, suitably from about 8 to about 745 amino acids in length, more suitably from about 8 to about 300 amino acids in length, for example from about 8 to about 200 amino acids in length, or from about 10 to about 50 or 100 amino acids in length. It has been found that the antigen-binding function of an antibody may be performed by fragments of the full-length antibody. Examples of binding fragments encompassed by the terms "antigen-binding portion" or "antigen-binding fragment" of an antibody, for example, an anti-c-Kit antibody disclosed herein, include, but are not limited to: (i) a Fab fragment, which is a monovalent fragment consisting of VL, VH, CL, and CH1 domains; (ii) a F(ab')2 fragment, which is a bivalent fragment comprising two Fab fragments connected by a disulfide bridge at a hinge region; (iii) a Fd fragment, which is composed of VH and CH1 domains; (iv) a Fv fragment, which is composed of VL and VH domains of a single arm of an antibody, and a disulfide-linked Fv (sdFv); (v) a dAb fragment, which is composed of a VH domain (Ward et al., (1989) Nature 341: 544-546); and (vi) a separate complementarity determining region (CDR); or (vii) a combination of two or more separate CDRs that may be optionally connected by a synthetic linker. In addition, although the two domains, VL and VH, are encoded by separate genes, they may be joined by synthetic linkers and using recombinant methods, whereby the VL and VH regions are paired to form a single protein chain to form a monovalent molecule (referred to as a single chain Fv(scFv)) (see, for example, Bird et al., (1988) Science 242:423-426; and Huston et al., (1988) Proc. Natl. Acad. Sci. USA 85:5879-5883). Such single chain antibodies are also included within the term "antigen-binding portion" or "antigen-binding fragment" of an antibody. These antibody fragments are obtained using conventional techniques known to those of ordinary skill in the art, and the fragments are screened for utility in the same manner as intact antibodies. The antigen-binding portion may be produced by recombinant DNA techniques, or by enzymatic or chemical cleavage of intact immunoglobulins.

As used herein, the term "variable region" or "variable domain" is commonly used interchangeably in the art. A variable region typically refers to a portion of an antibody, generally a portion of the light chain or heavy chain, typically about the amino-terminal 110 to 120 amino acids in the mature heavy chain and about 90 to 115 amino acids in the mature light chain, which differ widely in sequence from antibodies and are used because of the binding and specificity of specific antibodies for their specific antigens. Sequence variability is concentrated in regions referred to as a complementarity determining region (CDR), while the more highly conserved region in the variable domain is referred to as a framework region (FR).

The CDRs of the light and heavy chains are believed to be primarily responsible for the interaction and specificity of the antibody with the antigen. In certain embodiments, the variable region is a human variable region. In certain embodiments, the variable region includes a rodent or murine CDR and a human framework region (FR). In certain embodiments, the variable region is a primate (for example, non-human primate) variable region. In certain embodiments, the variable region includes a rodent or murine CDR and a primate (for example, non-human primate) framework region (FR).

As used herein, the term "heavy chain," when used in reference to an antibody, may be of any of the different types, for example, alpha ($\alpha$), delta ($\delta$), epsilon ($\varepsilon$), gamma ($\gamma$) and mu ($\mu$), based on the amino acid sequence of the

constant domains, which give rise to the IgA, IgD, IgE, IgG, and IgM types of antibodies, including subtypes of IgG, such as IgG1, IgG2, IgG3, and IgG4, respectively.

As used herein, the term "light chain," when used in reference to an antibody, may refer to any of the different types, such as kappa (κ) and lambda (λ), based on the amino acid sequence of the constant domains. An amino acid sequence of the light chain are well known in the art. In certain embodiments, the light chain is a human light chain.

The terms "VL" and "VL domain" are used interchangeably and refer to the light chain variable region of an antibody.

The terms "VH" and "VH domain" are used interchangeably and refer to the heavy chain variable region of an antibody.

As used herein, the terms "constant region" and "constant domain" are used interchangeably and have their ordinary meaning in the art. The constant domain is the portion of the antibody, for example, the carboxy terminal portion of the light and/or heavy chain that is not directly involved in the binding of the antibody to antigen but may exhibit various effector functions, such as interaction with Fc receptors. The constant regions of immunoglobulin molecules generally have more conserved amino acid sequences than the immunoglobulin variable domains.

"Fc region" (fragment crystallizable region) or "Fc domain" or "Fc" refers to the C-terminal region of the heavy chain of an antibody that mediates the binding of the immunoglobulin to host tissues or factors, including the binding of the Fc receptor located on various cells of the immune system (for example, effector cells) and the first component of the classical complement system (C1q). Therefore, the Fc region comprises the constant region of the antibody excluding the first constant region immunoglobulin domain (for example, CH1 or CL). In the IgG, IgA and IgD antibody isotypes, the Fc region comprises two identical protein fragments derived from the second (CH2) and third (CH3) constant domains of the two heavy chains of the antibody. The IgM and IgE Fc regions comprise three heavy chain constant domains (CH domains 2 to 4) in each polypeptide chain. For IgG, the Fc region comprises the hinge between the immunoglobulin domains Cγ2 and Cγ3 and between Cγ1 and Cγ2. Although the boundaries of the Fc region of immunoglobulin heavy chains vary, the human IgG heavy chain Fc region is generally defined as extending from the amino acid residue at position C226 or P230 (or the amino acid between these two amino acids) to the carboxy-terminus of the heavy chain, where numbering follows the EU index as in Kabat. The CH2 domain of the human IgG Fc region extends from about amino acid 231 to about amino acid 340, and the CH3 domain is located on the C-terminal side of the Cm domain in the Fc region, i.e. it extends from about amino acid 341 to about amino acid 447 of the IgG. The Fc region may be a naturally-occurring sequence Fc, including any allotype variant, or a variant Fc (for example, a non-naturally-occurring Fc). In addition, Fc refers to the region in an isolated status, or refers to the region associated with an Fc-containing protein polypeptide, such as a "binding protein containing an Fc region", also referred to as an "Fc fusion protein" (for example, an antibody or immunoadhesion).

The "hinge", "hinge domain" or "hinge region" or "antibody hinge region" refers to the domain of the heavy chain constant region that connects the CH1 domain to the CH2 domain and includes the upper, middle, and lower portions of the hinge (Roux et al. J. Immunol. 1998 161:4083). The hinge changes the level of flexibility between the binding region and the effector region of the antibody, and also provides a site for intermolecular disulfide bonds between the two heavy chain constant regions. The sequences of wild type IgG1, IgG2, IgG3, and IgG4 hinges are known in the art (see, for example, Kabat EA et al., (1991) Sequences of Proteins of Immunological Interest, Fifth Edition, U.S. Department of Health and Human Services, NIH Publication No. 91-3242; Vidarsson G. et al., Front Immunol. 5:520 (published online October 20, 2014)).

As used herein, the term "isotype" refers to the antibody type (for example, IgG1, IgG2, IgG3, IgG4, IgM, IgA1, IgA2, IgD, and IgE antibodies) encoded by the heavy chain constant region genes.

The term "allotype" refers to a naturally-occurring variant within a specific isotype group that differs by several amino acids (see, for example, Jefferis et al. (2009) mAbs 1:1). The antibody presented herein may have any allotype. Allotypes of IgG1, IgG2, IgG3, and IgG4 are known in the art (see, for example, Kabat EA et al., (1991) (ibid.); Vidarsson G. et al., Front Immunol. 5:520 (published online October 20, 2014); and Lefranc MP, mAbs 1:4, 1-7(2009)).

The terms "antibody that recognizes an antigen" and "antibody specific for an antigen" are used interchangeably herein with the term "antibody that specifically binds to an antigen."

As used herein, the term "isolated antibody" refers to an antibody that is substantially free of other antibodies with a different antigenic specificity (for example, an isolated antibody that specifically binds to c-Kit is substantially free of an antibody that specifically binds to an antigen other than c-Kit). However, an isolated antibody that specifically binds to an epitope of c-Kit may have cross-reactivity to other c-Kit proteins from different species.

The term "binding affinity" generally refers to the overall combined strength of non-covalent interactions between a single binding site on a molecule (for example, an antibody) and its binding partner (for example, an antigen). Unless otherwise stated, as used herein, the term "binding affinity" refers to the intrinsic binding affinity that reflects a 1:1 interaction between members of a binding pair (for example, antibody and antigen). The affinity of molecule X for partner Y may generally be expressed by the dissociation constant ($k_d$). The affinity may be measured and/or expressed in a number of ways known in the art, including, but not limited to, equilibrium dissociation constant ($K_D$), and equilibrium association constant ($k_a$). $K_D$ is calculated from the quotient of koff/kon and expressed in molar concentration (M), and KA is calculated

from the quotient of kon/koff. The kon, for example, refers to the association rate constant of an antibody to an antigen, and the koff, for example, refers to the dissociation rate constant of an antibody to an antigen. The kon and koff may be determined by techniques known to those of ordinary skill in the art, such as immunoassays (for example, enzyme-linked immunosorbent assay (ELISA)), BIACORE® or kinetic exclusion assays (KinExA®).

As used herein, the terms "specifically binds", "specifically recognizes", "specific binding", "selective binding," and "selectively binds" are similar terms in relation to an antibody, and are terms relating to a molecule (for example, an antibody) that binds to an antigen (for example, an epitope or an immune complex), wherein such binding is understood by those of ordinary skill in the art. For example, a molecule that specifically binds to an antigen, as determined, for example, by immunoassay, BIACORE®, KinExA® 3000 instrument (Sapidyne Instruments, Boise, ID), or other assays known in the art, may generally bind other peptides or polypeptides with lower affinity.

An antibody specifically binds to its cognate antigen with high affinity, reflected by a dissociation constant of typically $10^{-5}$ to $10^{-11}$ M or less. A $K_D$ exceeding about $10^{-4}$ M is generally considered to indicate nonspecific binding. An antibody that "specifically binds" to an antigen is one that binds with high affinity to the antigen and to an antigen that is substantially identical to the antigen, and is meant to have a $K_D$ of $10^{-7}$ M or less, preferably $10^{-8}$ M or less, more preferably $10^{-9}$ M or less, even more preferably $10^{-10}$ M or less, or $10^{-11}$ M or less, as determined, for example, by immunoassay (for example, ELISA) using a defined antigen or by surface plasmon resonance (SPR) technology on a BIACORE® 2000 instrument, and it does not bind to unrelated antigens with high affinity.

As used herein, the term "antigen" refers to any natural or synthetic immunogenic substance, such as a protein, peptide, or hapten. The antigen may be c-Kit or a fragment thereof.

As used herein, the term "epitope" is a term in the art and refers to a localized region of an antigen to which an antibody may specifically bind. An epitope may be, for example, contiguous amino acids of a polypeptide (a linear or continuous epitope), or an epitope may be, for example, a combination of two or more non-contiguous regions of a polypeptide or polypeptides (a conformational, non-linear, discontinuous, or non-contiguous epitope). An epitope formed from contiguous amino acids is typically, but not always, retained upon exposure to denaturing solvents, and an epitope formed by tertiary folding is typically lost upon treatment with denaturing solvents. An epitope typically contains at least 1, 2, 3, 4, 5, 6, 7, 8, 9, 10, 11, 12, 13, 14, 15, or 20 amino acids in unique spatial conformations. Methods for determining whether an epitope is bound by a given antibody (i.e., epitope mapping) are well known in the art and include, for example, immunoblotting and immunoprecipitation assays, where overlapping or contiguous peptides (for example, c -Kit domains 2 and 3) are tested for reactivity with a given antibody (for example, anti-c-Kit antibody). Methods for determining the spatial conformation of an epitope include techniques in the art and those disclosed herein, including, for example, X-ray crystallography, two-dimensional nuclear magnetic resonance, and HDX-MS (see, for example, Epitope Mapping Protocols in Methods in Molecular Biology, Vol. 66, G. E. Morris, Ed. (1996)).

The term "binds to the same epitope" in relation to two or more antibodies means that the antibodies bind to the same segment of amino acid residues as determined by a given method. Techniques for determining whether antibodies bind to "the same epitope on the c-Kit" as an antibody presented herein include, for example, epitope mapping methods, such as X-ray analysis of crystals of antigen: antibody complexes, which provide atomic resolution of the epitope, and hydrogen/deuterium exchange mass spectrometry (HDX-MS). Another method monitors binding of an antibody to an antigen fragment or a mutated variant of the antigen, in which loss of binding due to modifications in amino acid residues within the antigen sequence is primarily considered an indication of the epitope component. In addition, computational combinatorial methods for epitope mapping may also be used. These methods rely on the ability of the antibody of interest to affinity isolate specific short peptides from combinatorial phage display peptide libraries. Antibodies with identical VH and VL or identical CDR1, 2 and 3 sequences are expected to bind to the same epitope.

An antibody that "cross-competes with another antibody for binding to the target" refers to an antibody that (partially or completely) inhibits the binding of the remaining antibodies to the target. Whether two antibodies compete with each other for binding to the target, that is, whether and to what extent one antibody inhibits the binding of the remaining antibody to the target may be determined using a known competition experiment. In certain embodiments, the antibody competes with another antibody for binding to the target and inhibits this binding by at least 10%, 20%, 30%, 40%, 50%, 60%, 70%, 80%, 90%, or up to 100%. The level of inhibition or competition may differ depending on whether the antibody is a "blocking antibody" (i.e., a cold antibody that has been first incubated with the target). Competition analysis may be performed, for example, as presented in E.d. Harlow and David Lane, Cold Spring Harb Protoc; 2006; doi:10.1101/pdb.prot4277 or as presented in Chapter 11 of "Using Antibodies" by E.d. Harlow and David Lane (Cold Spring Harbor Laboratory Press, Cold Spring Harbor, NY, USA 1999). Competitive antibodies bind to the same epitope, overlapping epitopes, or adjacent epitopes (for example, by steric hindrance).

The term "reference antibody" or "standard antibody" refers to an antibody that serves as a standard for analysis of competitive antibodies that bind to the same epitope, overlapping epitope, or adjacent epitope, and that cross-competes in binding between the competitive antibody and the epitope.

Other competitive binding assays include solid phase direct or indirect radioimmunoassay (RIA), solid phase direct or indirect enzyme immunoassay (EIA), sandwich competition assay (see Stahli et al., Methods in Enzymology

9:242 (1983)); solid phase direct biotin-avidin EIA (see Kirkland et al., J. Immunol. 137:3614 (1986)); solid phase direct labeling assay, solid phase direct labeling sandwich assay (see Harlow and Lane, Antibodies: A Laboratory Manual, Cold Spring Harbor Press (1988)); solid phase direct labeling RIAusing I-125 label (see Morel et al., Mol. Immunol. 25(1):7 (1988)); solid phase direct biotin-avidin EIA (see Cheung et al., Virology 176:546 (1990)); and direct labeling RIA (see Moldenhauer et al., Scand. J. Immunol. 32:77 (1990)).

c-Kit comprises IgG like repeats, juxtamembrane, and five extracellular domains consisting of a cytoplasmic kinase domain comprising ATP-binding (TK1) and phosphotransferase (TK2) domains separated by kinase inserts. Upon ligand binding, c-Kit is activated by autophosphorylation through ligand-mediated dimerization. As a result, signaling mediators including Src, PI3K, STAT1 and JAK2 are recruited to regulate various cell-specific responses such as proliferation, survival and motility. In addition, overexpression of RTKs is known to be activated spontaneously to induce cell growth in a ligand-independent manner. Overexpression and activation of c-Kit are reported in various cancers, including GBM, astrocytoma, germinoma, and SCLC, and are correlated with poor prognosis.

The 2G4 antibody is a fully human anti-c-Kit antibody that binds to domain 2/3 of c-Kit and blocks SCF binding, thereby exhibiting the effect of inhibiting c-Kit. The specific c-Kit binding site of 2G4 has been confirmed using the 2G4/c-Kit complex docking model (J.-O. Kim et al., International Journal of Biological Macromolecules 159 (2020) 66-78), which is incorporated herein by reference.

In order to understand the molecular mechanism of the 2G4 antibody, molecular docking analysis was performed on the crystal structures of 2G4 Fab and c-Kit (PDB ID: 2E9W) using the HADDOCK/ZDOCK program. Residues 26-309 of c-Kit (D1-D3 regions, SEQ ID NO: 12) were used in docking calculations as follows.

First, blind docking calculations were performed using ZDOCK. The ten model structures with the highest ZDOCK scores generally indicate that the CDR region of 2G4 Fab interacts with the D2 and D3 regions of c-Kit (D113-D309; SEQ ID NO: 11). Based on the model structure of the 2G4 Fab:c-Kit complex, five residues (R122, Y125, R181, K203, and K205) were selected from the c-Kit D2 region (SEQ ID NO: 9), and five residues (S240, S241, Y243, N260, and W262) were selected from the c-Kit D3 region (SEQ ID NO: 10). The selected residues were expected to be responsible for 2G4 binding. Next, 10 mutant c-Kit proteins were produced, and the binding affinity of 2G4 was compared with the wild type and mutant c-Kit proteins using ELISA (Figures 14a to 14c). ELISA results showed that residues R122, Y125, R181, K203, K205, S261 and H263 of c-Kit were important for binding to 2G4.

The second docking calculation was performed using HADDOCK. The key residues of c-Kit identified by mutagenesis and ELISA analysis and all residues constituting the CDR of 2G4 were used as constraints. The docking model calculated by HADDOCK showed that the relative orientation and binding interface between 2G4 and c-Kit (i.e., the CDR loop of 2G4 and the D2/D3 region of c-Kit) were similar to those of the model calculated by ZDOCK. In the structural model of the 2G4 Fab:c-Kit complex with the highest score, it was confirmed that the heavy chain of 2G4 and the D2-D3 region of c-Kit were closely related (Figure 14d). Specifically, the binding interface between 2G4 Fab and c-Kit is mainly formed by electrostatic interactions between the heavy chain CDR of 2G4 and the D2 region of c-Kit. The basic amino acids of c-Kit, including R122, R181, K203, and R205, were shown to interact with negatively charged residues of 2G4 (D74H, E119H, D120H, E123H, and D126H) (Figure 14e). Additional interactions between S261 and H263 of the D3 region of c-Kit and the light chain residues (D82L, Q118L, T119L) of 2G4 also contributed to complex formation (Figure 14e).

Through the above docking calculation, it was proven that 2G4 binds to R122, Y125, R181, K203, R205, S261, and H263 in the D2/3 region of c-Kit.

Therefore, the present invention provides an antibody-drug conjugate in which the antibody or antigen-binding fragment thereof specifically binds to c-Kit. In certain embodiments of the antibody-drug conjugate, the antibody or antigen-binding fragment thereof specifically binds to domains 1-3 of human c-Kit (SEQ ID NO: 12), more specifically domain 2/3 of human c-Kit (SEQ ID NO: 11), even more specifically the R122 to R205 region of domain 2 of human c-Kit (SEQ ID NO: 9) and the S240 to H263 region of domain 3 (SEQ ID NO: 10), most specifically the epitopes of R122, Y125, R181, K203, R205, S261, and H263 of domain 2/3 of human c-Kit.

As used herein, the term "monoclonal antibody" refers to an antibody that exhibits a single binding specificity and affinity for a specific epitope, or a composition of antibodies in which all antibodies exhibit a single binding specificity and affinity for a specific epitope. Therefore, the term "human monoclonal antibody" refers to an antibody or a composition of an antibody that exhibits a single binding specificity and has variable and optional constant regions derived from human germline immunoglobulin sequences. In one embodiment, the human monoclonal antibody is produced by a hybridoma that comprises B cells obtained from a transgenic non-human animal, for example, a transgenic mouse with a genome containing a human heavy chain transgene and a light chain transgene fused to immortalized cells.

The term "recombinant human antibody" includes all human antibodies prepared, expressed, produced, or isolated by recombinant means, such as (a) an antibody isolated from an animal (for example, a mouse) that is transgenic or transchromosomal for a human immunoglobulin gene, or a hybridoma prepared therefrom, (b) an antibody isolated from host cells transformed to express antibodies, for example from transfectomas, (c) an antibody isolated from recombinant, combinatorial human antibody libraries, and (d) an antibody prepared, expressed, produced, or isolated by any other means involving splicing a human immunoglobulin gene sequence to another DNA sequence. These recombinant human

antibodies are encoded by germline genes, but comprise variable and constant regions utilizing specific human germline immunoglobulin sequences, including subsequent rearrangements and mutations that occur, for example, during antibody maturation. As known in the art (see, for example, Lonberg (2005) Nature Biotech. 23(9): 1117-1125), the variable region contains an antigen binding domain, encoded by various genes that are rearranged to form antibodies specific for foreign antigens. In addition to rearrangements, the variable region may be further modified by multiple single amino acid changes (somatic mutations or hypermutations) to increase the affinity of the antibody for foreign antigens. The constant region will change in further response to an antigen (i.e., isotype switch). Therefore, a rearranged and somatically mutated nucleic acid molecule that encodes light and heavy chain immunoglobulin polypeptides in response to an antigen may not have sequence identity with the original nucleic acid molecule, but will instead be substantially identical or similar (i.e., has at least 80% identity).

The term "human" antibody (HuMAb) refers to an antibody with variable regions in which both the framework and CDR regions are derived from a human germline immunoglobulin sequence. In addition, if the antibody contains a constant region, the constant region is also derived from a human germline immunoglobulin sequence. The antibody presented herein may comprise amino acid residues that are not encoded by a human germline immunoglobulin sequence (for example, mutations introduced by random or site-directed mutagenesis *in vitro* or by somatic mutation *in vivo*). However, the term "human antibody" does not include an antibody in which CDR sequences derived from the germline of other mammalian species, such as a mouse, have been grafted onto human framework sequences. The terms "human" antibody and "fully human" antibody are used synonymously.

The term "humanized antibody" refers to an antibody in which some, most or all of the amino acids outside the CDR domains of a non-human antibody have been substituted with corresponding amino acids derived from a human immunoglobulin. In one embodiment of the humanized form of the antibody, some, most or all of the amino acids outside the CDR domains are substituted with amino acids from a human immunoglobulin, and some, most or all of the amino acids within one or more CDR regions remain intact. Additions, deletions, insertions, substitutions or modifications of amino acids are permitted as long as they do not abolish the ability of the antibody to bind to the specific antigen. The "humanized" antibody possesses antigen specificity similar to that of the original antibody.

The term "chimeric antibody" refers to an antibody whose variable region is derived from one species and whose constant region is derived from another species, such as an antibody whose variable region is derived from a mouse antibody and whose constant region is derived from a human antibody.

As used herein, the term "cross-reacts" refers to the ability of an antibody presented herein to bind to c-Kit from different species. For example, an antibody provided herein that binds to human c-Kit may also bind to c-Kit from other species (for example, mouse c-Kit). This cross-reactivity may be measured by detecting specific reactivity with purified antigen in a binding assay (for example, SPR, ELISA) or by detecting binding, or functional interaction, with cells that physiologically express c-Kit. Methods for determining cross-reactivity include standard binding assays disclosed herein, such as BIACORE® surface plasmon resonance (SPR) analysis using a BIACORE® 2000 SPR instrument (Biacore AB, Uppsala, Sweden), or flow cytometry techniques.

The term "conservative amino acid substitution" refers to the substitution of an amino acid residue with an amino acid residue having a similar side chain. Families of an amino acid residue with a similar side chain is defined in the art. These families include amino acids with basic side chains (for example, lysine, arginine, histidine), acidic side chains (for example, aspartic acid, glutamic acid), uncharged polar side chains (for example, glycine, asparagine, glutamine, serine, threonine, tyrosine, cysteine, tryptophan), non-polar side chains (for example, alanine, valine, leucine, isoleucine, proline, phenylalanine, methionine), beta-branched side chains (for example, threonine, valine, isoleucine), and aromatic side chains (for example, tyrosine, phenylalanine, tryptophan, histidine). In certain embodiments, a expected non-essential amino acid residue in an anti-c-Kit antibody is substituted with another amino acid residue from the same side chain family. Methods for identifying nucleotide and amino acid conservative substitutions that do not impair antigen binding are well known in the art (see, for example, Brummell et al., Biochem. 32: 1180-1187 (1993); Kobayashi et al. Protein Eng. 12(10):879-884 (1999); and Burks et al. Proc. Natl. Acad. Sci. USA 94:412-417 (1997)).

The term "substantial homology" indicates that two polypeptides, or their designated sequences, are identical when optimally aligned and compared, wherein at least about 80% of the amino acids, at least about 90% to 95%, or at least about 98% to 99.5% of the amino acids have the appropriate amino acid insertion or deletion.

The percent identity between two sequences is a function of the number of identical positions shared by the sequences, taking into account the number of gaps, and the length of each gap, that must be introduced for optimal alignment of the two sequences (i.e., % homology = # of identical positions / total # of positions $\times$ 100). Comparison of sequences and percent identity between two sequences may be determined using mathematical algorithms such as those described in the non-limiting examples below.

The percent identity between two amino acid sequences may be determined using the Needleman and Wunsch (J. Mol. Biol. (48):444-453 (1970)) algorithm integrated in the GAP program of the GCG software package (available at http://www.gcg.com), which uses a Blossum 62 matrix or a PAM250 matrix, and gap weights of 16, 14, 12, 10, 8, 6, or 4, and length weights of 1, 2, 3, 4, 5, or 6.

Nucleic acid and protein sequences presented herein may be used, for example, as "query sequences" to perform searches in public databases to identify related sequences. These searches may be performed using the NBLAST and XBLAST programs (version 2.0) from Altschul, et al. (1990) J. Mol. Biol. 215:403-10. BLAST nucleotide searches may be performed under the NBLAST program, score = 100, word length = 12, resulting in nucleotide sequences homologous to the nucleic acid molecules presented herein. BLAST protein searches may be performed under the XBLAST program, score = 50, word length = 3, resulting in amino acid sequences homologous to the protein molecules presented herein. In order to obtain gapped alignments for comparison purposes, Gapped BLAST may be used as described in Altschul et al., (1997) Nucleic Acids Res. 25(17): 3389-3402. When using BLAST and Gapped BLAST programs, the default variables for each program (for example, XBLAST and NBLAST) may be used (see worldwideweb.ncbi.nlm.nih.gov).

As used herein, the term "linked" refers to the association of two or more molecules. The linkage may be a covalent linkage or a non-covalent linkage. The linkage may also be a genetic (i.e., recombinantly fused) linkage. Such linkages may be achieved using a variety of art-recognized techniques, such as chemical conjugation and recombinant protein production.

As used herein, the term "antibody-drug conjugate" refers to the linkage of an antibody or an antigen-binding fragment thereof with another agent, such as an anticancer agent, a chemotherapy agent, a toxin, an immunotherapy agent, an imaging probe, a spectroscopic probe, and the like. This linkage may be a covalent bond, or a non-covalent interaction such as an interaction through electrostatic forces. A variety of linkers known in the art may be used to form an antibody-drug conjugate. Additionally, the antibody-drug conjugate may be provided in the form of a fusion protein that may be expressed from a polynucleotide encoding the antibody-drug conjugate. As used herein, the term "fusion protein" refers to a protein produced through the linkage of two or more genes or gene fragments that originally encode separate proteins (including peptides and polypeptides). Translation of the fusion gene results in a single protein with functional properties derived from each of the original proteins.

As used herein, the term "spacer" refers to a substance that serves to prevent aggregation of the antibody-drug conjugate in the bloodstream and provide stability in the bloodstream and target pharmacokinetic properties.

As used herein, the term "linker" is any chemical moiety that may link an antibody, an antibody fragment (for example, an antigen-binding fragment), or a functional equivalent to another moiety, such as a drug moiety. Under conditions where the compound or antibody is still active, the linker may be susceptible to cleavage such as acid-induced cleavage, light-induced cleavage, peptidase-induced cleavage, esterase-induced cleavage, and disulfide bond cleavage (a cleavable linker). Alternatively, the linker may be substantially resistant to cleavage (for example, a stable linker or a non-cleavable linker). In some embodiments, the linker is a procharged linker, a hydrophilic linker, or a dicarboxylic acid-based linker.

The term "non-cleavable linker" has relatively high plasma stability and are resistant to proteolysis. After being introduced into the cell, the antibody must be degraded to release the drug in the form of a complex with the linker, and this complex has drug activity. Thioether linker is a representative non-cleavable linker, which allows the drug to be released after the antibody is non-selectively degraded within the cell. Kadcyla ® adopts a non-cleavable form.

The term "cleavable linker" refers to a linker that may be cleaved in response to specific environmental factors to release the drug into the cytoplasm. There are two types of cleavable forms: an enzyme cleavable form and a non-enzyme cleavable form.

The enzyme cleavable form is cleaved by enzymes such as cathepsin B, β-glucuronidase, phosphatase, pyrophosphatase, and sulfatase. The peptide linker is degraded by proteolytic enzymes, and proteolytic enzyme inhibitors are present in plasma, resulting in high plasma stability. Cathepsin B is a representative proteolytic enzyme used in antibody-drug conjugates, and cathepsin B is present at high levels in tumor tissue, giving tumor selectivity to antibody-drug conjugates. The peptide linker may mainly be a dipeptide in which two amino acids are attached. The β-glucuronide linker is degraded by β-glucuronidase, a glycolytic enzyme present in lysosomes. β-glucuronidase is overexpressed in some tumor cells, giving it tumor specificity.

Non-enzyme cleavable forms include an acid-labile linker and a oxidation-reduction reaction linker.

An Acid-labile linker is a linker with relatively low stability that was developed early on, but are still used. A representative example is a hydrazone linker, which is internalized in tumor cells and then hydrolyzed in the slightly acidic environment of endosomes or lysosomes to release the drug.

A representative oxidation-reduction reaction linker is a disulfide linker. After internalization, the linker is degraded by disulfide exchange or reducing agents such as glutathione, to release cytotoxic drugs. Glutathione has a concentration about 1,000 times higher in hypoxic tumor cells than in normal cells, to give ADC tumor cell-selectivity.

A procharged linker is are derived from charged cross-linking reagents that retain their charge after incorporation into an antibody-drug conjugate. An example of a procharged linker may be found in US Patent Application Publication No. 2009/0274713.

## II. Antibody-drug conjugate (ADC)

According to one embodiment of the present invention, the present invention provides an antibody-drug conjugate of the following formula 1:

<Formula 1> $Ab\text{-}(L)_x\text{-}(D)_y$

in the formula,

Ab is an anti-c-Kit antibody or an antigen-binding fragment thereof that specifically binds to the epitope of human c-Kit in SEQ ID NO: 9 and SEQ ID NO: 10;
L is a linker comprising a cleavable linker;
D is a drug moiety;
x is an integer from 1 to 8; and
y is an integer from 1 to 8.

In some embodiments, the Ab is an antibody or an antigen-binding fragment thereof that specifically binds to one or more of R122, Y125, R181, K203, R205, S261, and H263 in SEQ ID NO: 12. Preferably, the Ab is an antibody or an antigen-binding fragment thereof that specifically binds to R122, Y125, R181, K203, R205, S261, and H263 in SEQ ID NO: 12. At this time, SEQ ID NO: 12 is the sequence of domains 1 to 3 (Q26 to D309) of human c-Kit excluding the 25 amino acids of signal peptide, and the number of each amino acid is a number including the 25 amino acids of signal peptide. Accordingly, for example, R122 refers to the 97th amino acid sequence in SEQ ID NO: 12, Y125 refers to the 100th amino acid sequence, and the remaining amino acids may also be interpreted in the same manner.

In some embodiments, the antibody or antigen-binding fragment thereof cross-competes for binding to the epitope of human c-Kit in SEQ ID NO: 9 and SEQ ID NO: 10 with a reference antibody comprising the heavy chain CDR1 of SEQ ID NO: 1, the heavy chain CDR2 of SEQ ID NO: 2, the heavy chain CDR3 of SEQ ID NO: 3, the light chain CDR1 of SEQ ID NO: 4, the light chain CDR2 of SEQ ID NO: 5, and the light chain CDR3 of SEQ ID NO: 6.

In some embodiments, the antibody or antigen-binding fragment thereof cross-competes for binding to the epitope of human c-Kit in SEQ ID NO: 9 and SEQ ID NO: 10 with a reference antibody comprising the heavy chain variable domain represented by SEQ ID NO: 7 and the light chain variable domain represented by SEQ ID NO: 8.

In some embodiments, the antibody or antigen-binding fragment thereof comprises the heavy chain CDR1 of SEQ ID NO: 1, the heavy chain CDR2 of SEQ ID NO: 2, the heavy chain CDR3 of SEQ ID NO: 3, the light chain CDR1 of SEQ ID NO: 4, the light chain CDR2 of SEQ ID NO: 5, and the light chain CDR3 of SEQ ID NO: 6.

In some embodiments, the antibody or antigen-binding fragment thereof comprises the heavy chain variable domain represented by SEQ ID NO: 7 and the light chain variable domain represented by SEQ ID NO: 8.

The antibody or antigen-binding fragment thereof may have one or two amino acids in the CDR modified, deleted, or substituted, but is not limited thereto.

In some embodiments, the antibody or antigen-binding fragment thereof may maintain at least 90, 91, 92, 93, 94, 95, 96, 97, 98, or 99% identity through the heavy chain variable region or the light chain variable region.

In some embodiments, the antibody or antigen-binding fragment thereof may be a monoclonal antibody, a chimeric antibody, a humanized antibody, a human engineered antibody, a human antibody, a single chain antibody (scFv), or an antibody fragment.

In some embodiments, the antibody or antigen-binding fragment thereof may have an equilibrium dissociation constant $K_D$ value for human c-KIT of $10^{-11}$ M or less, specifically, for example, about 2.8237 ($\pm$ 0.9) $\times$ $10^{-12}$ M.

The cleavable linker includes a linker selected from the group consisting of valine-citrulline-p-aminobenzyl carbamoyl (Val-Cit-PAB), alanine-phenylalanine-p-aminobenzyl carbamoyl (Ala-Phe-PAB), alanine-alanine-p-aminobenzyl carbamoyl (Ala-Ala-PAB), valine-alanine-p-aminobenzyl carbamoyl (Val-Ala-PAB), phenylalanine-lysine-p-aminobenzyl carbamoyl (Phe-Lys-PAB), alanine-alanine-glycine-p-aminobenzyl carbamoyl (Ala-Ala-Gly-PAB), and glycine-glycine-glycine-p-aminobenzyl carbamoyl (Gly-Gly-Gly-PAB) linkers, but is not limited thereto.

L may include a spacer in a form in which a polymer is bound or not bound.

According to one embodiment of the present invention, L is a linker including a linker derived from a cross-linking reagent comprising a substituent selected from the group consisting of

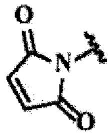 , 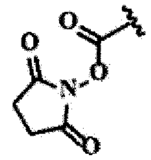 , 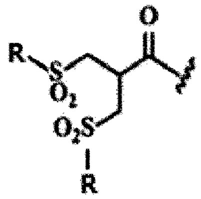

(for example,

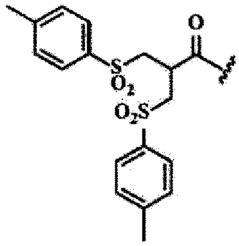

),

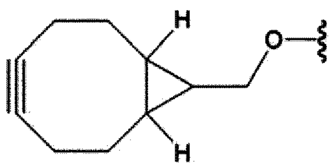 , 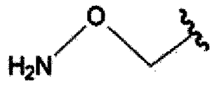 , 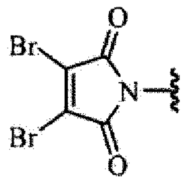 , 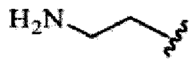 ,

and

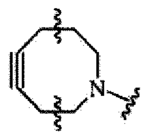

In one embodiment, when the linker is prepared using a cross-linking reagent comprising a substituent of

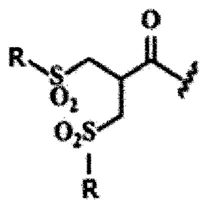

(for example,

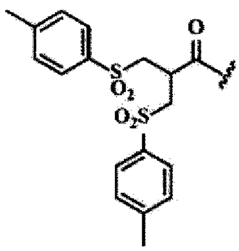

), for example, the linker may include a disulfide bridge connecting two cysteine residues, such as ThioBridge® (Abzena) technology.

In the above formula, the wavy line ( ∿∿∿ ) represents the site where another linker or S or D binds.

R may be hydrogen, halogen, substituted or unsubstituted $C_1$-$C_6$ alkyl, $C_1$-$C_6$ alkoxy, $C_1$-$C_4$ haloalkyl, $C_1$-$C_4$ haloalkoxy, cyano, nitro, phenyl, toluene, or halophenyl.

In some embodiments, L is a linker comprising a spacer in a form in which a polymer is bound or not bound.

In some embodiments, L comprises a structure of the following formula 2:

<Formula 2> $(L_1)_m$-$(S)_n$-$(L_2)_p$

in the formula,

$L_1$ is a linker connecting Ab and S or Ab and $L_2$;
S is a spacer in a form in which a polymer is bound or not bound;
$L_2$ is a cleavable linker;
m is an integer from 0 to 8;
n is an integer from 0 to 8; and
p is an integer from 1 to 8.

In some embodiments, m is 1 to 4, n is 1 to 4, and p is 1 to 4.

In some embodiments, $L_1$ includes a linker selected from the group consisting of a cleavable linker, a non-cleavable linker, a hydrophilic linker, a procharged linker, and a dicarboxylic acid-based linker.

In some embodiments, L or $L_1$ includes

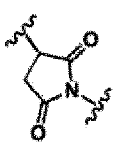 , 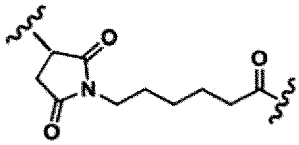 ,

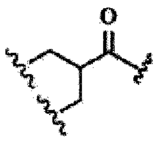 , 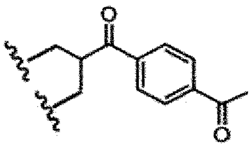 , 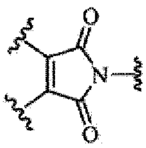 , 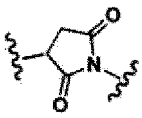 , 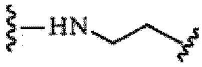 , 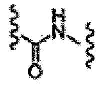 ,

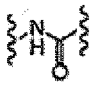 , 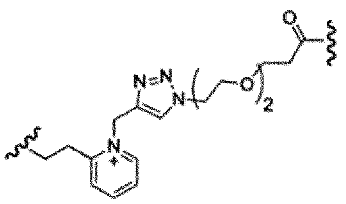 , 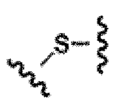 , 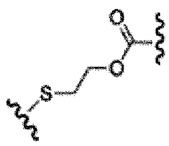 , 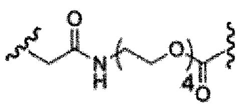 ,

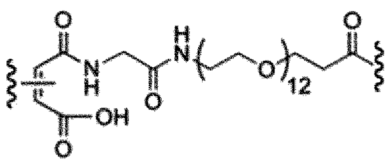 , 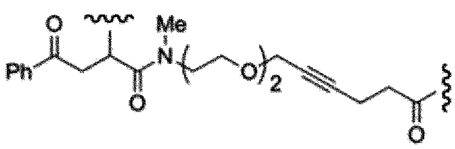 , 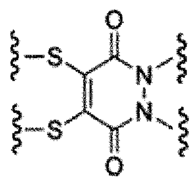 ,

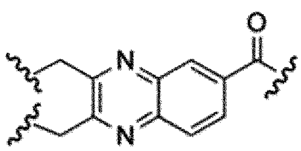 , 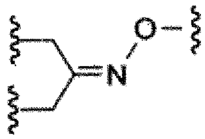 , 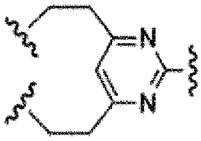 , 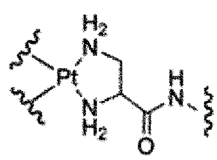 ,

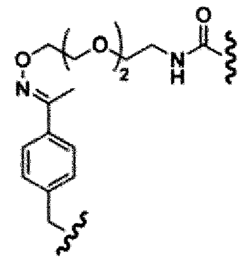, 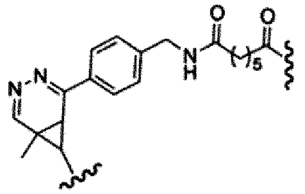, 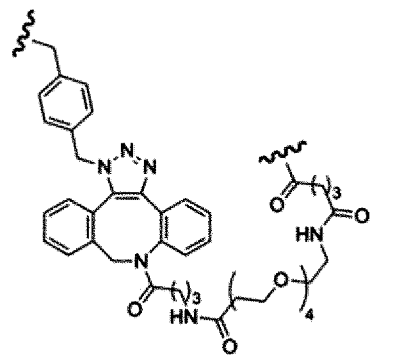,

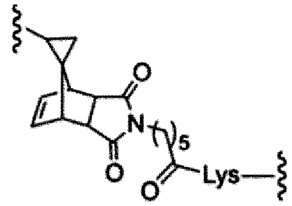, 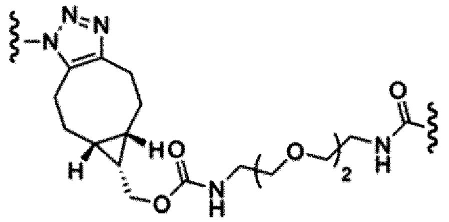, 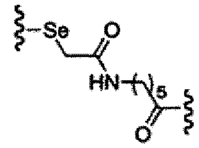,

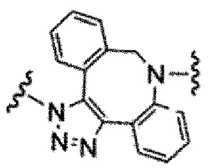, 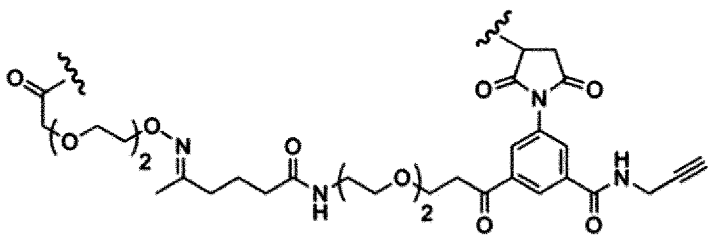,

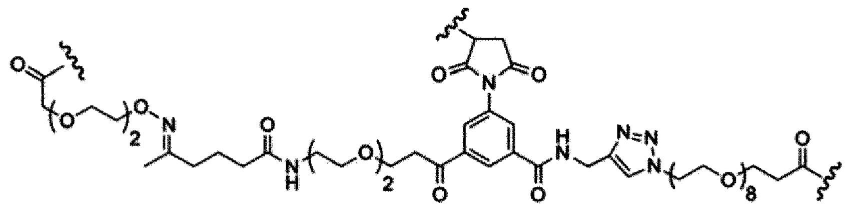, or 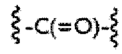

In the above formula, the wavy line ( 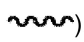) represents the site where Ab or S, $L_2$ or D binds.

In some embodiments, $L_1$ is a linker including a linker derived from a cross-linking reagent comprising a substituent selected from the group consisting of

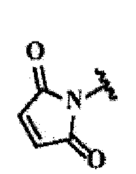, 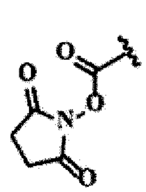,

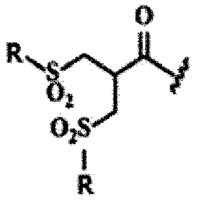

(for example,

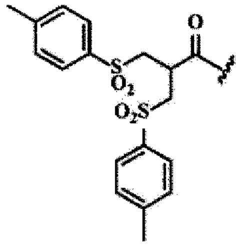

),

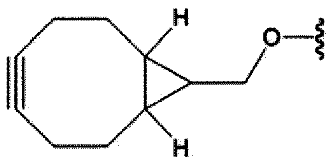

,

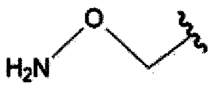

,

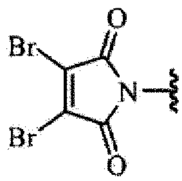

,

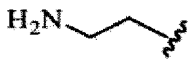

,

and

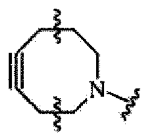

In the above formula, the wavy line ( 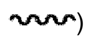 ) represents the site where S binds.

R may be hydrogen, halogen, substituted or unsubstituted $C_1$-$C_6$ alkyl, $C_1$-$C_6$ alkoxy, $C_1$-$C_4$ haloalkyl, $C_1$-$C_4$ haloalkoxy, cyano, nitro, phenyl, toluene, or halophenyl.

According to one embodiment of the present invention, the S (spacer) may be selected from the group consisting of

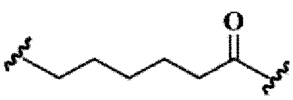

,

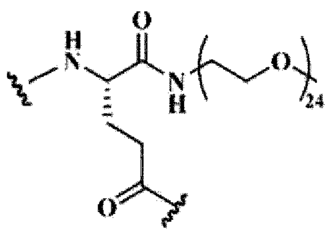

,

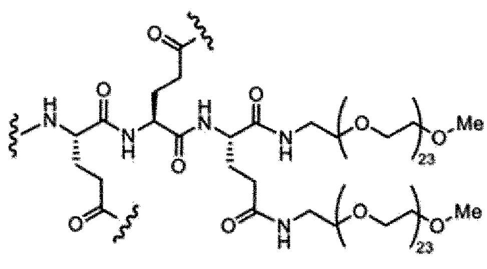

,

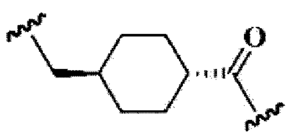

,

and

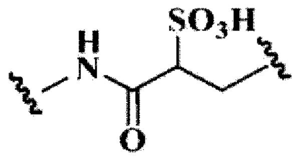

In the above formula, the wavy line ( 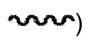) represents the site where $L_1$ or $L_2$ binds, and preferably $L_1$ binds to the left wavy line and $L_2$ binds to the right wavy line.

In some embodiments, S may be a spacer comprising aspartate, glutamate, or a combination thereof in a form in which a polymer is bound or not bound.

As an example of the polymer, polyalkylene, polyalkylene glycol, polyvinylpyrrolidone, polyacrylate, polyoxazoline, polyvinyl alcohol, polyacrylamide or polymethacrylamide, HPMA copolymer, polyester, polyacetal, poly(orthoester), polycarbonate, poly(imino carbonate), polyamide, a copolymer of divinylether-maleic anhydride or styrenemaleic anhydride, polysaccharide, or polyglutamic acid, etc. may be used, but is not limited thereto.

In some embodiments, the polymer is polyethylene or polyethylene glycol.

The polyethylene glycol may comprise 10 to 30, for example, 24 ethylene glycol units ($-O-CH_2-CH_2-$).

According to one embodiment of the present invention, L includes a linker selected from the group consisting of the following formulas 3 to 5:

<Formula 3>

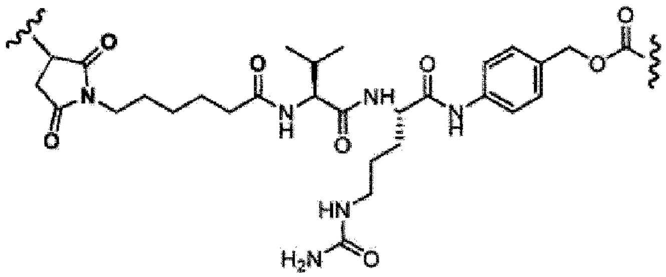

,

<Formula 4>

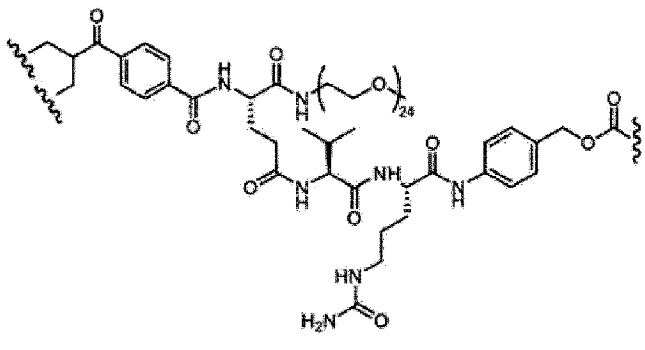

,

or

<Formula 5>

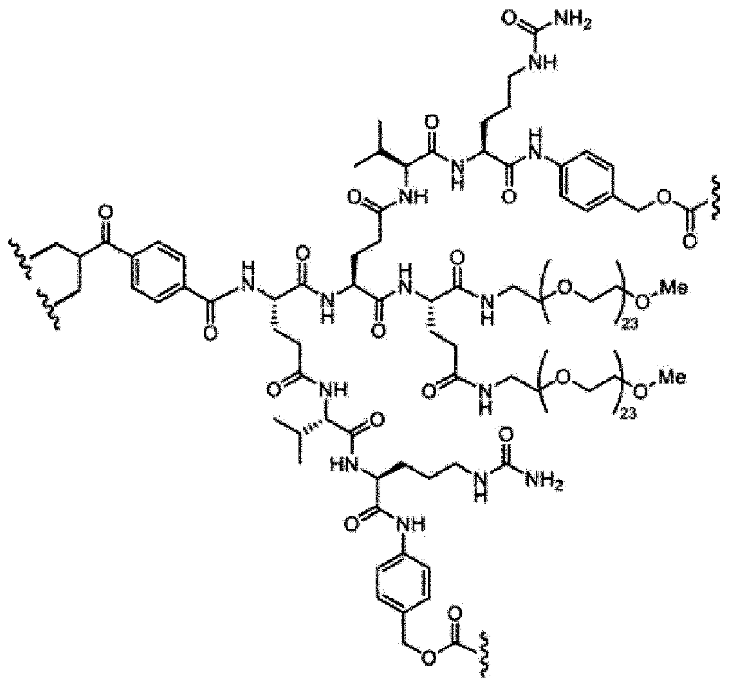

In the above formula, the wavy line ( ) represents the site where Ab or D binds, and preferably Ab binds to the left wavy line and D binds to the right wavy line.

D is a drug moiety, and is preferably a moiety selected from the group consisting of a V-ATPase inhibitor, an apoptosis promoting agent, a Bcl2 inhibitor, an MCL1 inhibitor, a HSP90 inhibitor, an IAP inhibitor, an mTor inhibitor, a microtubule inhibitor, an auristatin, a dolastatin, a maytansinoid, a MetAP (methionine aminopeptidase), a inhibitor of nuclear export of protein CRM1, a DPPIV inhibitor, a proteasome inhibitor, a inhibitor of phosphoryl transfer reaction in mitochondria, a protein synthesis inhibitor, a kinase inhibitor, a CDK2 inhibitor, a CDK9 inhibitor, a kinesin inhibitor, a HDAC inhibitor, a DNA damaging agent, a DNA alkylating agent, a DNA intercalating agent, a DNA minor groove binder, and a DHFR inhibitor.

An antibody, an antibody fragment (for example, an antigen-binding fragment), or a functional equivalent of the present disclosure may be conjugated to a drug moiety that modifies a desired biological response. The drug moiety should not be construed as limited to classical chemical therapeutic agents. For example, the drug moiety may be a protein, peptide, or polypeptide that possesses the desired biological activity. Such proteins may include, for example, toxins such as abrin, ricin A, pseudomonas exotoxin, cholera toxin, or diphtheria toxin, proteins such as tumor necrosis factor, α-interferon, β-interferon, nerve growth factor, platelet-derived growth factor, tissue plasminogen activator, cytokine, apoptotic agent, anti-angiogenic agent, or biological response modifiers such as lymphokine.

In some embodiments, an antibody, an antibody fragment (for example, an antigen-binding fragment), or a functional equivalent of the present disclosure is conjugated to a drug moiety, such as a cytotoxin, a drug (for example, an immunosuppressant), or a radiotoxin. Examples of cytotoxins also include taxanes (see, for example, International (PCT) Patent Application Nos. WO 01/38318 and PCT/US03/02675), DNA-alkylating agents (for example, CC-1065 analogs), anthracyclines, tubulysin analogs, duocarmycin analogs, auristatin E, auristatin F, maytansinoids, and cytotoxic agents comprising a reactive polyethylene glycol moiety (see, for example, literatures [Sasse et al., J. Antibiot. (Tokyo), 53, 879-85 (2000)], [Suzawa et al., Bioorg. Med. Chem., 8, 2175-84 (2000)], [Ichimura et al., J. Antibiot. (Tokyo), 44, 1045-53 (1991)], [Francisco et al., Blood 2003 15;102(4):1458-65], US Patent Nos. 5,475,092, 6,340,701, 6,372,738, and 6,436,931, US Patent Application Publication No. 2001/0036923 A1, pending US Patent Application Serial Nos. 10/024,290 and 10/116,053, and International (PCT) Patent Application No. WO 01/49698), taxone, cytochalasin B, gramicidin D, ethidium bromide, emetine, mitomycin, etoposide, tenoposide, vincristine, vinblastine, T. colchicine, doxorubicin, daunorubicin, dihydroxyanthracene dione, mitoxantrone, mithramycin, actinomycin D, 1-dehydrotestosterone, glucocorticoids, procaine, tetracaine, lidocaine, propranolol, and puromycin, and analogs or homologs thereof, but are not limited thereto. Therapeutic agents also include, for example, anti-metabolites (for example, methotrexate, 6-mercaptopurine, 6-thioguanine, cytarabine, 5-fluorouracil decarbazine), alkylating agents (for example, mechlorethamine, thiotepa chlorambucil, melphalan, carmustine (BSNU) and lomustine (CCNU), cyclophosphamide, busulfan, dibromomannitol, streptozotocin, mitomycin C, and cis-dichlorodiamine platinum (II) (DDP) cisplatin, anthracyclines (for example, daunorubicin (formerly daunomycin) and doxorubicin), antibiotics (for example, dactinomycin (formerly actinomycin), bleomycin,

mithramycin and anthramycin (AMC)), and anti-mitotic agents (for example, vincristine and vinblastine) (see, for example, Seattle Genetics US20090304721).

Other examples of cytotoxins that may be conjugated to an antibody, an antibody fragment (an antigen-binding fragment), or a functional equivalent of the present disclosure include duocarmycin, calicheamicin, maytansine and auristatin, and derivatives thereof.

Methods for conjugating various types of cytotoxins, linkers, and therapeutic agents to antibodies are known in the art, and see, for example, literatures [Saito et al., (2003) Adv. Drug Deliv. Rev. 55:199-215]; [Trail et al., (2003) Cancer Immunol. Immunother. 52:328-337]; [Payne, (2003) Cancer Cell 3:207-212]; [Allen, (2002) Nat. Rev. Cancer 2:750-763]; [Pastan and Kreitman, (2002) Curr. Opin. Investig. Drugs 3:1089-1091]; [Senter and Springer, (2001) Adv. Drug Deliv. Rev. 53:247-264].

An antibody, an antibody fragment (for example, an antigen-binding fragment), or a functional equivalent of the present disclosure may also be conjugated to a radioisotope to produce cytotoxic radiopharmaceuticals, referred to as radioimmunoconjugates. Examples of radioisotopes that may be conjugated to antibodies for diagnostic or therapeutic use include, but are not limited to, iodine-131, indium-111, yttrium-90, and lutetium-177. Methods for preparing radioimmunoconjugates are established in the art. Examples of radioimmunoconjugates are commercially available, including Zevalin™ (IDEC Pharmaceuticals) and Bexxar™ (Corixa Pharmaceuticals), and similar methods may be used using the antibodies disclosed herein to prepare radioimmunoconjugates. In certain aspects, the macrocyclic chelator is 1,4,7,10-tetraazacyclododecane-N,N′,N″,N‴-tetraacetic acid (DOTA), which may be attached to an antibody through a linker molecule. Such linker molecules are commonly known in the art and are described in the literatures [Denardo et al., (1998) Clin Cancer Res. 4(10):2483-90]; [Peterson et al., (1999) Bioconjug. Chem. 10(4):553-7]; and [Zimmerman et al., (1999) Nucl. Med. Biol. 26(8):943-50] (each incorporated by reference in its entirety).

In some embodiments, D is a microtubule inhibitor (MTI) moiety.

The microtubule inhibitor may include, preferably, a drug selected from the group consisting of SN-38 ((4S)-4,11-diethyl-4,9-dihydroxy-1,4-dihydro-3*H*,14*H*-pyrano[3',4':6,7]indolizino[1,2- *b*]quinoline-3,14-dione), auristatin, dolastatin, monomethyl auristatin E (MMAE), monomethyl auristatin F (MMAF), monomethyl dolastatin 10 (MMAD), or a combination thereof, but is not limited thereto.

SN-38 prevents cell division by inhibiting DNA isomerase I (DNA topoisomerase I). It has lower potency compared to previously used drugs, so there are fewer side effects, and it may be used to increase the potency of an antibody-drug conjugate compared to the side effects by increasing DAR.

The antibody or antigen-binding fragment thereof preferably binds specifically to the epitope of domain 2/3 of human c-Kit represented by SEQ ID NO: 11 (i.e., D113 to D309), more preferably binds specifically to the epitope of domain 2 of human c-Kit represented by SEQ ID NO: 9 (i.e., R122 to R205) and/or domain 3 of human c-Kit represented by SEQ ID NO: 10 (i.e., S240 to H263), even more preferably binds specifically to one or more amino acid residues selected from the group consisting of R122, Y125, R181, K203, and R205 in SEQ ID NO: 9 and/or one or more amino acid residues selected from the group consisting of S261 and H263 in SEQ ID NO: 10.

In some embodiments, the antibody or antigen-binding fragment thereof cross-competes for binding to the epitope of domain 2/3 of human c-Kit in SEQ ID NO: 11 (i.e., D113 to D309) with a reference antibody comprising the heavy chain variable domain represented by SEQ ID NO: 7 and the light chain variable domain represented by SEQ ID NO: 8.

According to one embodiment of the present invention, the antibody-drug conjugate is selected from the group consisting of the following formulas 6 to 8:

<Formula 6>

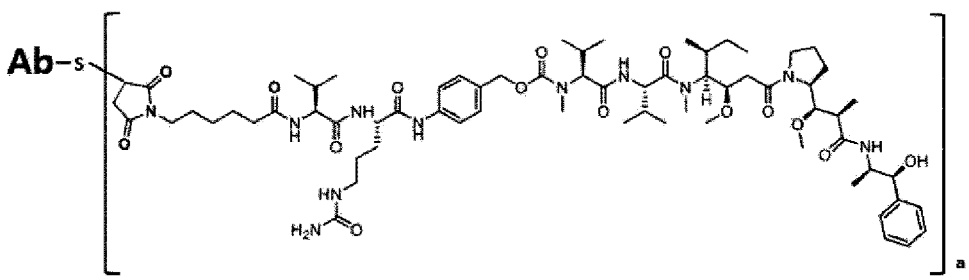

,

<Formula 7>

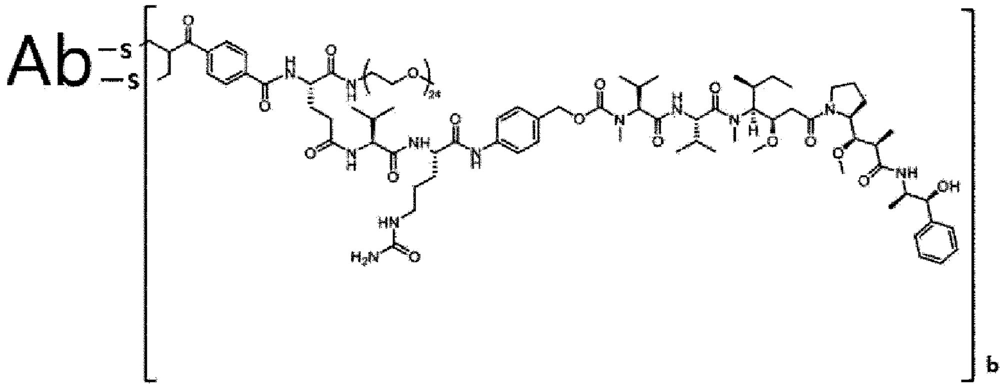

and

<Formula 8>

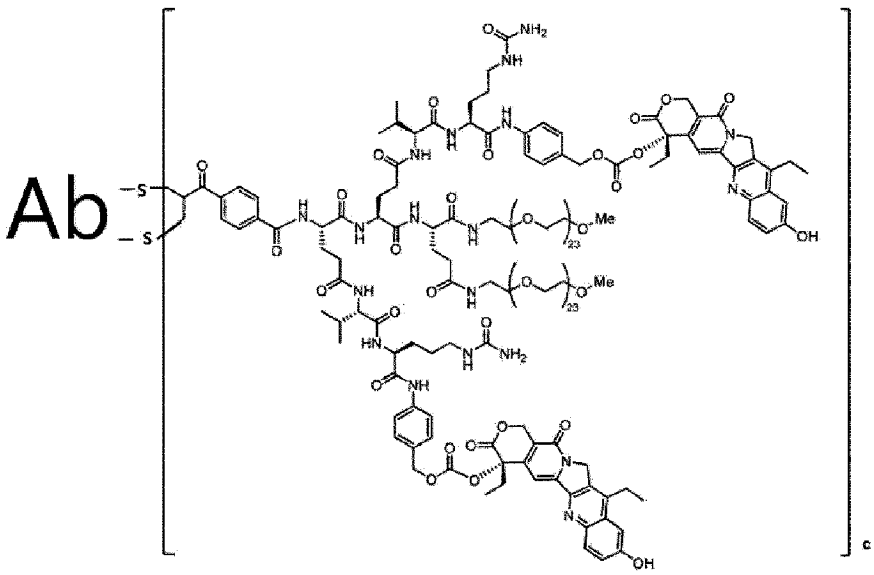

In the formulas, a is an integer from 1 to 8, and b and c are each an integer from 1 to 4.

According to another embodiment of the present invention, the present invention provides an antibody-drug conjugate selected from the group consisting of the following formulas 6 to 8:

<Formula 6>

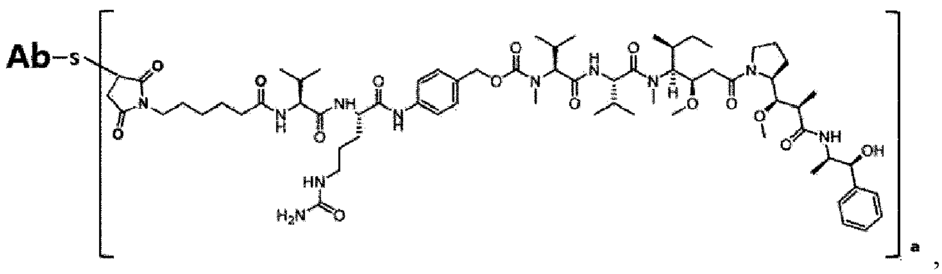

<Formula 7>

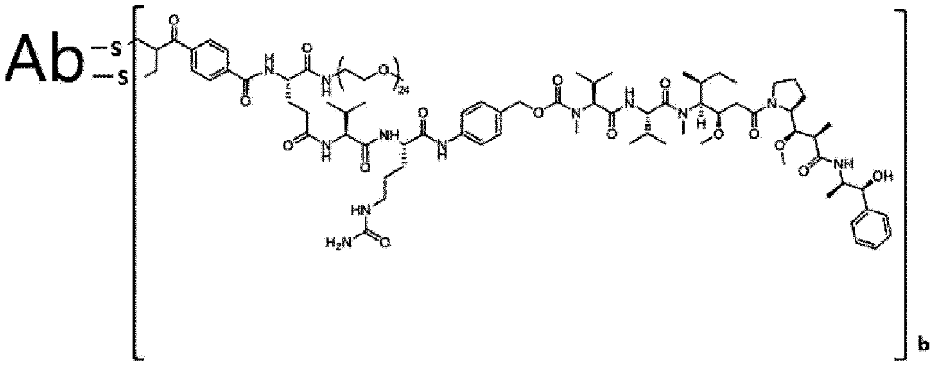

and

<Formula 8>

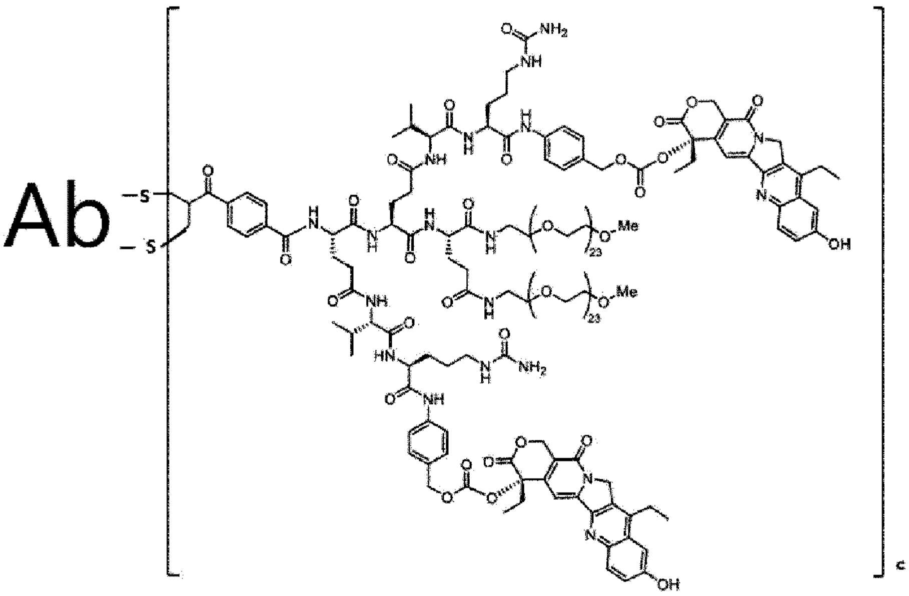

in the formulas,

Ab is an antibody or an antigen-binding fragment thereof comprising the heavy chain CDR1 of SEQ ID NO: 1, the heavy chain CDR2 of SEQ ID NO: 2, the heavy chain CDR3 of SEQ ID NO: 3, the light chain CDR1 of SEQ ID NO: 4, the light chain CDR2 of SEQ ID NO: 5, and the light chain CDR3 of SEQ ID NO: 6; and
a is an integer from 1 to 8, and b and c are each an integer from 1 to 4.

In some embodiments, the antibody-drug conjugate has a structure of formula 7, and b is 2.
In some embodiments, the antibody-drug conjugate has a structure of formula 7, and b is 4.

**III. Composition**

According to another embodiment of the present invention, the present invention provides a composition comprising an antibody-drug conjugate or a pharmaceutically acceptable salt thereof.
In some embodiments, the composition is a pharmaceutical composition for preventing or treating cancer.
According to another embodiment of the present invention, the present invention provides a use of the composition for use in therapy.
In some embodiments, the use is for the treatment of cancer.
According to another embodiment of the present invention, the present invention provides a use of the pharmaceutical composition for manufacture of a medicament.

According to another embodiment of the present invention, the present invention provides a method for preventing or treating cancer, comprising administering the composition to a subject in need thereof.

Disclosed herein is a method for treating a tumor (or cancer) in a subject, wherein the method comprises administering the antibody-drug conjugate or a pharmaceutically acceptable salt thereof to a subject. In certain embodiments, the antibody-drug conjugate specifically binds to c-Kit protein and reduces c-Kit activity.

In some embodiments, the method disclosed herein inhibits and/or reduces the tumor growth in a subject. In certain embodiments, the tumor growth (for example, tumor volume or weight) is reduced by at least 5%, at least 10%, at least 20%, at least 30%, at least 40%, at least 50%, at least 60%, at least 70%, at least 80%, at least 90%, or up to 100% compared to a reference (for example, the corresponding tumor volume or weight in a subject that did not receive the composition disclosed herein, for example, the antibody-drug conjugate). In some embodiments, the method disclosed herein increases the average tumor growth inhibition (TGI) by at least 5%, at least 10%, at least 20%, at least 30%, at least 40%, at least 50%, at least 60%, at least 70%, at least 80%, at least 90%, or up to 100% compared to a reference (for example, the corresponding frequency in a subject that did not receive the composition disclosed herein, for example, the antibody-drug conjugate). In some embodiments, administration of the composition disclosed herein (for example, the antibody-drug conjugate) increases the average survival by at least 1 day, 2 days, 3 days, 4 days, 5 days, 6 days or more compared to a reference (for example, the corresponding value in a subject that did not receive the composition disclosed herein, for example, the antibody-drug conjugate).

In some embodiments, tumors that may be treated with the method disclosed herein are derived from cancers that are typically responsive to existing anticancer agents and cancers that are typically non-responsive to existing anticancer agents. In some embodiments, the cancer is a solid tumor or cancer with hematological malignancy (liquid tumor).

Non-limiting examples of cancers requiring treatment include squamous cell carcinoma, small cell lung cancer (SCLC), non-small cell lung cancer, squamous non-small cell lung cancer (NSCLC), non-squamous NSCLC, gastrointestinal cancer, renal cancer (for example, clear cell carcinoma), ovarian cancer, liver cancer, colorectal cancer, endometrial cancer, kidney cancer (for example, renal cell carcinoma (RCC)), prostate cancer (for example, hormone refractory prostate adenocarcinoma), thyroid cancer, pancreatic cancer, cervical cancer, gastric cancer, bladder cancer, hepatocellular cancer, breast cancer, colon carcinoma, and head and neck cancer (or carcinoma), germ cell tumor, pediatric sarcoma, nasal natural killer, melanoma (for example, metastatic malignant melanoma, such as cutaneous or intraocular malignant melanoma), bone cancer, skin cancer, uterine cancer, cancer of the anal region, testicular cancer, carcinoma of the fallopian tube, carcinoma of the endometrium, carcinoma of the cervix, carcinoma of the vagina, carcinoma of the vulva, esophageal cancer, small intestine cancer, large intestine cancer, mast cell tumor, cancer of the endocrine system, cancer of the parathyroid gland, cancer of the adrenal gland, sarcoma of soft tissue, cancer of the urethra, cancer of the genitals, solid tumor in children, cancer of the ureter, carcinoma of the renal pelvis, tumor angiogenesis, pituitary adenoma, Kaposi's sarcoma, epithelial cancer, squamous cell cancer, T-cell lymphoma, environmentally induced cancers, including those caused by asbestos, virus-related cancers or cancers of viral origin (for example, human papillomavirus (HPV-related or -origin tumors)), and hematological malignancies derived from either of the two major blood cell lineages, i.e., myeloid cell lines (which produce granulocytes, erythrocytes, platelets, macrophages, and mast cells) or lymphoid cell lines (which produce B, T, NK, and plasma cells), such as all types of leukemia, lymphoma, and myeloma, for example, acute, chronic, lymphocytic and/or myeloid leukemia, such as acute leukemia (ALL), acute myeloid leukemia (AML), chronic lymphocytic leukemia (CLL) and chronic myeloid leukemia (CML), undifferentiated AML (MO), myeloblastic leukemia (M1), myeloblastic leukemia (M2; with cell maturation), promyelocytic leukemia (M3 or M3 variant [M3V]), myelomonocytic leukemia (M4 or M4 variant with eosinophilia [M4E]), monocytic leukemia (M5), erythroleukemia (M6), megakaryoblastic leukemia (M7), isolated granulocytic sarcoma, and chloroma; lymphoma, such as Hodgkin's lymphoma (HL), non-Hodgkin's lymphoma (NHL), B cell hematological malignancies, for example B-cell lymphoma, T-cell lymphoma, lymphoplasmacytic lymphoma, monocytic B-cell lymphoma, mucosa-associated lymphoid tissue (MALT) lymphoma, anaplastic (for example, Ki 1+) large-cell lymphoma, adult T-cell lymphoma/leukemia, mantle cell lymphoma, angioimmunoblastic T-cell lymphoma, vascular central lymphoma, intestinal T-cell lymphoma, primary mediastinal B-cell lymphoma, precursor T-lymphoblastic lymphoma, T-lymphoblastic; and lymphoma/leukemia (T-Lbly/TALL), peripheral T-cell lymphoma, lymphoblastic lymphoma, post-transplant lymphoproliferative disorder, true histiocytic lymphoma, primary effusion lymphoma, B-cell lymphoma, lymphoblastic lymphoma (LBL), hematopoietic tumors of the lymphoid lineage, acute lymphoblastic leukemia, diffuse large B-cell lymphoma, Burkitt lymphoma, follicular lymphoma, diffuse histiocytic lymphoma (DHL), immunoblastic large cell lymphoma, precursor B-lymphoblastic lymphoma, cutaneous T-cell lymphoma (CTLC) (also referred to as mycosis fungoides or Sezary syndrome), and lymphoplasmacytic lymphoma (LPL) with Waldenstrom macroglobulinemia; myeloma, such as IgG myeloma, light chain myeloma, nonsecretory myeloma, asymptomatic multiple myeloma (smoldering myeloma) (also referred to as indolent myeloma), solitary plasmocytoma, and multiple myeloma, chronic lymphocytic leukemia (CLL), pilocytic lymphoma; tumors of mesenchymal origin, including hematopoietic tumors of the myeloid lineage, fibrosarcoma, and rhabdomyosarcoma; seminomas, teratocarcinomas, tumors of mesenchymal origin, including fibrosarcoma, rhabdomyosarcoma, and osteosarcoma; and other tumors, including melanoma, xeroderma pigmentosum, keratoacanthoma, seminoma, thyroid follicular cancer, and teratocarcinoma, hematopoietic tumors of the lymphoid lineage, including, but not limited to, T-cells and T-cell neoplasms, including T-cell disorders such as T-prolymphocytic leukemia (T-PLL), including small cell and cerebral cell types; T-cell large granular lymphocytic leukemia (LGL); a/d T-NHL hepatosplenic lymphoma; peripheral/post-thymic T cell lymphoma (pleomorphic and immunoblastic subtypes); angiocentric (nasal) T-cell lymphoma; cancer of the head or neck, renal cancer, rectal cancer, thyroid cancer; acute myeloid lymphoma, and any combination thereof.

According to one embodiment of the present invention, the cancer is one or more cancers selected from the group consisting of esophageal cancer, gastric cancer, GIST, large intestine cancer, rectal cancer, lung cancer, especially SCLC, breast cancer, mast cell tumor and leukemia, especially AML.

In some embodiments, the method disclosed herein may also be used to treat metastatic cancer, unresectable, refractory cancer (for example, cancer that is resistant to conventional cancer therapy, for example, immunotherapy, for example, therapy with a blocking PD-(L) 1 antibody), and/or recurrent cancer. In certain embodiments, the method disclosed herein may be used to treat recurrent cancer.

In some embodiments, the method disclosed herein includes conventional cancer therapy including chemotherapy. For example, it may be administered in combination with imatinib and other c-KIT pathway inhibitors.

In some embodiments, the method disclosed herein effectively increases the duration of survival of a subject (for example, affected by a tumor). For example, the duration of survival of a subject is increased by at least about 1 month, at least about 2 months, at least about 3 months, at least about 4 months, at least about 5 months, at least about 6 months, at least about 7 months, at least about 8 months, at least about 9 months, at least about 10 months, at least about 11 months, or at least about 1 year or more compared to a reference subject (for example, another subject not treated with the composition disclosed herein, for example the antibody-drug conjugate). In another embodiment, the method disclosed herein increases the duration of survival of a subject to a level (approximately 1 month longer, about 2 months longer, about 3 months longer, about 4 months longer, about 5 months longer, about 6 months longer, about 7 months longer, about 8 months longer, about 9 months longer, about 10 months longer, about 11 months longer, or about 1 year longer) that is longer than the duration of survival of a reference subject (for example, another subject not treated with the composition disclosed herein, for example the antibody-drug conjugate).

In some embodiments, the method of the present invention increases the duration of disease progression-free survival of a subject. For example, the duration of disease progression-free survival of a subject is increased by at least about 1 months, at least about 2 months, at least about 3 months, at least about 4 months, at least about 5 months, at least about 6 months, at least about 7 months, at least about 8 months, at least about 9 months, at least about 10 months, at least about 11 months, or at least about 1 year compared to a reference subject (for example, another subject not treated with the composition disclosed herein, for example the antibody-drug conjugate).

In some embodiments, the method disclosed herein effectively increases the rate of response in a group of subjects. For example, the rate of response in a group of subjects is increased by at least about 2%, at least about 3%, at least about 4%, at least about 5%, at least about 10%, at least about 15%, at least about 20%, at least about 25%, at least about 30%, at least about 35%, at least about 40%, at least about 45%, at least about 50%, at least about 55%, at least about 60%, at least about 70%, at least about 75%, at least about 80%, at least about 85%, at least about 90%, at least about 95%, at least about 99%, or at least about 100% compared to a reference subject (for example, another subject not treated with the composition disclosed herein, for example the antibody-drug conjugate).

In some embodiments, a subject to be treated in the present method is a non-human animal, such as a rat or mouse. In some embodiments, a subject to be treated in the present method is a human.

The present invention also comprises a method for treating cancer in a subject in combination with other cancer agents. In some embodiments, the antibody-drug conjugate useful in the method of the present invention may be administered in combination therapy, that is, in combination with at least one other anticancer agent and/or immunomodulatory agent, such as a T-cell stimulating (for example, activating) agent. In some embodiments, the antibody-drug conjugate useful in the method of the present invention may be administered in combination with other compounds, drugs, and/or agents used in the treatment of cancer. Such compounds, drugs, and/or agents may include, for example, chemotherapy drugs, small molecule drugs, or antibodies that stimulate an immune response against cancer. In some embodiments, the method disclosed herein is used in combination with standard treatments (for example, surgery, radiation, and chemotherapy). In other embodiments, the method disclosed herein is used as maintenance therapy, for example, therapy intended to prevent the development or recurrence of a tumor.

In some embodiments, the antibody-drug conjugate disclosed herein may be used in combination with one or more additional cancer agents, including immunotherapy, chemotherapy, targeted therapy, radiotherapy (radiation therapy), or any combination thereof.

Disclosed herein is a composition comprising the antibody-drug conjugate disclosed herein, having the desired degree of purity in a physiologically acceptable carrier, excipient, or stabilizer (Remington's Pharmaceutical Sciences (1990) Mack Publishing Co., Easton, PA). The acceptable carrier, excipient, or stabilizer is nontoxic to the recipient at the

dosage and concentration employed, and includes buffers such as phosphate, citrate, and other organic acids; antioxidants including ascorbic acid and methionine; preservatives (for example, octadecyl dimethyl benzyl ammonium chloride; hexamethonium chloride; benzalkonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins such as serum albumin, gelatin, or immunoglobulin; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose, or sorbitol; salt-forming counter-ions such as sodium; metal complexes (for example, Zn-protein complexes); and/or non-ion surfactants such as TWEEN®, PLURONICS® or polyethylene glycol (PEG).

In some embodiments, a pharmaceutical composition comprises the antibody-drug conjugate disclosed herein in a pharmaceutically acceptable carrier. In certain embodiments, a pharmaceutical composition comprises an effective amount of the antibody-drug conjugate disclosed herein, and optionally one or more additional prophylactic or therapeutic agents, in a pharmaceutically acceptable carrier. In some embodiments, the antibody-drug conjugate is the only active ingredient included in the pharmaceutical composition. The pharmaceutical composition disclosed herein may be useful in reducing c-Kit activity, thereby treating cancer.

Pharmaceutically acceptable carriers used in parenteral preparations include aqueous vehicles, non-aqueous vehicles, antibacterial agents, isotonic agents, buffers, antioxidants, local anesthetics, suspending and dispersing agents, emulsifying agents, sequestering or chelating agents of metal ions, and other pharmaceutically acceptable substances. Examples of aqueous vehicles include sodium chloride injection, Ringer's injection, isotonic dextrose injection, sterile water injection, dextrose and lactate Ringer's injection. Non-aqueous vehicles include fixed oils of vegetable origin, cottonseed oil, corn oil, sesame oil, and peanut oil. Antibacterial agents in a bacteriostatic or fungistatic concentration may be added to parenteral preparations packaged in multi-dose containers, including phenol or cresol, mercury-containing substances, benzyl alcohol, chlorobutanol, methyl and propyl p-hydroxybenzoic acid ester, thimerosal, benzalkonium chloride and benzethonium chloride. Isotonic agents include sodium chloride and dextrose. Buffers include phosphate and citrate. Antioxidants include sodium bisulfate. Local anesthetics include procaine hydrochloride. Suspending and dispersing agents include sodium carboxymethyl cellulose, hydroxypropyl methylcellulose, and polyvinylpyrrolidone. Emulsifiers include polysorbate 80 (TWEEN® 80). Sequestering or chelating agents for metal ions include EDTA. Pharmaceutical carriers also include ethyl alcohol, polyethylene glycol and propylene glycol for water-miscible vehicles, and include sodium hydroxide, hydrochloric acid, citric acid or lactic acid for pH adjustment.

The pharmaceutical composition may be formulated to suit any route of administration to a subject. Specific examples of routes of administration include intranasal, oral, parenteral, intrathecal, intracerebroventricular, pulmonary, subcutaneous, or intraventricular route. Parenteral administration, which is characterized by subcutaneous, intramuscular or intravenous injection, is also contemplated. Injectables may be prepared in conventional forms, as liquid solutions or suspensions, solid forms suitable for being brought into solution or suspension in a liquid prior to injection, or emulsions. Injectables, solutions and emulsions also contain one or more excipients. Suitable excipients are, for example, water, saline, dextrose, glycerol or ethanol. In addition, if desired, the pharmaceutical composition to be administered may also contain small amounts of non-toxic auxiliary substances, such as wetting agents or emulsifying agents, pH buffering agents, stabilizers, solubility enhancers, and other agents such as sodium acetate, sorbitan monolaurate, triethanolamine oleate and cyclodextrin.

Preparations for parenteral administration of the antibody-drug conjugate include sterile ready-to-injectable solutions, sterile dry soluble products that may be combined with a solvent immediately prior to use including tablets for subcutaneous injection, such as lyophilized powders, sterile ready-to-injectable suspensions, sterile dry insoluble products and sterile emulsions that may be combined with the vehicle immediately prior to use. Solutions may be aqueous or non-aqueous.

The antibody-drug conjugate disclosed herein or a pharmaceutically acceptable salt thereof may also be formulated to target specific tissues, receptors, or other body regions of the subject to be treated. A variety of targeting methods are known to those of ordinary skill in the art. Such targeting methods are contemplated for use in the present compositions. For non-limiting examples of targeting methods, see US Patent Nos. 6,316,652, 6,274,552, 6,271,359, 6,253,872, 6,139,865, 6,131,570, 6,120,751, 6,071,495, 6,060,082, 6,048,736, 6,039,975, 6,004,534, 5,985,307, 5,972,366, 5,900,252, 5,840,674, 5,759,542, and 5,709,874. In certain embodiments, the antibody-drug conjugate disclosed herein or a pharmaceutically acceptable salt thereof may be used to treat cancer.

Compositions used for *in vivo* administration may be sterilized. For example, it may be sterilized by filtration through a sterile filtration membrane.

According to another embodiment of the present invention, a composition for diagnosing cancer, an antibody-drug conjugate comprising or a pharmaceutically acceptable salt thereof is provided.

## IX. Kit

Disclosed herein is a kit comprising one or more antibody-drug conjugates disclosed herein or a pharmaceutically acceptable salt thereof. In some embodiments, disclosed herein is a pharmaceutical pack or kit comprising one or more containers filled with one or more of the components of the composition disclosed herein, comprising one or more antibody-drug conjugates disclosed herein or a pharmaceutically acceptable salt thereof, and optional instructions for use. In some embodiments, the kit contains the pharmaceutical composition disclosed herein and any prophylactic or therapeutic agent disclosed herein. In some embodiments, the kit is disclosed as a composition for diagnosing cancer, comprising the composition disclosed herein and instructions for detection and/or diagnostic methods.

In some embodiments, there is provided a method of providing information for the diagnosis of cancer, comprising: treating a sample isolated from a subject with an antibody-drug conjugate or a pharmaceutically acceptable salt thereof.

## X. Production method

Disclosed herein is a method of producing one or more anti-c-Kit antibody-drug conjugates disclosed herein.

The conjugate of the present disclosure may be prepared by any method known in the art, such as those described in US Patent Nos. 7,811,572, 6,411,163, 7,368,565, and 8,163,888, and US Patent Application Publication Nos. 2011/0003969, 2011/0166319, 2012/0253021 and 2012/0259100. The entire teachings of these patent and patent application publications are incorporated herein by reference.

In addition, a method for conjugating various types of cytotoxins, linkers, and drugs to antibodies are known in the art, and see, for example, literatures (Saito et al., Adv. Drug Deliv. Rev. 55:199-215(2003); Trail et al., Cancer Immunol. Immunother. 52:328-337(2003); Payne, Cancer Cell 3:207-212(2003); Allen, Nat. Rev. Cancer 2:750-763(2002); Pastan and Kreitman, Curr. Opin. Investig. Drugs 3:1089-1091(2002); Senter and Springer, Adv. Drug Deliv. Rev. 53:247-264(2001)].

In some embodiments, the production method comprises: conjugating $(L_1)_m$-$(S)_n$-$(L_2)_p$-$(D)_q$ to an antibody or an antigen-binding fragment thereof (Ab) that specifically binds to human c-Kit:
in the formula,

$L_1$ is a linker connecting Ab and S or Ab and $L_2$;
S is a spacer in a form in which a polymer is bound or not bound;
$L_2$ is a cleavable linker;
D is a drug moiety;
m is an integer from 0 to 8;
n is an integer from 0 to 8;
p is an integer from 1 to 8; and
q is an integer from 1 to 8.

The definitions and related explanations of the above terms are the same as those described in I. Definition and II. Antibody-drug conjugate (ADC).

In some embodiments, the production method is a method for producing an anti-c-Kit antibody-drug conjugate, comprising: conjugating a linker (L)-drug (D) to an antibody or an antigen-binding fragment thereof comprising the heavy chain CDR1 of SEQ ID NO: 1, the heavy chain CDR2 of SEQ ID NO: 2, the heavy chain CDR3 of SEQ ID NO: 3, the light chain CDR1 of SEQ ID NO: 4, the light chain CDR2 of SEQ ID NO: 5, and the light chain CDR3 of SEQ ID NO: 6, wherein L comprises a cleavable linker.

In some embodiments, D is a microtubule inhibitor moiety.

In some embodiments, m is 1 to 4, n is 1 to 4, p is 1 to 4, and q is 1 to 8.

### Effects of Invention

The features and advantages of the present invention are summarized as follows:

(i) The present invention provides an antibody-drug conjugate comprising an antibody that specifically binds to human c-Kit, a cleavable linker, and a microtubule inhibitor moiety.
(ii) In addition, the present invention provides a pharmaceutical composition for preventing or treating cancer, comprising an antibody-drug conjugate as an active ingredient.
(iii) The antibody-drug conjugate of the present invention has the characteristic of inducing complete remission by possessing potent anticancer activity against c-Kit-positive and -negative cancer cell lines and/or imatinib-resistant

cancer cell lines.

## Brief Description of Drawings

Figure 1 shows a method for producing an ADC using a cleavable linker. Figure 1a shows the linker-drug used in one embodiment, and Figure 1b shows a schematic manufacturing process for ThioBridge®-Glu-(Val-Cit-PAB-MMAE)-PEG(24u).

Figure 2 shows the results obtained by performing the *in vitro* cell viability analysis in order to confirm the efficacy of the ADC of the present invention in c-Kit-positive and - negative cancer cell lines.

Figure 3 shows the results obtained by confirming the *in vivo* efficacy of the ADC of the present invention against GIST in mice (GIST T1 cells) (Figure 3a shows the results for PoC-DM1; Figure 3b shows the results for 003-1, 2G4 ThioBridge®-Glu-(Val-Cit-PAB-MMAE)-PEG(24u) DAR 2; Figure 3c shows the results for 004-2, 2G4 ThioBridge®-Glu-(Val-Cit-PAB-MMAE)-PEG(24u) DAR 4; and Figure 3d shows the results for 003-2, 2G4-MC-Val-Cit-PAB-MMAE).

Figure 4 shows the results obtained by confirming the *in vivo* efficacy of the ADC of the present invention against GIST in mice (GIST-430/654 cells). (Figure 4a shows the results for PoC-DM1; Figure 4b shows the results for 003-1, 2G4 ThioBridge®-Glu-(Val-Cit-PAB-MMAE)-PEG(24u) DAR 2; Figure 4c shows the results for 004-2, 2G4 ThioBridge®-Glu-(Val-Cit-PAB-MMAE)-PEG(24u) DAR 4; Figure 4d shows the results for 003-2, 2G4-MC-Val-Cit-PAB-MMAE; and Figure 4e shows the results for 007-1, 2G4-ThioBridge®-Glu-[(Val-Cit-PAB-SN-38)]2-Glu-[PEG(24u)]2).

Figure 5 shows the results obtained by confirming the *in vivo* efficacy of the ADC of the present invention against SCLC in mice (SCLC-H526 cells) (Figure 5a shows the results for PoC-DM1; Figure 5b shows the results for 003-1, 2G4 ThioBridge®-Glu-(Val-Cit-PAB-MMAE)-PEG(24u) DAR 2; Figure 5c shows the results for 004-2, 2G4 ThioBridge®-Glu-(Val-Cit-PAB-MMAE)-PEG(24u) DAR 4; and Figure 5d shows the results for 003-2, 2G4-MC-Val-Cit-PAB-MMAE).

Figure 6 shows the results obtained by confirming the *in vivo* efficacy of the ADC of the present invention against mast cell tumor in mice (HMC 1.2 cells).

Figure 7 shows the results obtained by confirming the efficacy of the ADC of the present invention upon re-inoculation of tumor.

Figure 8 shows the results obtained by confirming the *in vivo* efficacy of the ADC of the present invention against AML in mice (Kasumi-1 cells).

Figure 9 shows the results obtained by confirming the *in vivo* efficacy of the ADC of the present invention against breast cancer in mice (MDA-MB-468 cells) (Figure 9a shows the results for PoC-DM1; Figure 9b shows the results for 003-1, 2G4 ThioBridge®-Glu-(Val-Cit-PAB-MMAE)-PEG(24u) DAR 2; Figure 9c shows the results for 004-2, 2G4 ThioBridge®-Glu-(Val-Cit-PAB-MMAE)-PEG(24u) DAR 4; and Figure 9d shows the results for 003-2, 2G4-MC-Val-Cit-PAB-MMAE).

Figure 10 shows the results of re-challenge analysis of the ADC of the present invention against SCLC in mice (SCLC-H526 cells).

Figure 11 shows the results of re-challenge analysis of the ADC of the present invention against mast cell tumor in mice (HMC 1.2 cells).

Figure 12 shows the results obtained by performing the *in vivo* pre-toxicity analysis of the ADC of the present invention when administered at a high concentration (10 mg/kg).

Figure 13 shows the results obtained by performing the *in vivo* pre-toxicity analysis of the ADC of the present invention when administered at a higher concentration (20 / 40 / 60 mg/kg) (Figure 13a shows the results for 2G4 antibody; Figure 13b shows the results for PoC-DM1; Figure 13c shows the results for 003-1, 2G4 ThioBridge®-Glu-(Val-Cit-PAB-MMAE)-PEG(24u) DAR 2; and Figure 13d shows the results for 004-2, 2G4 ThioBridge®-Glu-(Val-Cit-PAB-MMAE)-PEG(24u) DAR 4).

Figure 14 shows the docking model of 2G4 Fab:c-Kit. (Figure 14a) It summarizes c-Kit mutants. (Figures 14b and 14c) c-Kit wild type and mutant proteins were purified, serially diluted, and then coated on a 96-well plate. Binding of 2G4 was examined by ELISA. (Figure 14d) It shows the structural model of the 2G4 Fab:c-Kit complex with the highest score calculated by HADDOCK. The heavy chain (H) variable (V) and constant (C) regions and light chain (L) variable (V) and constant (C) regions of 2G4 were designated as VH, CH1, VL, and CL, respectively. (Figure 14e) It shows a stick representation of the interface between 2G4 and c-Kit. Residues in the D2 and D3 regions of c-Kit are each indicated by shaded bars.

## Mode for Carrying out the Invention

Hereinafter, the present invention will be described in more detail through examples. These examples are only for illustrating the present invention in more detail, and it will be apparent to those of ordinary skill in the art that the scope of the present invention is not limited by these examples according to the gist of the present invention.

## Example

### Preparation Example

### Preparation Example 1. Construction of anti-c-Kit antibody 2G4

#### Preparation Example 1-1. Construction of immunized mouse

50 μg (per mouse) of recombinant c-Kit protein (cat# PKSH030939) purchased from Elabscience was mixed with an equal volume of complete Freund's adjuvant (Sigma, USA) to prepare an emulsion. The emulsion prepared as above was injected into the abdominal cavity of six 7-week-old female humanized NSG mice constructed by human CD34+ cell injection. 50 μg of antigen was injected into each mouse in a total volume of 500 μl. After 1 and 2 weeks, an emulsion mixed with incomplete Freund's adjuvant (Sigma, USA) and the antigen was additionally injected into the abdominal cavity of the mice.

#### Preparation Example 1-2. Confirmation of antibody production

Blood was collected from the eyes of mice immunized using the above method, placed in a 1.5 ml microcentrifuge tube, and then centrifuged at 13,000 rpm for 10 minutes. Serum was separated and stored at -20 °C until an experiment to confirm antibody production was performed. An enzyme immunoassay using the antigen protein was performed to confirm antibody production, and then an emulsion mixed with incomplete Freund's adjuvant (Sigma, USA) and the antigen was injected into the abdominal cavity of the mice once again 3 days before cell fusion.

#### Preparation Example 1-3. Preparation of hybridoma

After confirming antibody production, the mouse was sacrificed. Splenocytes were isolated and fused with myeloma cells P3X63Ag 8.653 (ATCC CRL-1580) to prepare a hybridoma.

Specifically, the mouse P3X63Ag 8.653 cells were cultured in a culture plate using RPMI1640 medium supplemented with 10% fetal bovine serum. In order to perform cell fusion, P3X63Ag 8.653 cells were washed twice with serum-free RPMI1640 medium (Hyclone, USA) and adjusted to a cell concentration of $1 \times 10^7$. The mouse was sacrificed by cervical dislocation, the spleen was collected, then placed in a mesh container (Sigma, USA), and the cells were isolated. A suspension of splenocytes was prepared, and then the suspension was washed using centrifugation. The splenocyte solution was exposed to Tris-$NH_4Cl$ (TRIS 20.6 g/L, $NH_4Cl$ 8.3 g/L) to lyse red blood cells. Completely isolated antibody-producing cells were centrifuged at 400 × g for 5 minutes. Thereafter, the cells were washed twice in serum-free medium and resuspended in 10 ml of medium. Lymphocytes were counted using a hemocytometer, and $1 \times 10^8$ lymphocytes were mixed with 1 × 10 P3X63Ag 8.653 cells (10:1) in serum-free medium.

After centrifugation at 400 × g for 5 minutes, 1 ml solution was added dropwise using 50% (M/V) polyethylene glycol 1500 (Sigma, USA) warmed at 37 °C and mixed for 1 minute. The fusion mixture solution prepared as above was diluted with serum-free RPMI1640 and centrifuged at 400 × g for 3 minutes. The cells were suspended in 35 ml of RPMI1640 selection medium supplemented with 20% fetal bovine serum and HAT (100 μM hypoxanthine, 0.4 μM aminopterin, 16 μM thymidine). 100 μl of the suspension was loaded onto a 96-well plate coated with feeder cells (macrophages isolated from the abdominal cavity using RPMI1640) 1 day ago, and cultured at 37 °C 5% $CO_2$. After 5 days, the HAT medium was replaced every 2 to 3 days, and the cells were cultured for 14 days. After 14 days, secondary culture was performed by replacing the medium with RPMI1640 medium supplemented with 20% fetal bovine serum and HT (medium excluding 0.4 μM aminopterin from HAT).

#### Preparation Example 1-4. Selection and isolation of antibody-producing fused cells

The supernatant of the fused cell culture solution prepared as above was collected, and enzyme immunoassay was performed to determine whether antibodies specific for the prepared antigen were produced. A culture solution of the fused cells that showed an appropriate concentration of more than 4 times that of the negative control was selected, transferred to a 24-well plate, and cultured. Additionally, the culture solution was diluted (limiting dilution) to include 1 cell

per well in a 96-well plate and cultured, and then the culture solution was recovered, and the 96-well plate was coated with 0.1 μg of c-KIT protein, used as an antigen, per well. Thereafter, enzyme immunoassay was performed to finally select the fused cells producing 15 monoclonal antibodies (1C6, 1H2, 1A6, AFA, 2B3, 2G4, 4G5, 4C4, 4C7, 4D7, 1E1, 2H6, 1G3, 1A3, 1D3).

**Preparation Example 1-5. Selection of anti-c-KIT antibody**

SPR (Surface Plasmon Resonance) was performed in order to confirm the c-KIT binding ability of the selected antibodies. Using SR7500DC (Reichert, USA), 20 μg of human c-Kit (elabscience, PKSH030939), 20 μg of mouse c-Kit (SB, Lot# LC05DE2304), and 20 μg of rat c-Kit (SB, Lot# LC06SE1787), which were used for antibody construction, were immobilized on a PEG (Reichert, USA) chip. Thereafter, the antibodies were flowed at different concentrations, and then the KD value, which is the affinity for c-Kit, was analyzed using the Scrubber2 program. The KD value is calculated by dividing kd by ka, and the lower the value, the greater the binding ability to the target.

As a result, the 2G4 antibody showed potent affinity for human c-Kit, with a KD value of about 2.8237 ($\pm$ 0.9) $\times$ $10^{-12}$ M. The affinity for humans was highest, followed by mice and rats.

The CDR sequences of the selected 2G4 antibodies are shown in Table 1 below:

[Table 1]

| CDR | Sequence | SEQ ID NO |
|---|---|---|
| H-CDR1 | GFTFSRYG | SEQ ID NO: 1 |
| H-CDR2 | IWYDGTNK | SEQ ID NO: 2 |
| H-CDR3 | AREDWAEAFD M | SEQ ID NO: 3 |
| L-CDR1 | QSLLHSNGYN Y | SEQ ID NO: 4 |
| L-CDR2 | LGS | SEQ ID NO: 5 |
| L-CDR3 | MQALQTIT | SEQ ID NO: 6 |

The sequences of the heavy chain variable region and light chain variable region of the 2G4 antibody are shown in Table 2 below:

[Table 2]

| Variable region | Sequence | SEQ ID NO |
|---|---|---|
| heavy chain | QVQLVESGGG VVQPGRSLRL SCAASGFTFS RYGMHWVRQA PGKGLEWVAV IWYDGTNKDY TDSVRGRFTI SRDNSKNTLY LQMNSLRAED TAVYYCARED WAEAFDMWGQ GTTVTVSS | SEQ ID NO: 7 |
| light chain | DIVMTQSPLS LPVTPGEPAS ISCRSSQSLL HSNGYNYLDW YLQKPGQSPQ LLIYLGSNRA SGVPDRFSGS GSGTDFTLKI SRVEAEDVGV YYCMQALQTI TFGQGTRLEI K | SEQ ID NO: 8 |

**Preparation Example 2. Preparation of "2G4-SMCC-DM1" (#POC-DM1)**

2G4-ADC (antibody-drug conjugate) comprising SMCC (*N*-succinimidyl-4-(*N*-maleimidomethyl) cyclohexane-1-carboxylate) as a non-cleavable linker and DM1 (*N*(2')-deacetyl-*N*(2')-(3-mercapto-1-oxopropyl)-maytansine) as a microtubule inhibitor was prepared. Conjugation of the linker-payload to 2G4 was measured using a UV-Vis spectrophotometer, and the drug-to-antibody ratio (DAR) was determined via LC-MS analysis.

Specifically, 2G4 and human IgG1 (Sino Biological) were dialyzed against conjugation buffer (0.1M sodium phosphate and 0.15 M NaCl, pH 7.2) and conjugated with SMCC-DM1 (MedChemExpress) at a molar ratio of 1:5 or 1:10 for 1.5 hours at RT. Fractionation was performed to remove unbound SMCC-DM1 and aggregated 2G4-DM1. The

antibody fractions were pooled and dialyzed against a formulation buffer (10 mM sodium succinate, 0.05% polysorbate 20, and 6% sucrose; pH 5.0). Conjugation was determined by examining the optical density (OD) at 280 nm and 252 nm using a SPECTROstar Nano (BMG LABTECH). DAR was determined using liquid chromatography-tandem mass spectrometry (LC-MS) analysis.

In order to confirm the conjugation of DM1 and 2G4, since DM1 absorbs ultraviolet light at 252 nm, the absorbance of naked 2G4 and ADC at 252 nm was analyzed using a UV-Vis spectrophotometer, and the absorbance of naked 2G4 and 2G4-DM1 were compared. The absorbance of 2G4-DM1 was higher than that of 2G4 at 252 nm. The results indicated that DM1 was potentially conjugated to 2G4.

Through mass spectrometry of 2G4-DM1, it was confirmed that it consists of various populations with a drug range from 1 to 4 per antibody, and the average DAR of 2G4-DM1 was 1.8.

**Preparation Example 3. Preparation of ADC using cleavable linker**

Four types of ADCs were prepared using cleavable linkers. Specifically, ADCs were prepared by classical maleimide conjugation or ThioBridge® (disulfide rebridging conjugation technology, Abzena, USA) to couple MMAE and SN-38, and an average DAR range was between 2 and 8. ThioBridge® MMAE ADCs with an average DAR ratio of 2 and 4 were prepared along with maleimide MMAE control ADCs with an average DAR of 4. The average DAR of the ThioBridge® SN-38 ADC was 8. The cathepsin cleavable 'Val-Cit-PAB' motif was included in all linkers to allow lysosomal release of the payload (Figure 1a). In the case of the ThioBridge® SN-38 ADC, the carbonate bond connecting SN-38 to the linker is sensitive to hydrolysis, also enabling a pH-based payload release mechanism. Taking subsequent biological tests into account, ADC with high monomer purity (>97%) was prepared in an amount of 29-56 mg. The manufacturing process of ThioBridge®-Glu-(Val-Cit-PAB-MMAE)-PEG(24u) is shown in Figure 1b.

**Preparation Example 3-1. Preparation of 2G4-MC-VC-PAB-MMAE**

13.38 mg/mL of mAb 2G4 in Dulbecco's PBS, pH 7.4, 5 mM EDTA (5.232 mL; 70.0 mg; 470 nmol; 1.0 equivalent) was diluted in Dulbecco's PBS, pH 7.4, 5 mM EDTA (8.505 mL). A 5 mM solution of TCEP dissolved in endotoxin-free water (263.4 μL, 1317 nmol, 2.8 equivalent) was added to the diluted mAb 2G4 solution. Reduction was allowed to proceed for 2 hours at 40 °C with a final antibody concentration of 5.0 mg/mL.

After 2 hours at 40 °C, the reduction mixture was diluted in Dulbecco's PBS, pH 7.4, 5 mM EDTA (2.450 mL), cooled to 22 °C, and then further diluted with DMF (177 μL). A4.26 mg/mL (3.23 mM) solution of MC-VC-PAB-MMAE in DMF was prepared by dissolving 3.95 mg (3.00 μmol) of MC-VC-PAB-MMAE (MW = 1317 g.mol $^{-1}$) in 927 μL of DMF. A MC-VC-PAB-MMAE solution (873 μL; 3.72 mg; 2823 nmol; 6.0 equivalent) in DMF was added to the reduced mAb 2G4 solution to a final concentration of 6% DMF and a final antibody concentration of 4.0 mg/mL. The conjugation reaction was allowed to proceed for 1 hour at 22 °C.

After 1 hour at 22 °C, the reaction mixture was subjected to buffer exchange by spin filtration using a Zebaspin column (7 kDa MWCO; 10 mL) equilibrated with 20 mM histidine, 50 mM NaCl, 5% sucrose, pH 6.5 according to the manufacturer's instructions. The recovered conjugate sample was further triturated and concentrated to 7.13 mg/mL by ultrafiltration/diafiltration using a Vivaspin 20 centrifugal concentrator (PES membrane, 30 kDa MWCO) equilibrated with 20 mM histidine, 50 mM NaCl, 5% sucrose, pH 6.5. The concentrated conjugate sample (56.3 mg; 7.90 mL) was sterile filtered through a PVDF membrane filter with a pore size of 0.22 μm.

The antibody-drug conjugate obtained through this was designated as "2G4-MC-VC-PAB-MMAE", and the conjugate was characterized by HIC and SEC. 56 mg of 2G4-MC-VC-PAB-MMAE ADC was isolated with high monomer content (>98%, SEC) and an average DAR of 4.2 (HIC) (hereinafter referred to as 003-2).

**Preparation Example 3-2. Preparation of 2G4-ThioBridge® -Glu-(Val-Cit-PAB-MMAE)-PEG(24u) DAR 2**

14.77 mg/mL of mAb 2G4 in Dulbecco's PBS, pH 7.4, 5 mM EDTA (2.180 mL; 32.2 mg; 216 nmol; 1.0 equivalent) was diluted in Dulbecco's PBS, pH 7.4, 5 mM EDTA (4.148 mL). A 5 mM solution of TCEP dissolved in endotoxin-free water (112 μL, 562 nmol, 2.6 equivalent) was added to the diluted mAb 2G4 solution. Reduction was allowed to proceed for 2 hours at 40 °C with a final antibody concentration of 5.0 mg/mL.

After 2 hours at 40 °C, the reduction mixture was diluted in Dulbecco's PBS, pH 7.4, 5 mM EDTA (805 μL), cooled to 22 °C, and then further diluted with DMF (403 μL). A 4.52 mg/mL (1.61 mM) solution of ThioBridge®-Glu-(Val-Cit-PAB-MMAE)-PEG(24u) in DMF was prepared by dissolving 2.15 mg (768 nmol) of ThioBridge®-Glu-(Val-Cit-PAB-MMAE)-PEG(24u) (MW = 2805.0 g.mol $^{-1}$) in 477 μL of DMF. A ThioBridge®-Glu-(Val-Cit-PAB-MMAE)-PEG(24u) solution (402 μL; 1.82 mg; 648 nmol; 3.0 eq.) in DMF was added to the reduced 2G4 solution to a final concentration of 10% DMF and a final antibody concentration of 4.0 mg/mL. The conjugation reaction was allowed to proceed for 18 hours at 22 °C.

After 18 hours at 22 °C, the reaction mixture was subjected to buffer exchange by spin filtration using a Zebaspin

column (7 kDa MWCO; 10 mL) equilibrated with 20 mM histidine, 50 mM NaCl, 5% sucrose, pH 6.5 according to the manufacturer's instructions. The recovered conjugate sample was further triturated and concentrated to 7.25 mg/mL by ultrafiltration/diafiltration using a Vivaspin 20 centrifugal concentrator (PES membrane, 30 kDa MWCO) equilibrated with 20 mM histidine, 50 mM NaCl, 5% sucrose, pH 6.5. The concentrated conjugate sample (29.7 mg; 4.10 mL) was sterile filtered through a PVDF membrane filter with a pore size of 0.22 μm.

The antibody-drug conjugate obtained through this was designated as "2G4-ThioBridge®-Glu-(Val-Cit-PAB-MMAE)-PEG(24u) DAR 2", and the conjugate was characterized by HIC and SEC. 30 mg of 2G4-ThioBridge®-Glu-(Val-Cit-PAB-MMAE)-PEG(24u) DAR2 ADC was isolated with high monomer content (>97%, SEC) and an average DAR of 2.0 (HIC) (hereinafter referred to as 003-1).

**Preparation Example 3-3. Preparation of 2G4-ThioBridge® -Glu-(Val-Cit-PAB-MMAE)-PEG(24u) DAR 4**

20.71 mg/mL of mAb 2G4 in Dulbecco's PBS, pH 7.4, 5 mM EDTA (5.794 mL, 120 mg, 806 nmol, 1.0 equivalent) was diluted in Dulbecco's PBS, pH 7.4, 5 mM EDTA (17 mL). A 5 mM solution of TCEP dissolved in endotoxin-free water (967.2 μL, 4836 nmol, 6.0 equivalent) was added to the diluted mAb 2G4 solution. Reduction was allowed to proceed for 1 hour at 40 °C with a final antibody concentration of 5.0 mg/mL.

After 1 hour at 40 °C, the reduction mixture was diluted in propylene glycol (6.40 mL) and cooled to 22 °C. A 8.48 mg/mL (3.02 mM) solution of ThioBridge®-Glu-(Val-Cit-PAB-MMAE)-PEG(24u) in DMF was prepared by dissolving 20.60 mg (7345 nmol) of ThioBridge®-Glu-(Val-Cit-PAB-MMAE)-PEG(24u) (MW = 2805.0 g.mol $^{-1}$) in 2430 μL of DMF. A ThioBridge®-Glu-(Val-Cit-PAB-MMAE)-PEG(24u) solution (1600 μL; 13.57 mg; 4836 nmol; 6.0 equivalent) in DMF was added to the reduced 2G4 solution to a final concentration of 5% DMF, 20% propylene glycol and a final antibody concentration of 3.8 mg/mL. The conjugation reaction was allowed to proceed for 18 hours at 22 °C.

After 18 hours at 22 °C, the reaction mixture was diluted in 4 M sodium chloride, 50 mM sodium phosphate pH 7.0 (32.0 mL). The diluted reaction mixture was loaded onto a Proteus FliQ 10 mL column packed with HIC ToyoPearl® Phenyl-650S resin equilibrated with buffer A (buffer of 2.0 M sodium chloride, 50 mM sodium phosphate, pH 7.0). Elution was performed in buffer B (50 mM sodium phosphate, 20% isopropanol, pH 7.0) with 1.5 mL/min of a constant flow and a 200 mL gradient from 0 to 100%. The fractions were analyzed by HIC and SEC and pooled based on a content (>90%) of four DARs. The pooled fractions were subjected to buffer exchange and concentrated to 7.93 mg/mL by ultrafiltration/-diafiltration using a Vivaspin 20 centrifugal concentrator (PES membrane, 30 kDa MWCO) equilibrated with 20 mM histidine, 50 mM NaCl, 5% sucrose, pH 6.5. The concentrated conjugate sample (47.4 mg; 5.98 mL) was sterile filtered through a PVDF membrane filter with a pore size of 0.22 μm.

The antibody-drug conjugate obtained through this was designated as "2G4-ThioBridge®-Glu-(Val-Cit-PAB-MMAE)-PEG(24u) DAR 4 conjugate", and the conjugate was characterized by HIC and SEC. 47 mg of 2G4-ThioBridge®-Glu-(Val-Cit-PAB-MMAE)-PEG(24u) DAR4 ADC was isolated with high monomer content (>98%, SEC) and an average DAR of 4.0 (HIC) (hereinafter referred to as 004-2).

**Preparation Example 3-4. Preparation of 2G4-ThioBridge® -Glu-[(Val-Cit-PAB-SN-38)]2-Glu-[PEG(24u)]2**

18.71 mg/mL of mAb 2G4 in Dulbecco's PBS, pH 7.4, 5 mM EDTA (3.474 mL, 65.0 mg, 437 nmol, 1.0 equivalent) was diluted in Dulbecco's PBS, pH 7.4, 5 mM EDTA (9.00 mL). A 5 mM solution of TCEP dissolved in endotoxin-free water (524.0 μL; 2620 nmol, 6.0 equivalent) was added to the diluted mAb 2G4 solution. Reduction was allowed to proceed for 1 hour at 40 °C with a final antibody concentration of 5.0 mg/mL.

After 1 hour at 40 °C, the reduction mixture was diluted in propylene glycol (3.71 mL), cooled to 22 °C, and then further diluted with DMF (655 μL). A 10.0 mg/mL (2.18 mM) solution of ThioBridge®-Glu-[(Val-Cit-PAB-SN-38)]2-Glu-[PEG(24u)]2 in DMF was prepared by dissolving 14.09 mg (3072 nmol) of ThioBridge®-Glu-[(Val-Cit-PAB-SN-38)]2-Glu-[PEG(24u)]2 (MW = 4588.0 g.mol $^{-1}$) in 1409 μL of DMF. A solution (1202 μL; 12.02 mg; 2620 nmol; 6.0 equivalent) of ThioBridge®-Glu-[(Val-Cit-PAB-SN-38)]2-Glu-[PEG(24u)]2 in DMF was added to the reduced 2G4 solution to a final concentration of 10% DMF, 20% propylene glycol and a final antibody concentration of 3.5 mg/mL. The conjugation reaction was allowed to proceed for 18 hours at 22 °C.

After 18 hours at 22 °C, the reaction mixture was diluted in 4 M sodium chloride, 50 mM sodium phosphate pH 7.0 (18.6 mL). The diluted reaction mixture was loaded onto a Proteus FliQ 10 mL column packed with HIC ToyoPearl® Phenyl-650S resin equilibrated with buffer A (2.0 M sodium chloride, 50 mM sodium phosphate, pH 7.0 buffer). Elution was performed in buffer B (50 mM sodium phosphate, 20% isopropanol, pH 7.0) with a constant flow of 1.5 mL/min and a 200 mL gradient from 0 to 100%. The fractions were analyzed by HIC and SEC and pooled based on a content (>80%) of four DARs. The pooled fractions were subjected to buffer exchange and concentrated to 4.01 mg/mL by ultrafiltration/diafiltration using a Vivaspin 20 centrifugal concentrator (PES membrane, 30 kDa MWCO) equilibrated with 20 mM histidine, 50 mM NaCl, 5% sucrose, pH 6.5. The concentrated conjugate sample (29.0 mg; 7.22 mL) was sterile filtered through a PVDF membrane filter with a pore size of 0.22 μm.

The antibody-drug conjugate obtained through this was designated as "2G4-ThioBridge®-Glu-[(Val-Cit-PAB-SN-38)]2-Glu-[PEG(24u)]2 conjugate", and the conjugate was characterized by LC-MS and SEC. 29 mg of 2G4-ThioBridge®-Glu-[(Val-Cit-PAB-SN-38)]2-Glu-[PEG(24u)]2 was isolated with high monomer content (>98%, SEC) and an average DAR of 7.4 (LC-MS) (hereinafter referred to as 007-1).

## Analysis method

### (1) LC-MS analysis

LC-MS analysis was performed using a POROSHELL 300SB C3 column (2.1 × 12.5 mm, 5 μm) coupled to a Waters XEVO G2S TOF mass spectrometer and a Waters Acquity H Class UPLC system. The mobile phase was buffer A (0.1% formic acid in water). A gradient (2.5 min 10% B, 10-80% B gradient 3.5 min) was applied using buffer B (acetonitrile, 0.1% formic acid) at a flow rate of 0.4 mL/min. The column was maintained at 60 °C throughout the analysis. After reduction, maleimide ADC was analyzed (10 mM DTT, 1 hour at 40 °C). All ADCs were analyzed after dilution to 0.2 mg/mL. 10 μL of ADC solution was injected for analysis. The average DAR was calculated as the weighted average of the observed DAR species based on the signal intensity (SI) of the major glycoform in the deconvolved m/z spectrum for non-reduced samples and the signal intensity for light chain (LSI) and heavy chain (HSI) for reduced samples:

$$Average\ DAR_{non-reduced\ sample} = \frac{\sum_{i=0}^{n} SI_i \times i}{\sum_{i=0}^{n} SI_i}$$

$$Average\ DAR_{reduced\ sample} = 2 \times \frac{\sum_{i=0}^{n} LSI_i \times i}{\sum_{i=0}^{n} LSI_i} + 2 \times \frac{\sum_{i=0}^{n} HSI_i \times i}{\sum_{i=0}^{n} HSI_i}$$

(2) SEC analysis

Analytical SEC was performed using an ACQUITY UPLC BEH SEC column (4.6 mm × 15 cm, 200 Å, 1.7 μm) and a guard column (4.6 mm × 3 cm) connected to a Dionex Ultimate 3000 UPLC system. The mobile phase was 0.2 M potassium phosphate buffer, pH 6.8, 0.2 M potassium chloride, and 15% (v/v) isopropanol. The flow rate was maintained constant at 0.35 mL/min. The column was maintained at 30 °C throughout the analysis. The analysis was performed with 10 min isocratic elution with UV detection at 248 nm, 280 nm and 365 nm. 10 μg of ADC was injected for analysis. The percentage of high molecular weight (HMW) species was calculated by comparing the peak area corresponding to HWM species at 280 nm with the total peak area corresponding to both HWM species and monomeric species at 280 nm. The presence of free reagent-related species was evaluated by comparing chromatographic traces of the ADC sample and the buffer sample in the low molecular weight species region of the chromatogram for retention times greater than 6 min at the wavelength corresponding to the absorption maximum of the payload (λ = 248 nm for MMAE, λ = 365 nm for SN-38).

### (3) HIC analysis

Analytical HIC was performed using a TOSOH Bioscience TSKgel Butyl-NPR column (4.6 mm × 3.5 cm, 2.5 μm) coupled to a Dionex Ultimate 3000 UPLC system. The mobile phase was buffer A: 1.5 M ammonium sulfate, 50 mM sodium phosphate, pH 7.0. In order to elute the bound species, a linear gradient (0-100% B in 10.5 min) was applied using buffer B (20% isopropanol, 50 mM sodium phosphate, pH 7.0) at a flow rate of 1.35 mL/min. The column was maintained at °C throughout the analysis. Analysis was performed with UV detection at 280 nm. 10 μg of ADC was injected per assay. The percentage (i) of each DAR species was calculated by comparing the peak area of each assigned peak with the total peak area. The average DAR was calculated as a weighted average of the observed DAR species based on the peak area under the curve (AUCi), and the average molecular weight of the ADC was calculated based on the DAR and linker-payload mass contributions as follows:

$$Average\ DAR = \frac{\sum_{i=0}^{n} AUC_i \times i}{\sum_{i=0}^{n} AUC_i}$$

$$MW_{ADC} = MW_{mAb} + DAR \times MW_{LP}$$

**Example**

**Example 1. *In vitro* cell viability analysis**

In order to confirm the efficacy of the ADCs shown in Table 3 in c-Kit-positive and - negative cancer cell lines, the *in vitro* cell viability analysis was performed.

The cells were inoculated into a 96-well cell culture black plates (Greiner Bio-One, Kremsmuenster, Austria), and the number of cells was scanned with a Celigo Imaging Cytometer device. Nine concentration points of ADC were prepared by 5- or 10-fold serial dilutions with a starting concentration of 40 or 200 μg/ml. After 3-5 days of treatment, the cells were stained with Calcein-AM (1 μg/ml) or Hoechst 33342 (8 μM) fluorescence dye. The wells were scanned and analyzed using a Celigo Imaging Cytometer for viable cell counting. The cell viability was graphically displayed with Graph Pad Prism Software, and half-maximal inhibitory concentration (IC50) values were calculated (Table 4).

[Table 3]

| Name | Seeding number | Max Conc. | Dilution fold | Incubation time |
|---|---|---|---|---|
| GIST-T1 | $5\times10^3$ / well | 40 μg/mL | 10 | 3 days |
| GIST-430/654 | $5\times10^3$ / well | 40 μg/mL | 10 | 4 days |
| MDA-MB-468 | $4\times10^3$ / well | 200 μg/mL | 5 | 3 days |
| NCI-H526 | $7\times10^3$ / well | 200 μg/mL | 5 | 5 days |
| HMC1.2 | $5\times10^3$ / well | 200 μg/mL | 5 | 4 days |
| Kasumi-1 | $2\times10^4$ / well | 200 μg/mL | 5 | 4 days |

[Table 4]

| ADC | Batch Code | IC50 (μg/ml) | | | |
|---|---|---|---|---|---|
| | | GIST-T1 | GIS-T-430/654 | HMC1.2 | MDA-MB-468 |
| 2G4-SMCC-DM1 | POC-DM1 | 0.00451 | 0.0033 | 0.1636 | 1.246 |
| 2G4-MC-Val-Cit-PAB-MMAE | NOVN-1574-JN003-2 | 0.00367 | 0.0058 | 0.005 | 6.256 |
| 2G4-ThioBridge®-Glu-(Val-Cit-PAB-MMAE)-PEG(24u) DAR 2 | NO VN-1614-JN003-1 | 0.00745 | 0.0097 | 0.0097 | 44.92 |
| 2G4-ThioBridge®-Glu-(Val-Cit-PAB-MMAE)-PEG(24u) DAR 4 | NOVN-1574-JN004-2 | 0.00314 | 0.0051 | 0.0054 | 25.84 |
| 2G4-ThioBridge®-Glu-[(Val-Cit-PAB-SN-38)]2-Glu-[PEG(24u)]2 | NOVN-1574-JN007-1 | 0.0589 | 0.0546 | 0.095 | 0.168 |

As shown in Figure 2, it can be seen that the ADC group shows statistically significant efficacy compared to the control.

**Example 2. *In vivo* efficacy against GIST in mice (GIST T1 cells)**

In order to evaluate the efficacy of ADC substances against GIST, an experiment was performed. A GIST-T1 cell (c-Kit mutant and imatinib sensitive; accession number: CVCL_4976; c-Kit mutation site - Exon11 V560-L576) xenograft model was used.

4-week-old female CB-17 SCID immunodeficient mice (Charles River Laboratories Japan, Inc.) were acclimatized under SPF (Specific Pathogen Free) conditions for 12 days before being used in the experiment. GIST-T1 cells ($5 \times 10^6$/100 μL/head/serum free, DMEM/high glucose medium) in 50% Matrigel (Corning, 354248, NY, USA) were transplanted subcutaneously into 6-week-old mice. The total injection volume containing the suspended cells was 200 μL.

The mice with an average tumor volume of about 190 $mm^3$ on day 21 after transplantation were enrolled as day 0. The mice were randomly assigned to one of six groups (n = 5/group), and then the mice were administered with ADC vehicle (5 ml/kg, i.v. tail), each ADC substance (0.5, 1.5 or 3.0 mg/kg, i.v. tail), imatinib (100 mg/kg, p.o.) and ADC substance (3.0 mg/kg, i.v. tail) + imatinib (100 mg/kg, p.o.) (Table 5). As an ADC vehicle, the buffer for PoC-DM1 was a mixture of 10 mM sodium succinate, 6% sucrose, and 0.05% Tween20 (pH 5.0), and the buffer for the others was a mixture of 20 mM histidine (Merck, 104352), 50 mM sodium chloride (Sigma, S3014), 5% sucrose (Sigma, S0389) (pH 6.5), and 0.22 μM filter (Merck S2GPU11RE). Individual doses were calculated based on animal body weight recorded immediately prior to administration with a dose volume of 5 mL/kg body weight.

[Table 5]

| No. | Subject | Interval, Route |
|---|---|---|
| 1 | Control (vehicle, ADC buffer) | D0, D10, D20 (Three times), i.v. |
| 2 | Imatinib 100 mg/kg | D0 - D29 (Daily for 30 days), p.o. |
| 3 | ADC 0.5 mg/kg | D0, D10, D20 (Three times), i.v. |
| 4 | ADC 1.5 mg/kg | D0, D10, D20 (Three times), i.v. |
| 5 | ADC 3.0 mg/kg | D0, D10, D20 (Three times), i.v. |
| 6 | ADC 3.0 mg/kg + Imatinib 100 mg/kg | ADC : D0, D10, D20 (Three times), i.v.<br>Imatinib : D0 - D29 (Daily for 30 days), p.o. |

The administered ADC substances are shown in Table 6:

[Table 6]

| Study # | ADC |
|---|---|
| #PoC-DM1 | 2G4-SMCC-DM1 |
| #003-1 | 2G4-ThioBridge®-Glu-(Val-Cit-PAB-MMAE)-PEG(24u) DAR 2 |
| #004-2 | 2G4-ThioBridge®-Glu-(Val-Cit-PAB-MMAE)-PEG(24u) DAR 4 |
| #003-2 | 2G4-MC-Val-Cit-PAB-MMAE |

All animals were observed once a day for mortality, general condition and clinical signs (time, onset, severity and recovery). Tumor volume and body weight were measured twice a week. In order to measure tumor volume, length, width, and depth were measured using calipers.

The results are shown in Figures 3a to 3d.

During the test period before sacrifice, no abnormal symptoms were observed in any group except the imatinib 100 mg/kg group. In the imatinib 100 mg/kg group, 1 case died on day 91.

No body weight decrease or body weight increase of 10% or more was observed for 56 days in the imatinib 100 mg/kg group and the test ADC study group (0.5 mg/kg, 1.5 mg/kg, 3.0 mg/kg, 3.0 mg/kg + imatinib 100 mg/kg) compared to the control (vehicle). Thereafter, no rapid body weight decrease or rapid body weight increase was observed in any group. This suggests that all of the ADC substances of the present inventors are very safe.

On day 56, the imatinib 100 mg/kg group inhibited tumor growth compared to the control (vehicle), while all ADC study groups at 3.0 mg/kg showed a more potent tumor growth inhibition effect. In particular, the ADC study group of 2G4 ThioBridge®-Glu-(Val-Cit-PAB-MMAE)-PEG(24u) (#003-1 and #004-2) was found to have a significant tumor growth inhibition effect even at 1.5 mg/kg.

Although imatinib alone inhibited the tumor, the tumor recurred after treatment was discontinued. Surprisingly, treatment with each ADC at 3 mg/kg in combination with imatinib induced complete remission of the tumor without regrowth for up to 105 or 112 days even after discontinuation of imatinib administration.

As a result of measuring tumor weight after sacrifice, the imatinib 100 mg/kg administration group showed a decrease of about 59% compared to the control (vehicle), while all ADC study groups administered at 1.5 mg/kg or more (except #PoC-DM1 1.5 mg/kg group) showed higher reduction ability (Table 7). In particular, the ADC study group of 2G4 ThioBridge®-Glu-(Val-Cit-PAB-MMAE)-PEG(24u) (#003-1 and #004-2) showed significant tumor growth inhibition efficacy even in the 1.5 mg/kg group. When each ADC at 3 mg/kg was administered in combination with imatinib, most tumors disappeared.

[Table 7]

| (reduction %) | #PoC-DM1 | #003-1 | #004-2 | #003-2 |
|---|---|---|---|---|
| 0.5 mg/kg | 0.7% | 32.0% | 40.6% | 45.4% |
| 1.5 mg/kg | 20.3% | 85.0% | 98.1% | 61.5% |
| 3.0 mg/kg | 70.0% | 89.8% | 99.2% | 93.8% |
| 3.0 mg/kg + imatinib 100 mg/kg | 98.6% | 98.8% | 99.2% | 94.7% |

The above results support that the ADC group showed statistically significant efficacy compared to the control.

**Example 3: *In vivo* efficacy against GIST in mice (GIST-430/654 cells)**

In particular, in order to evaluate the efficacy of ADC substances against GIST resistant to imatinib, an experiment was also performed. A GIST-430/654 cell (imatinib resistant, Exon 11 V560-L576 + Exon 13 V654A) xenograft model was used.

5-week-old female NOG (NOD/Shi-scid, IL-2RγKO) immunodeficient mice (CIEA Japan, Inc.) were acclimatized under SPF (Specific Pathogen Free) conditions for 12 days before being used in the experiment. GIST-430/654 cells ($5 \times 10^6$/100 μL/head/serum free, IMDM medium) in 50% Matrigel (Corning, 354248, NY, USA) were injected subcutaneously into 6-week-old mice. The total injection volume containing the suspended cells was 200 μL.

The mice were enrolled in this study on day 21 after transplantation, and the average tumor volume was about 180 $mm^3$ (day 0). The mice were randomly assigned to one of five groups (n = 5/group), and then the mice were administered with ADC vehicle (5 ml/kg, i.v. tail), each ADC substance (1, 3 or 5 mg/kg, i.v. tail) and imatinib (100 mg/kg, p.o.) (Table 8). As an ADC vehicle, the buffer for PoC-DM1 was a mixture of 10 mM sodium succinate, 6% sucrose, and 0.05% Tween20 (pH 5.0), and the buffer for the others was a mixture of 20 mM histidine (Merck, 104352), 50 mMNaCl (Sigma, S3014), 5% sucrose (Sigma, S0389) (pH 6.5), and 0.22 μM filter (Merck, S2GPU11RE). Individual doses were calculated based on animal body weight recorded immediately prior to administration with a dose volume of 5 mL/kg body weight.

[Table 8]

| No. | Subject | Interval, Route |
|---|---|---|
| 1 | Control (vehicle, ADC buffer) | D0, D7, D14 (Three times), i.v. |
| 2 | Imatinib 100 mg/kg | D0 - D29 (Daily for 30 days), p.o. |
| 3 | ADC 1 mg/kg | D0, D7, D14 (Three times), i.v. |
| 4 | ADC 3 mg/kg | D0, D7, D14 (Three times), i.v. |
| 5 | ADC 5 mg/kg | D0, D7, D14 (Three times), i.v. |

The ADC substances are shown in Table 9 below:

[Table 9]

| Study # | ADC |
|---|---|
| #PoC-DM1 | 2G4-SMCC-DM1 |
| #003-1 | 2G4-ThioBridge®-Glu-(Val-Cit-PAB-MMAE)-PEG(24u) DAR 2 |
| #004-2 | 2G4-ThioBridge®-Glu-(Val-Cit-PAB-MMAE)-PEG(24u) DAR 4 |
| #003-2 | 2G4-MC-Val-Cit-PAB-MMAE |
| #007-1 | 2G4-ThioBridge®-Glu-$[(\text{Val-Cit-PAB-SN-38})]_2$-Glu-$[\text{PEG(24u)}]_2$ |

The sacrifice dates for each group were as follows:

- Vehicle and imatinib 100 mg/kg: sacrificed on day 49.
- All ADC groups (except 007-1): sacrificed on day 63.

- #007-1 group: sacrificed on day 42 (#007-1 group was terminated before the control (vehicle), so no comparison was made).

All animals were observed once a day for mortality, general condition and clinical signs (time, onset, severity and recovery). Tumor volume and body weight were measured twice a week. In order to measure tumor volume, length, width, and depth were measured using calipers.
The results are shown in Figures 4a to 4e.
During the test period before sacrifice, no adverse drug reaction were observed in any group.
No body weight decrease or body weight increase of 10% or more was observed for 49 days in the imatinib 100 mg/kg group and the test ADC study group (1 mg/kg, 3 mg/kg and 5 mg/kg) compared to the control (vehicle). Thereafter, no rapid body weight decrease or rapid body weight increase was observed in any group. This suggests that all of the ADC substances of the present invention are very safe.
On day 49, the imatinib 100 mg/kg group showed little inhibition of tumor growth compared to the control (vehicle), while the ADC study groups all showed more potent tumor growth inhibition effect (except #007-1 1 mg/kg). In particular, it was confirmed that the ADC study group of 2G4 ThioBridge®-Glu-(Val-Cit-PAB-MMAE)-PEG(24u) (#003-1 and #004-2) has a significant tumor growth inhibition effect in the 3 or 5 mg/kg group, and the tumor remained at almost the same level even after treatment was discontinued.
As a result of measuring tumor weight after sacrifice, all study groups (except #007-1; terminated before the control) showed a higher reduction compared to the control (vehicle) (see Table 10 below). In particular, the ADC study group of 2G4 ThioBridge®-Glu-(Val-Cit-PAB-MMAE)-PEG(24u) (#003-1 and #004-2) was found to have a significant tumor growth inhibition effect.

[Table 10]

| (reduction %) | #PoC-DM1 | #003-1 | #004-2 | #003-2 |
|---|---|---|---|---|
| 3 mg/kg | 23.9 | 49.1 | 78.0 | 34.2 |
| 5 mg/kg | 17.6 | 67.3 | 87.4 | 70.2 |

The above results support that the ADC group showed statistically significant efficacy compared to the control.

**Example 4 : *In vivo* efficacy against SCLC in mice (SCLC-H526 cells)**

In order to evaluate the efficacy of ADC substances against SCLC, an experiment was performed. The SCLC-H526 (NCI-H526, stage E, carcinoma; variant small cell lung cancer, c-Kit overexpression) xenograft model was used.
The analysis was performed in the same manner as in Example 2, except that SCLC-H526 cells ($2 \times 10^6$/ 100 μL/ Head/serum free, RPMI-1640 medium) in 50% Matrigel (Corning, 354248, NY, USA) were used. The mice were enrolled in this study on day 13 after transplantation, and the average tumor volume was about 165 $mm^3$ (day 0); and imatinib was administered orally for 22 days from D0 to D21. The study groups were #PoC-DM1, #003-1, #004-2 and #003-2.
The results are shown in Figures 5a to 5d.
During the test period before sacrifice, no adverse drug reaction were observed in any group.
No body weight decrease or body weight increase of 10% or more was observed for 21 days in the imatinib 100 mg/kg group and the test ADC study group (1 mg/kg, 3 mg/kg and 5 mg/kg) compared to the control (vehicle). Thereafter, no rapid body weight decrease or rapid body weight increase was observed in any group. This suggests that all of the ADC substances of the present invention are very safe.
On day 21, the imatinib 100 mg/kg group showed little inhibition of tumor growth compared to the control (vehicle), while the ADC study group at 3 or 5 mg/kg showed more potent tumor growth inhibition effect (except #007-1 1 mg/kg). In particular, surprisingly, treatment of the #004-2 3 mg/kg group and #004-2 5 mg/kg group induced complete remission of the tumor without regrowth even after discontinuation of administration for up to 105 days.
As a result of measuring tumor weight after sacrifice, all study groups (except #007-1; terminated before the control) showed a higher reduction compared to the control (vehicle) compared to the control (vehicle) (see Table 11 below). In particular, the ADC study group of 2G4 ThioBridge®-Glu-(Val-Cit-PAB-MMAE)-PEG(24u) (#004-2) was found to have a significant tumor growth inhibition effect.

[Table 11]

| (reduction %) | #PoC-DM1 | #003-1 | #004-2 | #003-2 |
|---|---|---|---|---|
| 5 mg/kg | 73.8 | 60.0 | 99.5 | 53.7 |

The above results support that the ADC group showed statistically significant efficacy compared to the control.

**Example 5: *In vivo* efficacy against mast cell tumor in mice (HMC 1.2 cells)**

In order to evaluate the efficacy of NN3201 (2G4-ThioBridge®-Glu(Val-Cit-PAB-MMAE)-PEG(24u) DAR 4; #004-2) against mast cell tumor, an experiment was performed. A HMC 1.2(c-Kit mutation and imatinib resistant; Exon11 (V560G) + Exon17 (D816V)) xenograft model was used.

4-week-old female CB-17 SCID immunodeficient mice (Janvier France, Inc.) were acclimatized under SPF (Specific Pathogen Free) conditions for 18 days before being used in the experiment. HMC-1.2 cells in 50% Matrigel (Corning, 354248, NY, USA) were injected subcutaneously into 6-week-old mice ($1 \times 10^6$/100 μL/head/serum free, IMDM medium). The total injection volume containing the suspended cells was 200 μL.

The mice were enrolled in this study on day 11 after transplantation, and the average tumor volume was about 175.4 $mm^3$ (day 0). The mice were randomly assigned to each group (n = 6/group), and then the mice were administered with ADC vehicle (5 ml/kg, i.v. tail), 2G4 (5 mg/kg, i.v. tail), #PoC-DM1 (5 mg/kg, i.v. tail), NN3201 (1, 3 or 5 mg/kg, i.v. tail), and imatinib (100 mg/kg, p.o.). As an ADC vehicle, a mixture of 20 mM histidine (Merck, 104352), 50 mM NaCl (Sigma, S3014), 5% sucrose (Sigma, S0389) (pH 6.5), and 0.22 μM filter (Merck, S2GPU11RE) was used. Individual doses were calculated based on animal body weight recorded immediately prior to administration with a dose volume of 5 mL/kg body weight. i.v. injections were administered three times on D0, D7, and D14, and imatinib was administered orally for 18 days from D0 to D17.

The results are shown in Figure 6.

During the test period, no adverse drug reaction were observed in any group.

No body weight decrease or body weight increase of 10% or more was observed for 17 days in the imatinib 100 mg/kg group and the test group compared to the control (vehicle). Thereafter, no rapid body weight decrease or rapid body weight increase was observed in any group. This suggests that all of the ADC substances of the present invention are very safe.

On day 17, the imatinib 100 mg/kg group showed little inhibition of tumor growth, while the ADC study group showed a more potent tumor growth inhibition effect compared to the control (vehicle).

On day 77, tumors were re-inoculated. On day 95, TGI (tumor growth inhibition) was 71.0% in the #NN3201 1 mg/kg (re-) inoculated group, 79.0% in the #NN3201 3 mg/kg (re-) inoculated group, and 74.8% in the #NN3201 5 mg/kg (re-) inoculated group compared to the control (first-) inoculated group. In addition, the #PoC-DM1 5 mg/kg (re-) inoculated group showed tumor inhibition of 46.2% (Figure 7).

The above results support that the ADC group showed statistically significant efficacy compared to the control.

**Example 6: *In vivo* efficacy against AML in mice (Kasumi-1 cells)**

In order to evaluate the efficacy of NN3201 (2G4 ThioBridge® -Glu(Val-Cit-PAB-MMAE)-PEG(24u) DAR 4) against AML, this experiment was performed. The Kasumi-1 (c-Kit mutant and partially sensitive to imatinib; Exon17 (N822K)) xenograft model was used.

The experiment was performed in the same manner as in Example 5, except that Kasumi-1 cells ($3 \times 10^6$/100 μL/head/serum free, RPMI1640 medium) in 50 % Matrigel (Corning, 354248, NY, USA) were used. The mice were enrolled in this study on day 29 after transplantation, and the average tumor volume was about 170 $mm^3$ (day 0).

The results are shown in Figure 8.

During the test period, no adverse drug reaction were observed in any group.

No body weight decrease or body weight increase of 10% or more was observed for 17 days in the imatinib 100 mg/kg group and the test group compared to the control (vehicle). Thereafter, no rapid body weight decrease or rapid body weight increase was observed in any group. This suggests that all of the ADC substances of the present invention are very safe.

On day 17, the imatinib 100 mg/kg group showed little inhibition of tumor growth compared to the control (vehicle), while the PoC-DM1 group showed a weak inhibition effect. The NN3201 group was found to have a more potent tumor growth inhibition effect.

The above results support that the ADC group showed statistically significant efficacy compared to the control.

**Example 7: *In vivo* efficacy against breast cancer in mice (MDA-MB-468 cells)**

In order to evaluate the efficacy of ADC substances against breast cancer, an experiment was performed. The MDA-MB-468 (c-Kit, Negative) xenograft model was used in this experiment.

The experiment was performed in the same manner as in Example 5, except that MDA-MB-468 cells ($5 \times 10^6$/100 μL/head/serum free, DMEM medium) in 50 % Matrigel (Corning, 354248, NY, USA) were used. The mice were

enrolled in this study on day 21 after transplantation, and the average tumor volume was about 167 $mm^3$ (day 0).

The results are shown in Figures 9a to 9d.

During the test period before sacrifice, no adverse drug reaction were observed in any group.

No body weight decrease or body weight increase of 10% or more was observed for 49 days in the imatinib 100 mg/kg group and the test ADC study group (1 mg/kg, 3 mg/kg and 5 mg/kg) compared to the control (vehicle). Thereafter, no rapid body weight decrease or rapid body weight increase was observed in any group. This suggests that all of the ADC substances of the present invention are very safe.

On day 49, #PoC-DM1 did not show any antitumor activity in xenografts transplanted with MDA-MB-468 cells lacking c-Kit expression compared to the control (vehicle); while #003-1, #004-2 and #003-2 were found to have a more potent tumor growth inhibition effect at all doses. In particular, it was found that the group treated with #004-2 at 5 mg/kg significantly inhibited tumor growth.

As a result of measuring tumor weight after sacrifice, tumors were reduced in #003-1, #004-2, #003-2 compared to the control (vehicle) (see Table 12 below). In particular, the group #004-2 was found to have a significant tumor growth inhibition effect.

[Table 12]

| (reduction %) | #003-1 | #004-2 | #003-2 |
|---|---|---|---|
| 5 mg/kg | 41.2 | 84.6 | 62.0 |

From the MDA-MB-468 results, it is interesting to note that an ADC using 2G4, a cleavable linker, and MMAE exhibited dose-dependent efficacy in a c-Kit negative cell-based xenograft model, whereas an ADC using a non-cleavable linker did not. The above results suggest that an ADC comprising 2G4, a cleavable linker and MMAE may be an ideal partner even when targeting non-c-Kit.

## Example 8: *In vivo* efficacy against SCLC in mice (SCLC-H526 cells) - re-challenge analysis

In order to determine whether NN3201 treatment was effective even after tumor re-inoculation, tumor re-challenge analysis was performed.

The analysis was performed in the same manner as in Example 3, except that the experimental group was the #004-2 group alone at 3 mg/kg and 5 mg/kg. Tumor re-challenge was performed at the time of complete tumor remission (day 63). The control cohort (squares) used animals of the same age as the control that received the first tumor inoculation on day 63 without initial tumor transplantation on the right flank. For xenograft analysis, SCLC-H526 ($2 \times 10^6$/100 μL/head/serum free, RPMI-1640 medium) cells in 50% Matrigel (Corning, 354248, NY, USA) were injected into C.B-17 SCID mice s.c. (subcutaneously, left flank).

The results are shown in Figure 10.

In tumor re-challenge, the #004-2 groups at 3 mg/kg and 5 mg/kg showed dose-dependent efficacy without additional ADC injections, whereas the control resulted in tumor growth.

## Example 9: *In vivo* efficacy against mast cell tumor in mice (HMC 1.2 cells) - re-challenge analysis

In order to determine whether NN3201 treatment was effective even after tumor re-inoculation, tumor re-challenge analysis was performed.

At the time when complete tumor remission was confirmed in Example 4 (day 77), tumor re-challenge analysis was performed in the NN3201 (1, 3 or 5 mg/kg, i.v. tail) group. The present inventors included PoC DM1 ADC to understand the differences between DM1 and MMAE as payload in terms of immunogenic cell death (ICD). animals of the same age were used as the control. For xenograft analysis, HMC-1.2 cells ($1 \times 10^6$/100 μL/head/serum free, IMDM medium) cells in 50% Matrigel (Corning, 354248, NY, USA) were injected into C.B-17 SCID mice s.c. (subcutaneously, left flank).

The results are shown in Figure 11.

In tumor re-challenge analysis, the NN3201 group showed dose-dependent efficacy without additional ADC injections, whereas the control resulted in tumor growth. The PoC-DM1 group showed lower tumor growth inhibition than NN3201, indicating that NN3201 had a more potent effect than PoC-DM1.

## Example 10: *In vivo* pre-toxicity analysis (10 mg/kg)

An experiment was performed to evaluate the toxicity of ADC substances by examining body weight changes for two weeks when the Balb/c mice were administered once i.v. at a high concentration.

7-week-old female BALB/c mice (Dae Han Bio Link) were acclimatized under SPF (Specific Pathogen Free) conditions before being used in the experiment. The mice were randomly assigned to one of six groups (n = 3/group), and then the mice were administered with ADC vehicle (5 ml/kg, i.v. tail), or each ADC substance (10 mg/kg, i.v. tail). As an ADC vehicle, the buffer was a mixture of 20 mM histidine (Merck, 104352), 50 mM NaCl (Sigma, S3014), 5% sucrose (Sigma, S0389) (pH 6.5), and 0.22 μM filter (Merck, S2GPU11RE). Individual doses were calculated based on animal body weight recorded immediately prior to administration with a dose volume of 5 mL/kg body weight (Table 13).

[Table 13]

| Group. (NN3201-Abzena ADC) #003-1, #004-2, #003-2 | | No. of Animals. | Volume. |
|---|---|---|---|
| 1 | Control (vehicle, Abzena-ADC buffer) | 3 | 5 mL/kg |
| 2 | #003-1 10 mg/kg | 3 | |
| 3 | #004-2 10 mg/kg | 3 | |
| 4 | #003-2 10 mg/kg | 3 | |

All animals were observed once a day for mortality, general condition and clinical signs (time, onset, severity and recovery). All animals were weighed daily from the date of grouping. The animals were sacrificed on day 14.
After completion of the experiment, the animals were euthanized (since pulmonary congestion may be affected during euthanasia using respiratory anesthetics or a $CO_2$ chamber, cervical dislocation was performed as quickly as possible to minimize pain), and an autopsy was performed to confirm the presence of symptoms of pulmonary congestion. Tissue (lung) was stored in 10% neutral buffered formalin (NBF).
The results are shown in Figure 12.
During the test period, no adverse drug reaction were observed in any group.
During the test period, no symptoms of body weight abnormalities (5% or more), such as rapid body weight decrease or rapid body weight increase due to administration, were observed in any group.
At the end of the experiment (day 14), the body weight was increased by 0.43 g (2.10%) in the #003-1 10 mg/kg group and by 0.63 g (3.00%) in the #004-2 10 mg/kg group compared to the day of administration (day 0). The body weight in the #003-2 10 mg/kg administration group was reduced by 0.27 g (1.29%) at the end of the experiment (day 14) compared to the day of administration (day 0).
There were no symptoms of pulmonary congestion in lung tissue in any group.

**Example 11: *In vivo* pre-toxicitv analysis (20 / 40 / 60 mg/kg)**

An experiment was performed to evaluate the toxicity of ADC substances by examining body weight changes for two weeks when the Balb/c mice were administered once i.v. at a high concentration.
Analysis was performed in the same manner as in Example 10, except that the groups were assigned as follows (Table 14).

[Table 14]

| Group | No. of Animals. | Volume. |
|---|---|---|
| Control (Non-treat) | 3 | - |
| Control (vehicle, ADC buffer) | 3 | 5 mL/kg |
| #003-1 20, 40, or 60 mg/kg | 3 | |
| #004-2 20, 40, or 60 mg/kg | 3 | |

The results are shown in Figures 13a to 13d.
During the test period, no adverse drug reaction were observed in any group.
During the test period, no symptoms of body weight abnormalities (5% or more), such as rapid body weight decrease or rapid body weight increase due to administration, were observed in any group. On the first day after administration (day 0), the body weight decrease was observed by 0.63 g (3.23%) in the #PoC-DM1 40 mg/kg group and by 0.70 g (3.57%) in the #PoC-DM1 60 mg/kg group. However, the decreased body weight was recovered.
Although the embodiments of the present invention have been described above, those of ordinary skill in the art will be able to modify and change the present invention in various ways by adding, changing, deleting or adding components, etc., without departing from the spirit of the present invention as set forth in the patent claims, and this

will also be included within the scope of the patent protection of the present invention.

**Claims**

1. An antibody-drug conjugate of the following formula 1:

<Formula 1> $Ab\text{-}(L)_x\text{-}(D)_y$

in the formula,

Ab is an anti-c-Kit antibody or an antigen-binding fragment thereof that specifically binds to the epitope of human c-Kit in SEQ ID NO: 9 and SEQ ID NO: 10;
L is a linker comprising a cleavable linker;
D is a drug moiety;
x is an integer from 1 to 8; and
y is an integer from 1 to 8.

2. The antibody-drug conjugate according to claim 1, wherein the Ab is an antibody or an antigen-binding fragment thereof that specifically binds to one or more of R122, Y125, R181, K203, R205, S261, and H263 in SEQ ID NO: 12.

3. The antibody-drug conjugate according to claim 1, wherein the antibody or antigen-binding fragment thereof cross-competes for binding to the epitope of human c-Kit in SEQ ID NO: 9 and SEQ ID NO: 10 with a reference antibody comprising the heavy chain CDR1 of SEQ ID NO: 1, the heavy chain CDR2 of SEQ ID NO: 2, the heavy chain CDR3 of SEQ ID NO: 3, the light chain CDR1 of SEQ ID NO: 4, the light chain CDR2 of SEQ ID NO: 5, and the light chain CDR3 of SEQ ID NO: 6.

4. The antibody-drug conjugate according to claim 1, wherein the antibody or antigen-binding fragment thereof comprises the heavy chain CDR1 of SEQ ID NO: 1, the heavy chain CDR2 of SEQ ID NO: 2, the heavy chain CDR3 of SEQ ID NO: 3, the light chain CDR1 of SEQ ID NO: 4, the light chain CDR2 of SEQ ID NO: 5, and the light chain CDR3 of SEQ ID NO: 6.

5. The antibody-drug conjugate according to claim 1, wherein the antibody or antigen-binding fragment thereof comprises the heavy chain variable domain represented by SEQ ID NO: 7 and the light chain variable domain represented by SEQ ID NO: 8.

6. The antibody-drug conjugate according to claim 3 or 5, wherein the antibody or antigen-binding fragment thereof has one or two amino acids in the CDR modified, deleted, or substituted.

7. The antibody-drug conjugate according to any one of claims 1 to 6, wherein the antibody or antigen-binding fragment thereof maintains at least 90, 91, 92, 93, 94, 95, 96, 97, 98, or 99% identity through the heavy chain variable domain or the light chain variable domain.

8. The antibody-drug conjugate according to claim 1, wherein L is a linker comprising a spacer in a form in which a polymer is bound or not bound.

9. The antibody-drug conjugate according to claim 1, wherein the linker L has a structure of the following formula 2: <

Formula 2> $(L_1)_m\text{-}(S)_n\text{-}(L_2)_p$

in the formula,

$L_1$ is a linker connecting Ab and S or Ab and $L_2$;
S is a spacer in a form in which a polymer is bound or not bound;
$L_2$ is a cleavable linker;
m is an integer from 0 to 8;
n is an integer from 0 to 8; and
p is an integer from 1 to 8.

**10.** The antibody-drug conjugate according to claim 9, wherein m is 1 to 4, n is 1 to 4, and p is 1 to 4.

**11.** The antibody-drug conjugate according to claim 9, wherein $L_1$ comprises a linker selected from the group consisting of a cleavable linker, a non-cleavable linker, a hydrophilic linker, a procharged linker, and a dicarboxylic acid-based linker.

**12.** The antibody-drug conjugate according to claim 1 or 9, wherein L or $L_1$ comprises

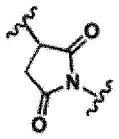, 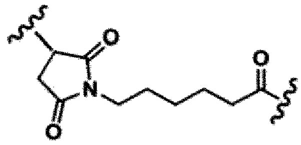, 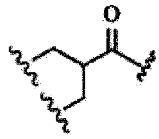, 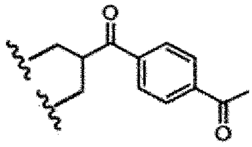, 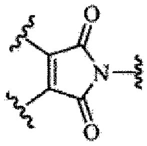,

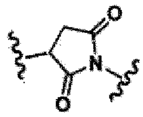, 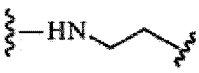, 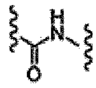, 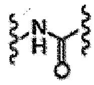, 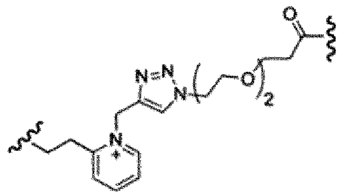, 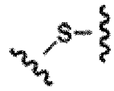,

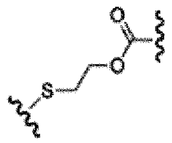, 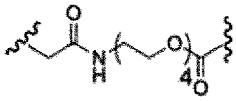, 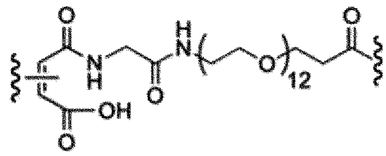,

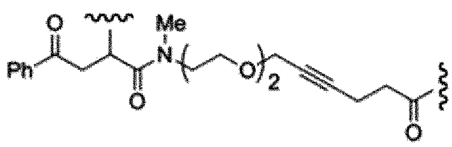, 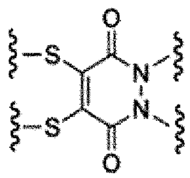, 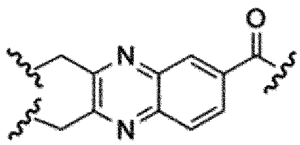,

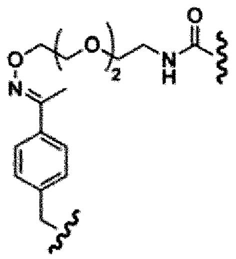, 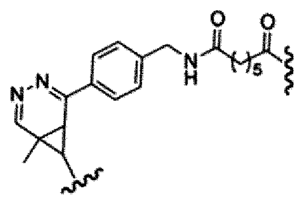, 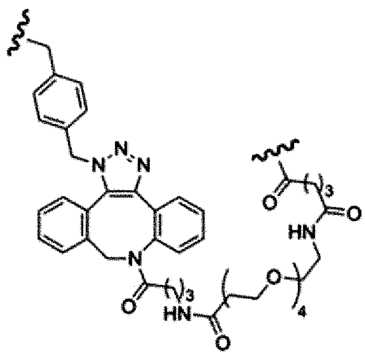,

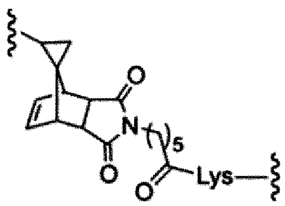, 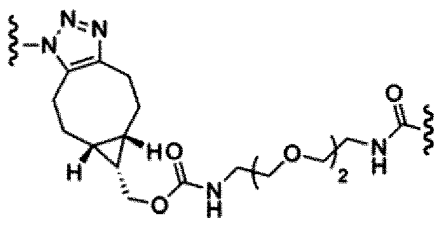, 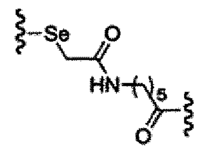,

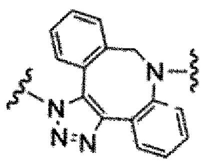
,
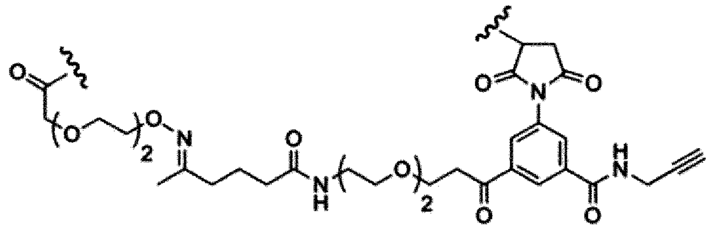
,

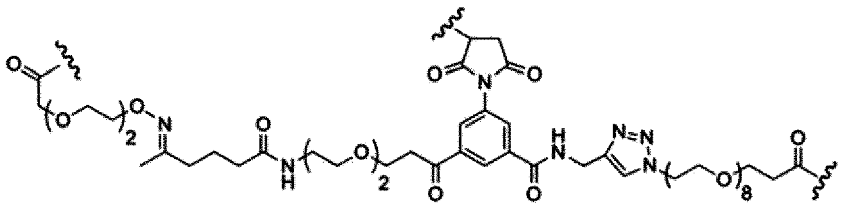
, or
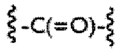

**13.** The antibody-drug conjugate according to claim 9, wherein S comprises a spacer selected from the group consisting of

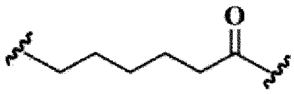
,
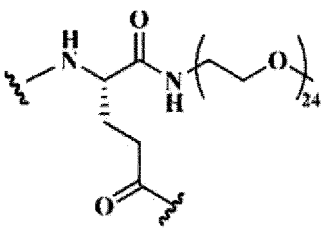
,

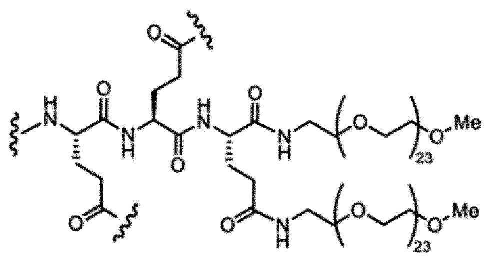
,
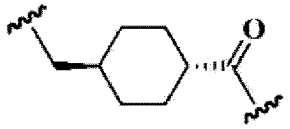
, and
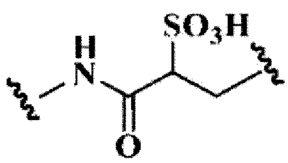

**14.** The antibody-drug conjugate according to claim 9, wherein S is a spacer comprising aspartate, glutamate, or a combination thereof in a form in which a polymer is bound or not bound.

**15.** The antibody-drug conjugate according to claim 9, wherein the polymer is polyalkylene, polyalkylene glycol, polyvinylpyrrolidone, polyacrylate, polyoxazoline, polyvinyl alcohol, polyacrylamide or polymethacrylamide, HPMA copolymer, polyester, polyacetal, poly(orthoester), polycarbonate, poly(imino carbonate), polyamide, a copolymer of divinylether-maleic anhydride or styrene-maleic anhydride, polysaccharide, or polyglutamic acid.

**16.** The antibody-drug conjugate according to claim 9, wherein $L_2$ comprises a linker selected from the group consisting of valine-citrulline-p-aminobenzyl carbamoyl (Val-Cit-PAB), alanine-phenylalanine-p-aminobenzyl carbamoyl (Ala-Phe-PAB), alanine-alanine-p-aminobenzyl carbamoyl (Ala-Ala-PAB), valine-alanine-p-aminobenzyl carbamoyl (Val-Ala-PAB), phenylalanine-lysine-p-aminobenzyl carbamoyl (Phe-Lys-PAB), alanine-alanine-glycine-p-aminobenzyl carbamoyl (Ala-Ala-Gly-PAB), and glycine-glycine-glycine-p-aminobenzyl carbamoyl (Gly-Gly-Gly-PAB) linkers.

**17.** The antibody-drug conjugate according to claim 1, wherein the linker (L) comprises a linker selected from the group consisting of the following formulas 3 to 5.

<Formula 3>

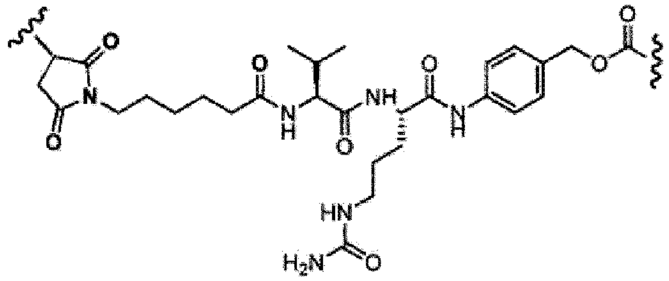

,

<Formula 4>

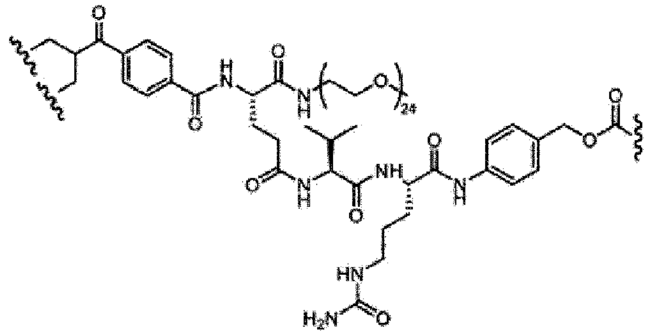

,

or

<Formula 5>

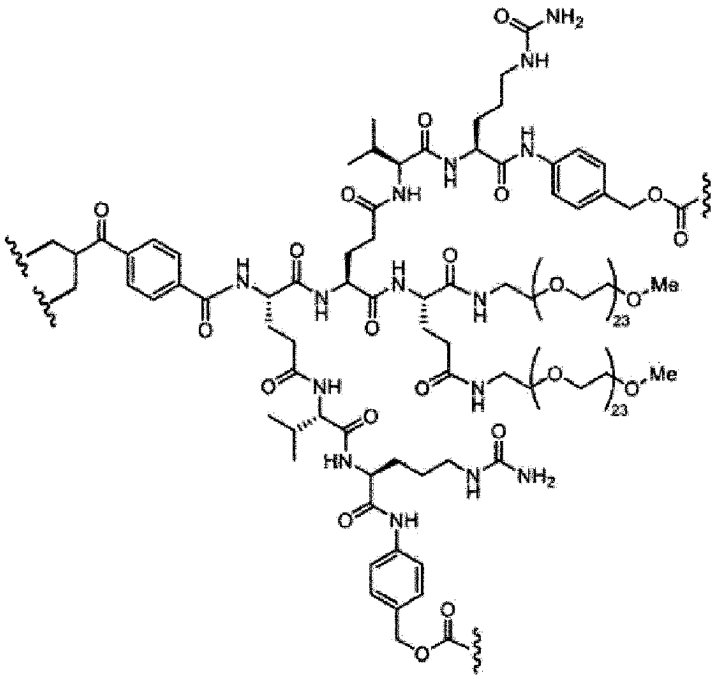

**18.** The antibody-drug conjugate according to claim 1, wherein the drug moiety (D) is selected from the group consisting of a V-ATPase inhibitor, an apoptosis promoting agent, a Bcl2 inhibitor, an MCL1 inhibitor, a HSP90 inhibitor, an IAP inhibitor, an mTor inhibitor, a microtubule inhibitor, an auristatin, a dolastatin, a maytansinoid, a MetAP (methionine aminopeptidase), a inhibitor of nuclear export of protein CRM1, a DPPIV inhibitor, a proteasome inhibitor, a inhibitor of phosphoryl transfer reaction in mitochondria, a protein synthesis inhibitor, a kinase inhibitor, a CDK2 inhibitor, a CDK9

inhibitor, a kinesin inhibitor, a HDAC inhibitor, a DNA damaging agent, a DNA alkylating agent, a DNA intercalating agent, a DNA minor groove binder, and a DHFR inhibitor.

**19.** The antibody-drug conjugate according to claim 18, wherein the drug moiety (D) is a microtubule inhibitor (MTI) moiety.

**20.** The antibody-drug conjugate according to claim 19, wherein the drug moiety (D) comprises a drug selected from the group consisting of SN-38 ((4S)-4,11-diethyl-4,9-dihydroxy-1,4-dihydro-3*H*,14*H*-pyrano[3',4':6,7]indolizino[1,2-*b*]quinoline-3,14-dione), auristatin, dolastatin, monomethyl auristatin E (MMAE), monomethyl auristatin F (MMAF), monomethyl dolastatin 10 (MMAD), or a combination thereof.

**21.** The antibody-drug conjugate according to claim 1, wherein the antibody-drug conjugate is selected from the group consisting of the following formulas 6 to 8:

<Formula 6>

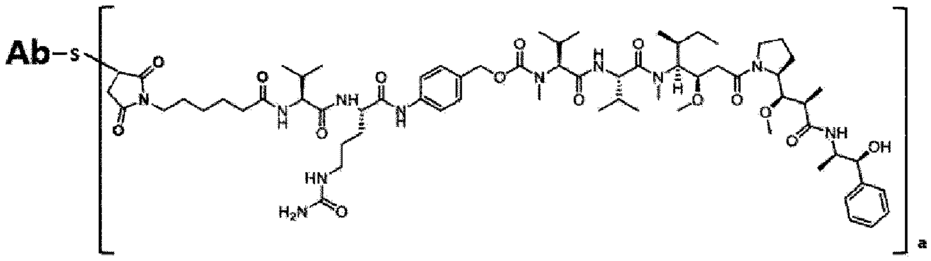

,

<Formula 7>

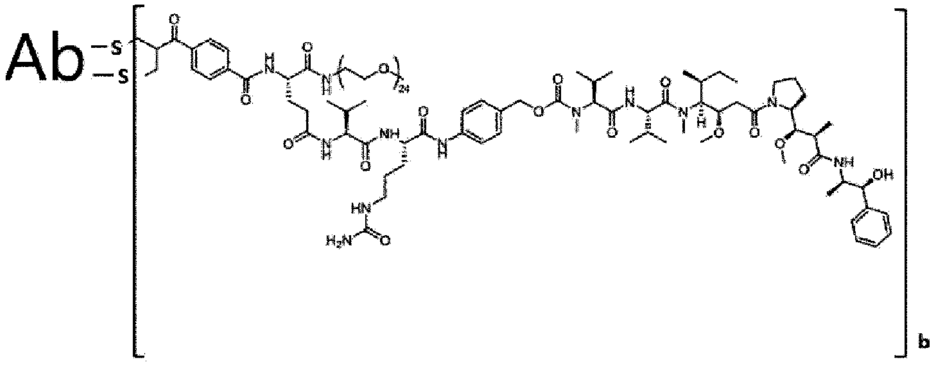

and

<Formula 8>

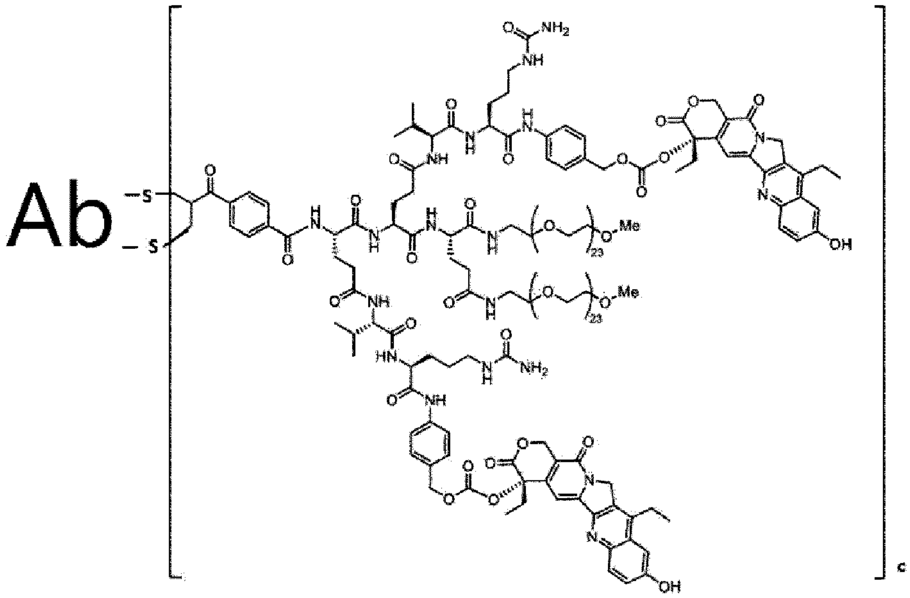

,

in the formulas, a is an integer from 1 to 8, and b and c are each an integer from 1 to 4.

**22.** An antibody-drug conjugate selected from the group consisting of the following formulas 6 to 8:

<Formula 6>

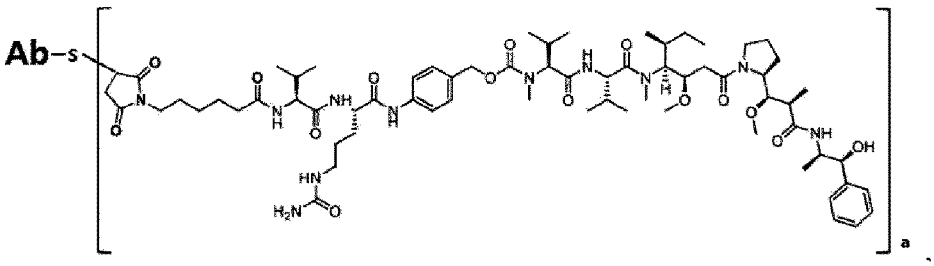

<Formula 7>

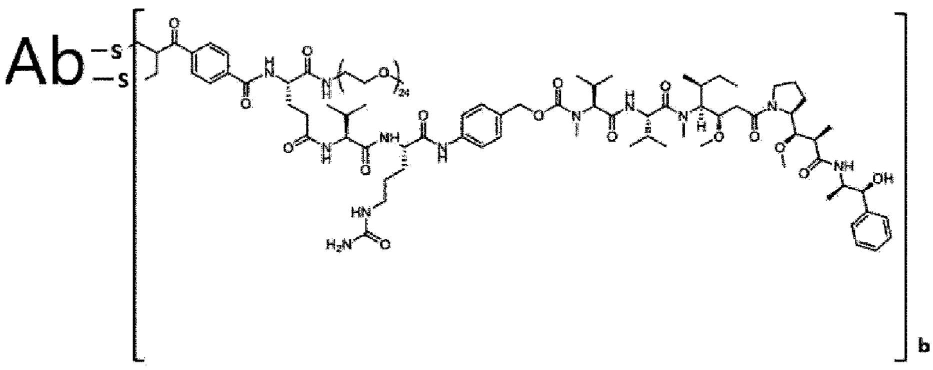

and

<Formula 8>

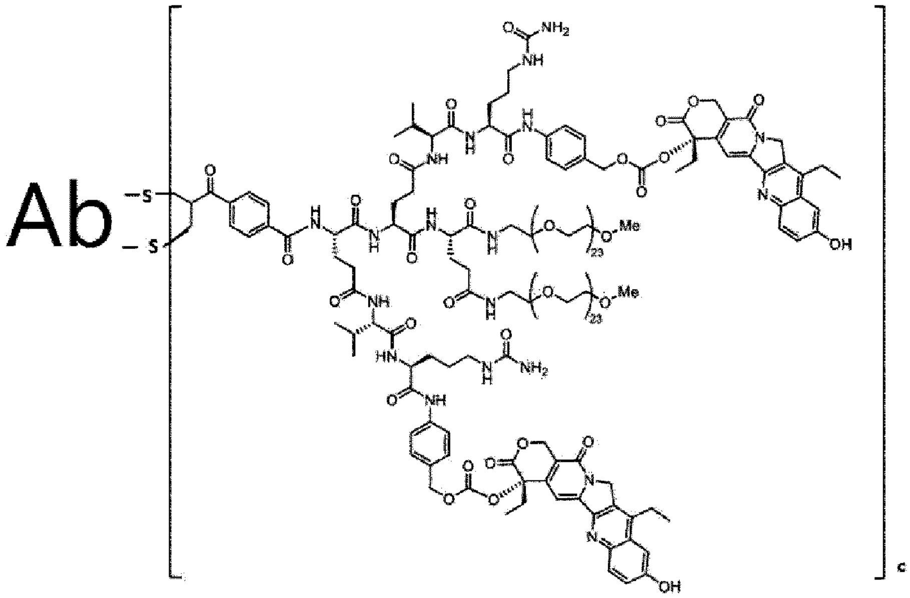

,

in the formulas,

Ab is an antibody or an antigen-binding fragment thereof comprising the heavy chain CDR1 of SEQ ID NO: 1, the heavy chain CDR2 of SEQ ID NO: 2, the heavy chain CDR3 of SEQ ID NO: 3, the light chain CDR1 of SEQ ID NO: 4, the light chain CDR2 of SEQ ID NO: 5, and the light chain CDR3 of SEQ ID NO: 6; and
a is an integer from 1 to 8, and b and c are each an integer from 1 to 4.

**23.** A pharmaceutical composition for preventing or treating cancer, comprising the antibody-drug conjugate according to claim 1 or 22.

**24.** A method for preventing or treating cancer, comprising administering a composition comprising a pharmaceutically effective amount of the antibody-drug conjugate according to claim 1 or 22 to a subject in need thereof.

**25.** Use of the antibody-drug conjugate according to claim 1 for use in therapy.

Figure 1a

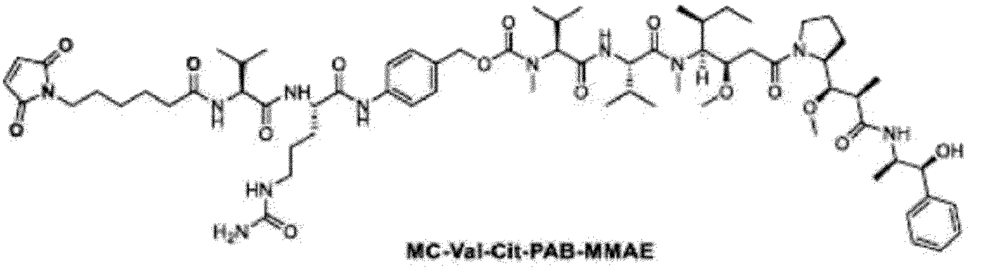

MC-Val-Cit-PAB-MMAE

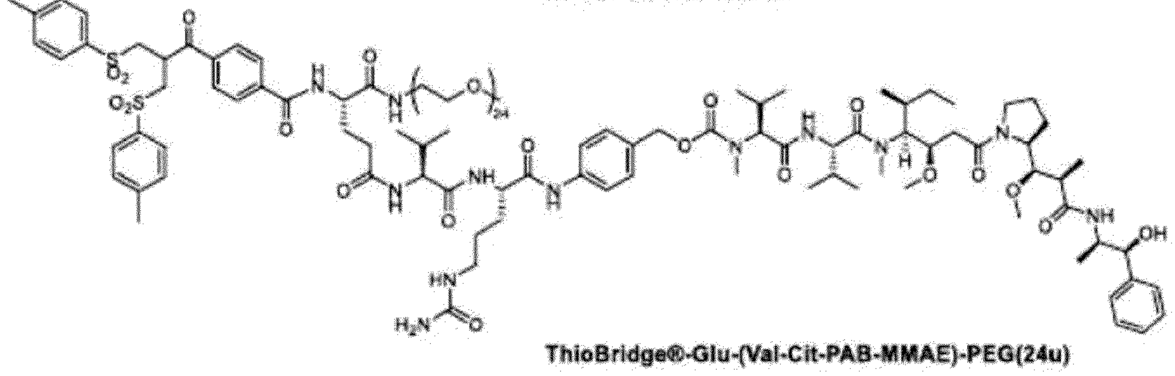

ThioBridge®-Glu-(Val-Cit-PAB-MMAE)-PEG(24u)

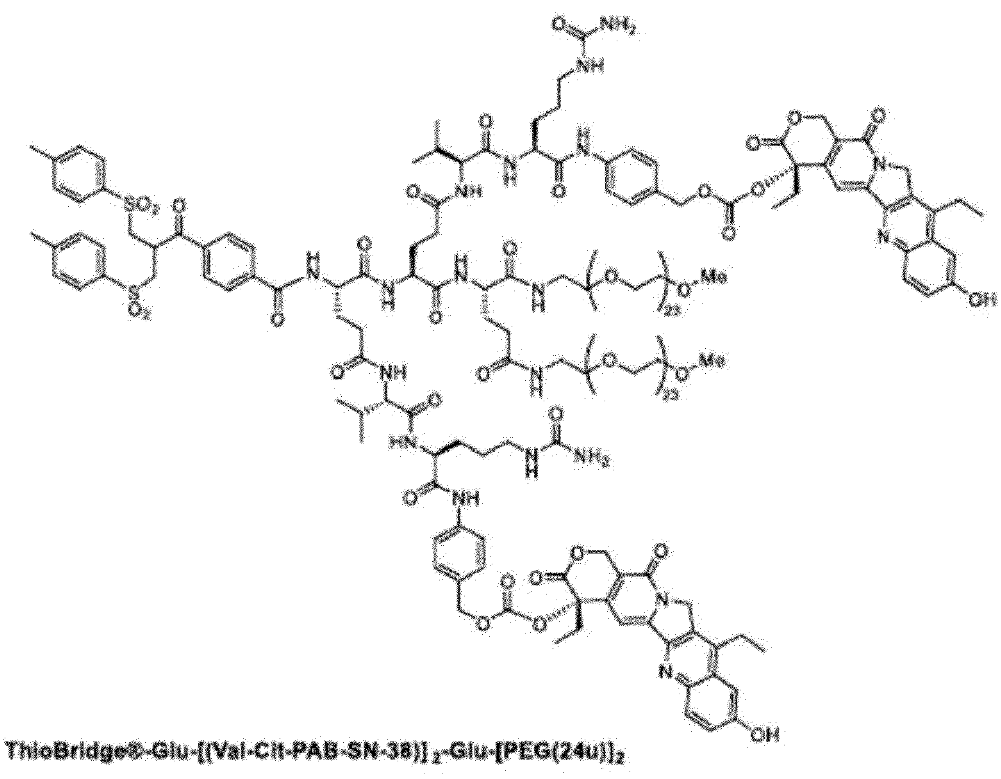

ThioBridge®-Glu-[(Val-Cit-PAB-SN-38)] $_2$-Glu-[PEG(24u)]$_2$

Figure1b

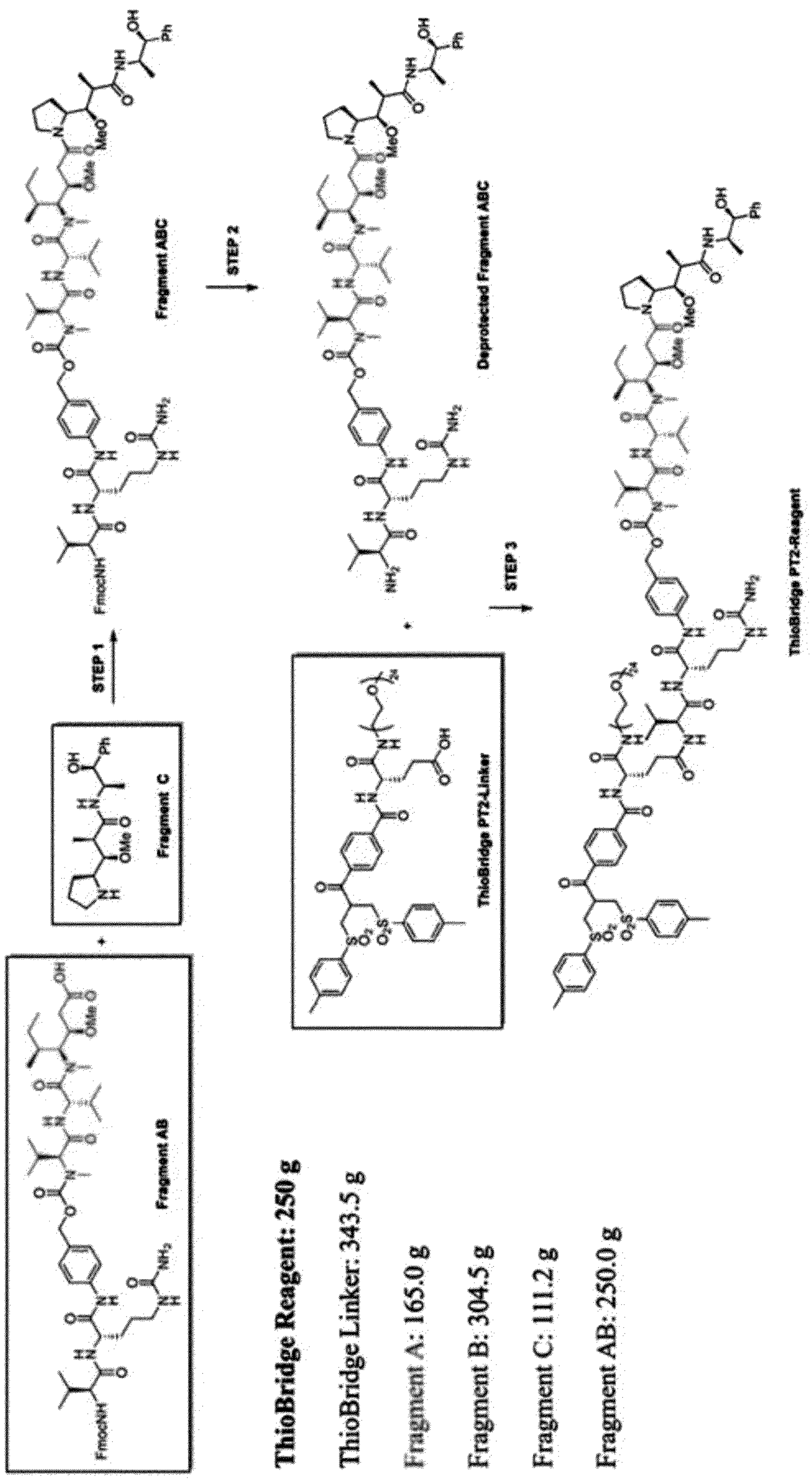
Fragment AB
Fragment C
STEP 1
Fragment ABC
STEP 2
Deprotected Fragment ABC
ThioBridge PT2-Linker
STEP 3
ThioBridge PT2-Reagent
ThioBridge Reagent: 250 g
ThioBridge Linker: 343.5 g
Fragment A: 165.0 g
Fragment B: 304.5 g
Fragment C: 111.2 g
Fragment AB: 250.0 g

Figure 2

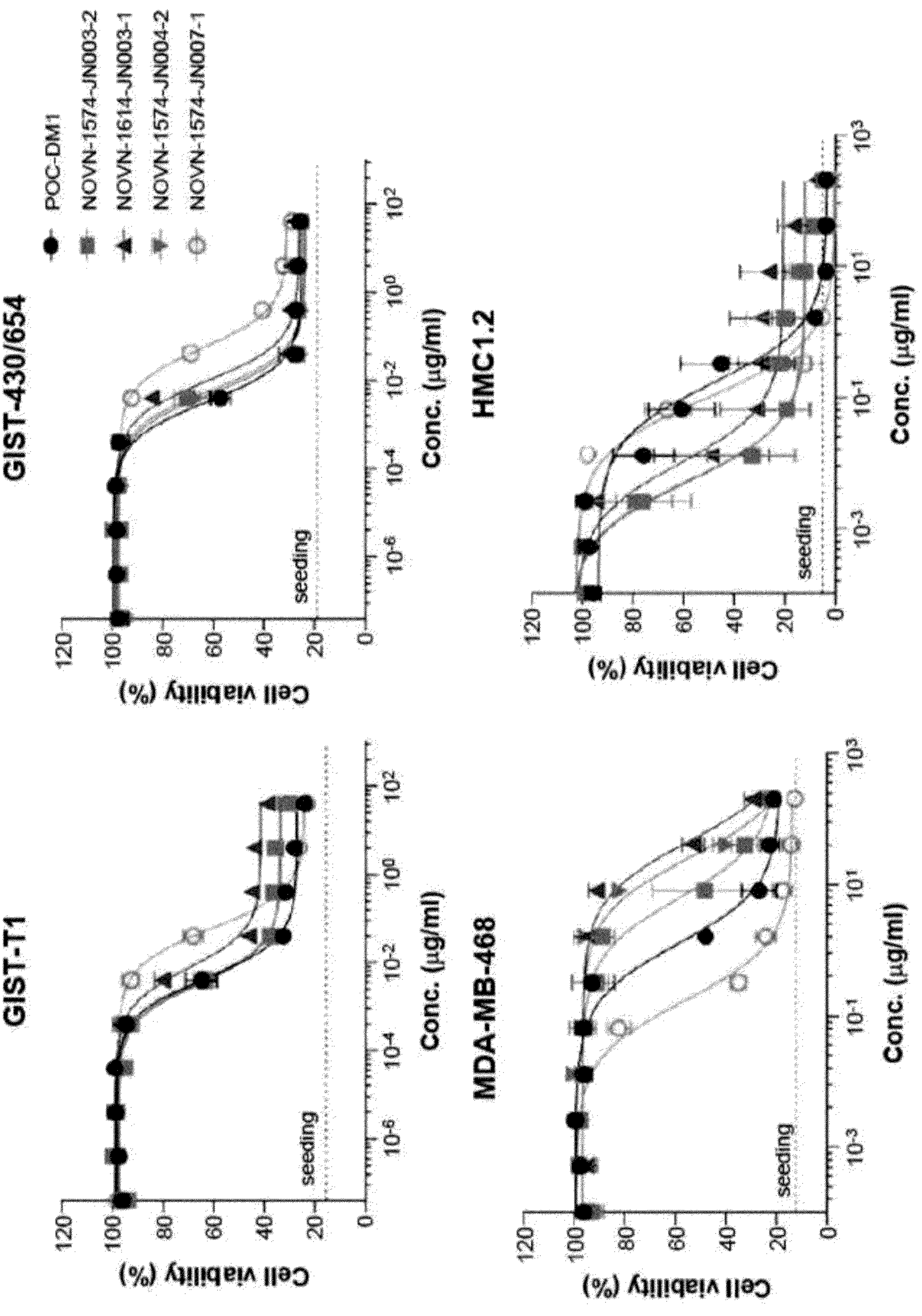
GIST-T1
GIST-430/654
MDA-MB-468
HMC1.2
POC-DM1
NOVN-1574-JN003-2
NOVN-1614-JN003-1
NOVN-1574-JN004-2
NOVN-1574-JN007-1
Cell viability (%)
Conc. (µg/ml)
seeding

Figure 3a

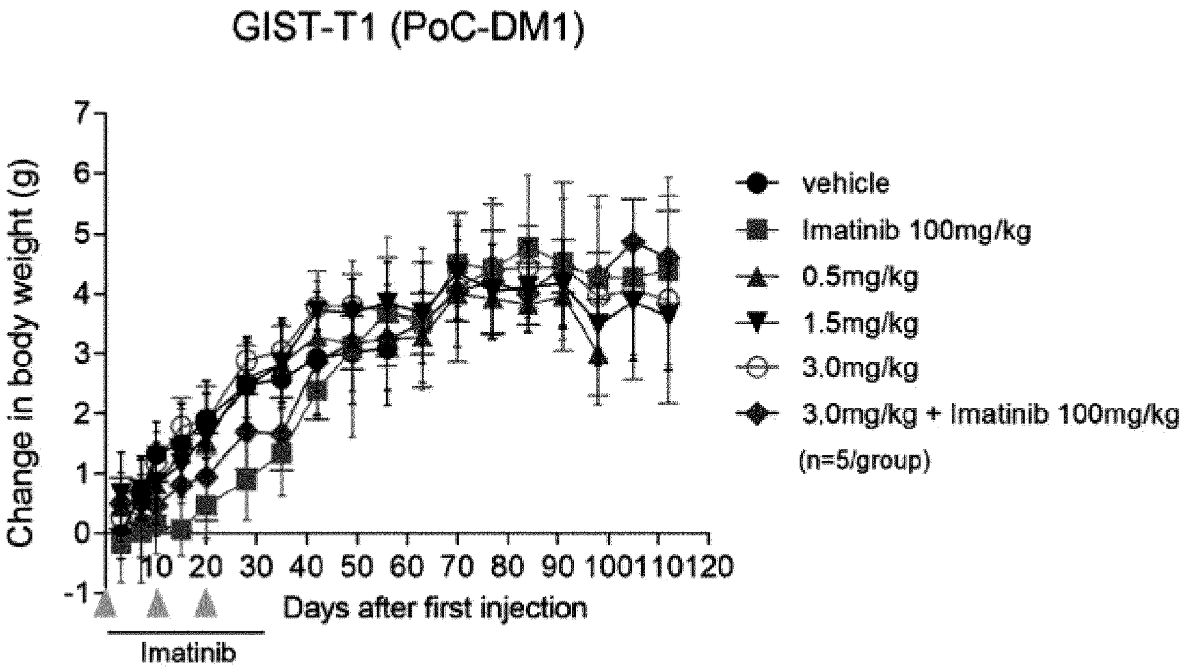
GIST-T1 (PoC-DM1)
Change in body weight (g)
Days after first injection
Imatinib
vehicle
Imatinib 100mg/kg
0.5mg/kg
1.5mg/kg
3.0mg/kg
3.0mg/kg + Imatinib 100mg/kg
(n=5/group)

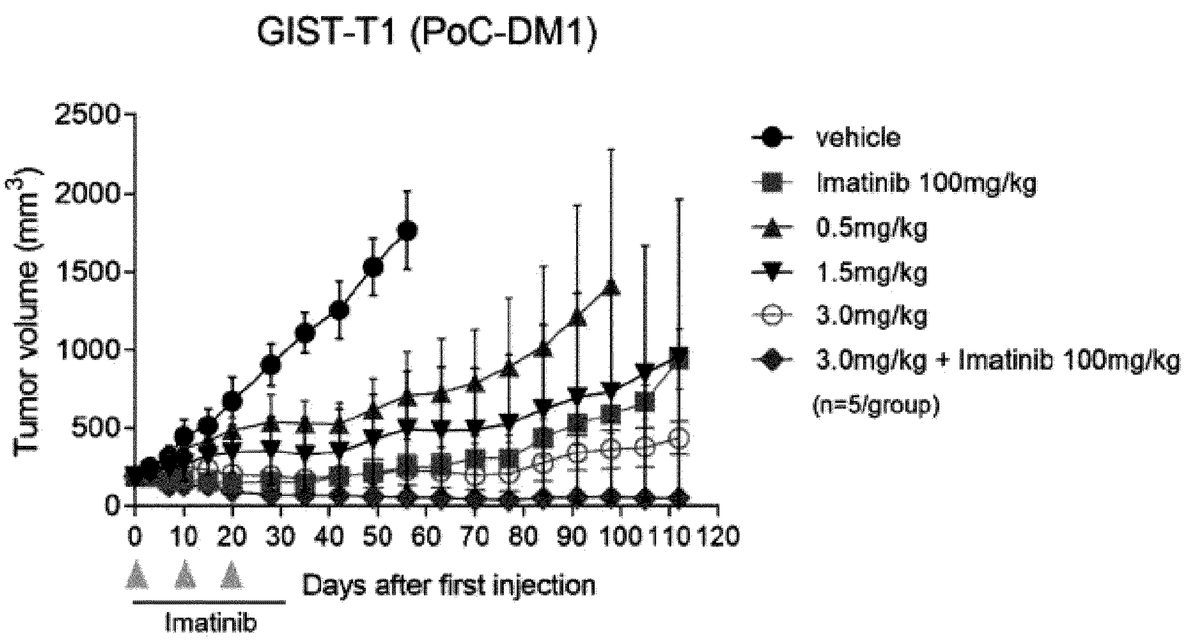
GIST-T1 (PoC-DM1)
Tumor volume (mm3)
Days after first injection
Imatinib
vehicle
Imatinib 100mg/kg
0.5mg/kg
1.5mg/kg
3.0mg/kg
3.0mg/kg + Imatinib 100mg/kg
(n=5/group)

Figure 3b

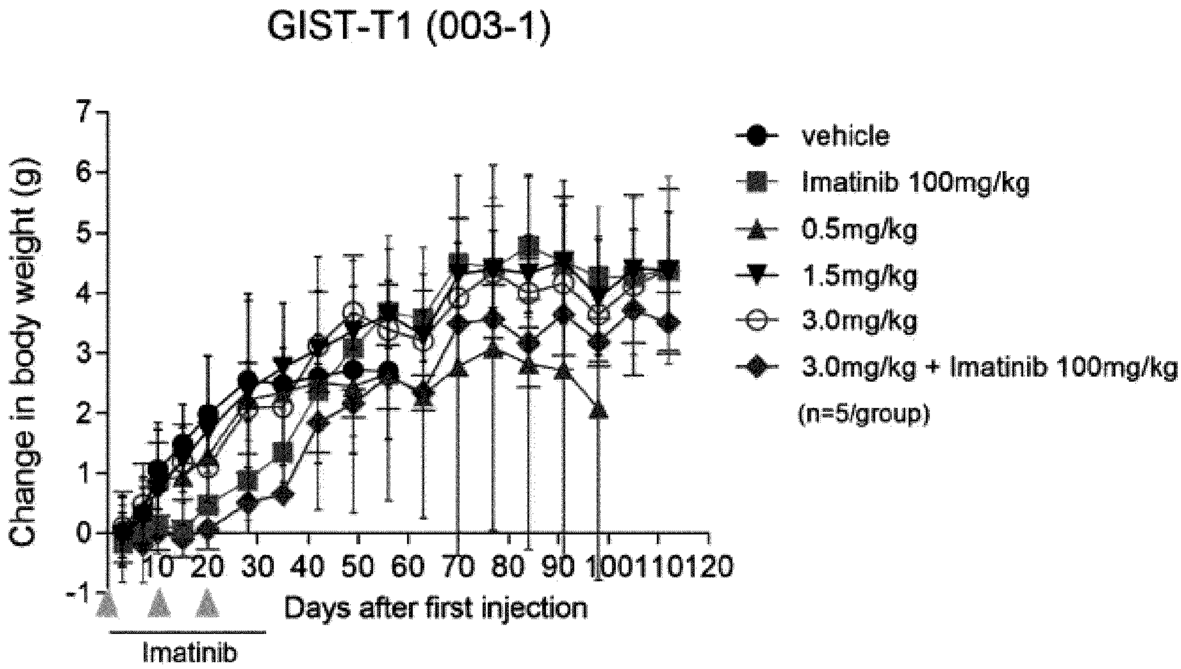
GIST-T1 (003-1)
Change in body weight (g)
Days after first injection
Imatinib
vehicle
Imatinib 100mg/kg
0.5mg/kg
1.5mg/kg
3.0mg/kg
3.0mg/kg + Imatinib 100mg/kg
(n=5/group)

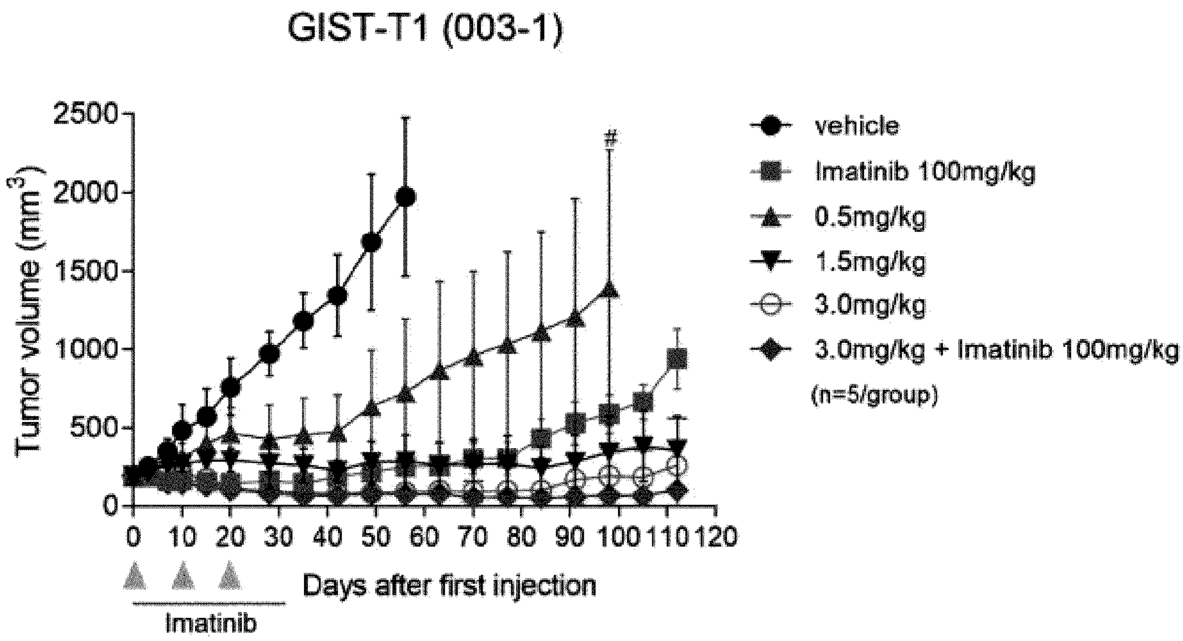
GIST-T1 (003-1)
Tumor volume (mm3)
Days after first injection
Imatinib
#
vehicle
Imatinib 100mg/kg
0.5mg/kg
1.5mg/kg
3.0mg/kg
3.0mg/kg + Imatinib 100mg/kg
(n=5/group)

Figure 3c

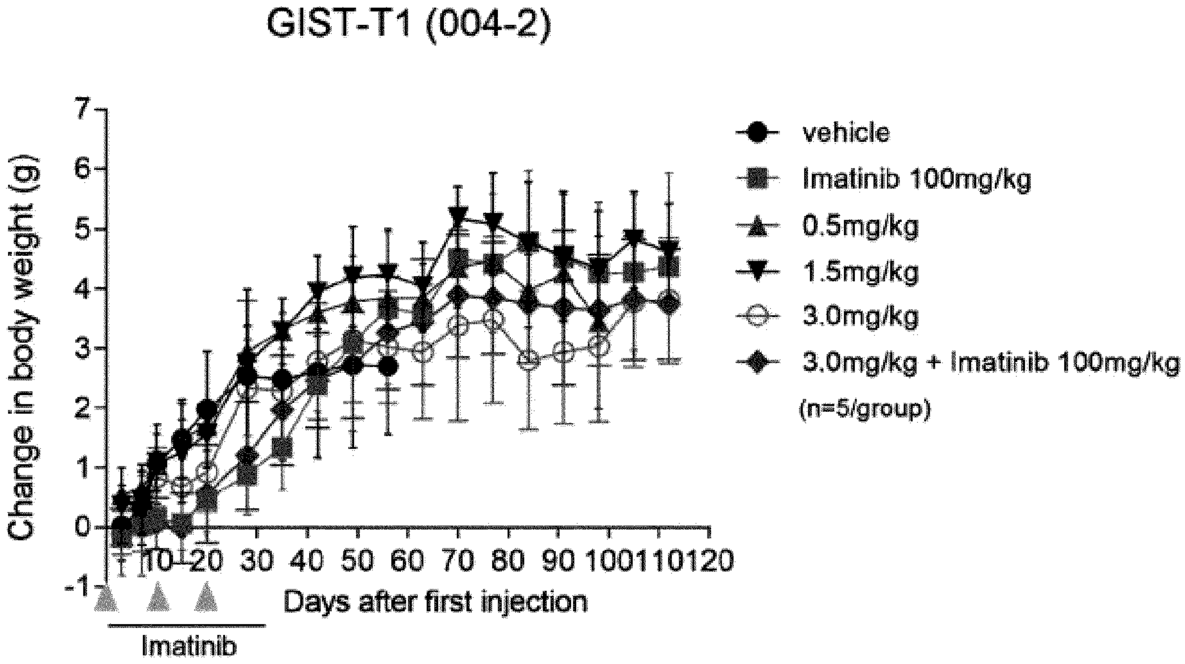
GIST-T1 (004-2)
Change in body weight (g)
Days after first injection
Imatinib
vehicle
Imatinib 100mg/kg
0.5mg/kg
1.5mg/kg
3.0mg/kg
3.0mg/kg + Imatinib 100mg/kg
(n=5/group)

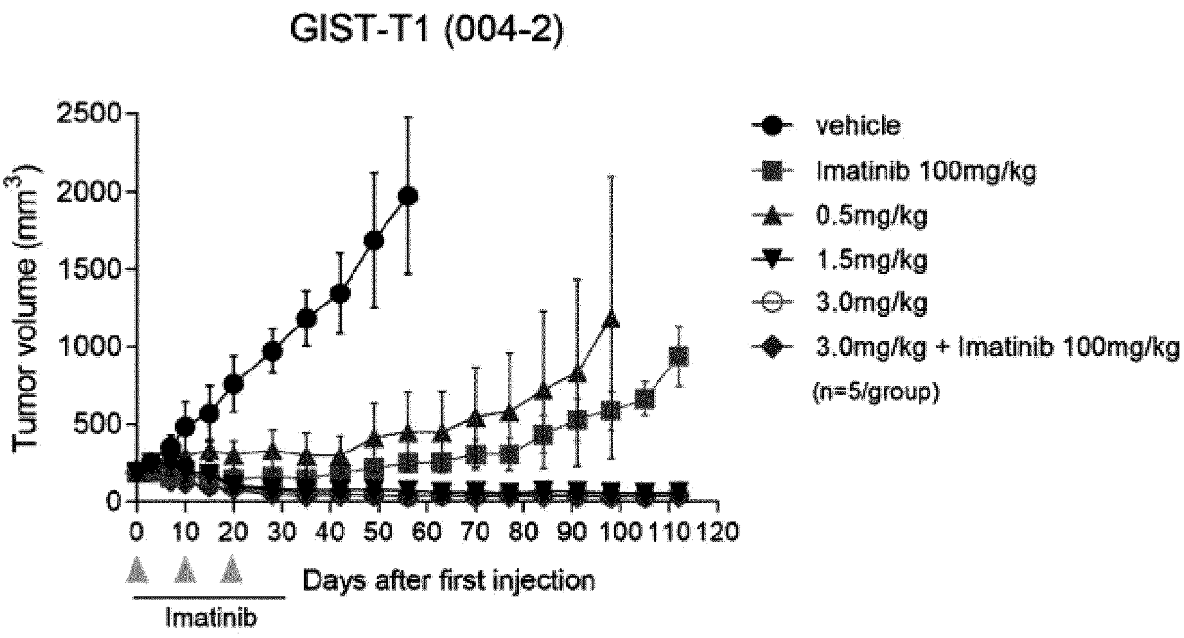
GIST-T1 (004-2)
Tumor volume (mm3)
Days after first injection
Imatinib
vehicle
Imatinib 100mg/kg
0.5mg/kg
1.5mg/kg
3.0mg/kg
3.0mg/kg + Imatinib 100mg/kg
(n=5/group)

Figure 3d

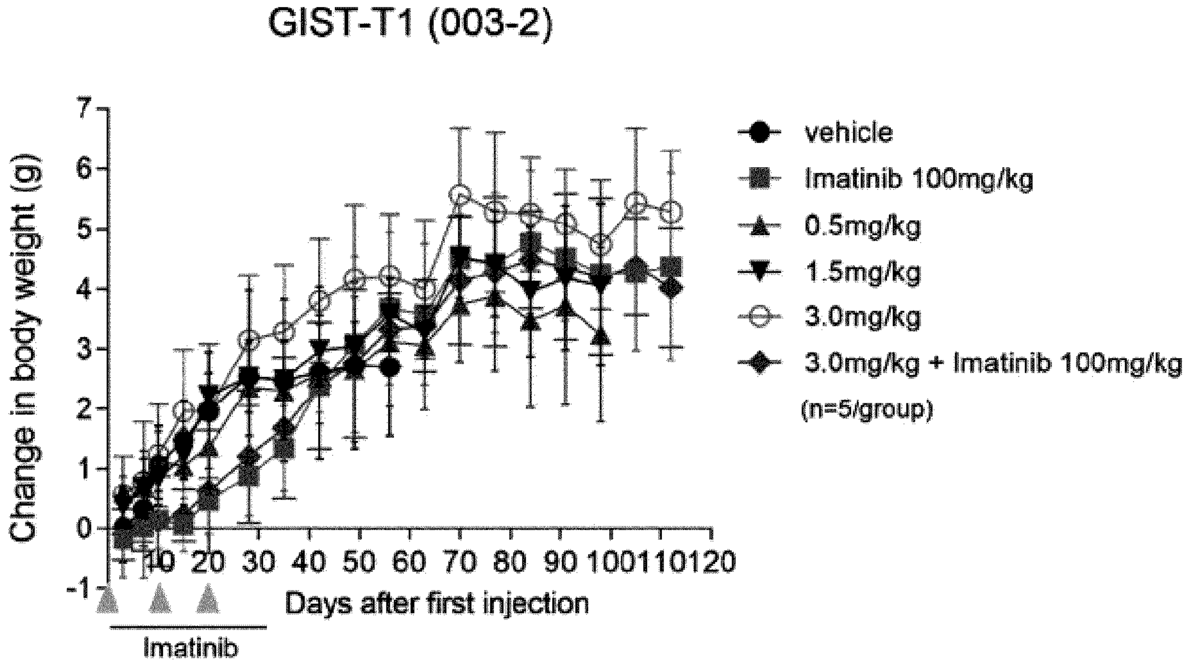
GIST-T1 (003-2)
Change in body weight (g)
Days after first injection
Imatinib
vehicle
Imatinib 100mg/kg
0.5mg/kg
1.5mg/kg
3.0mg/kg
3.0mg/kg + Imatinib 100mg/kg
(n=5/group)

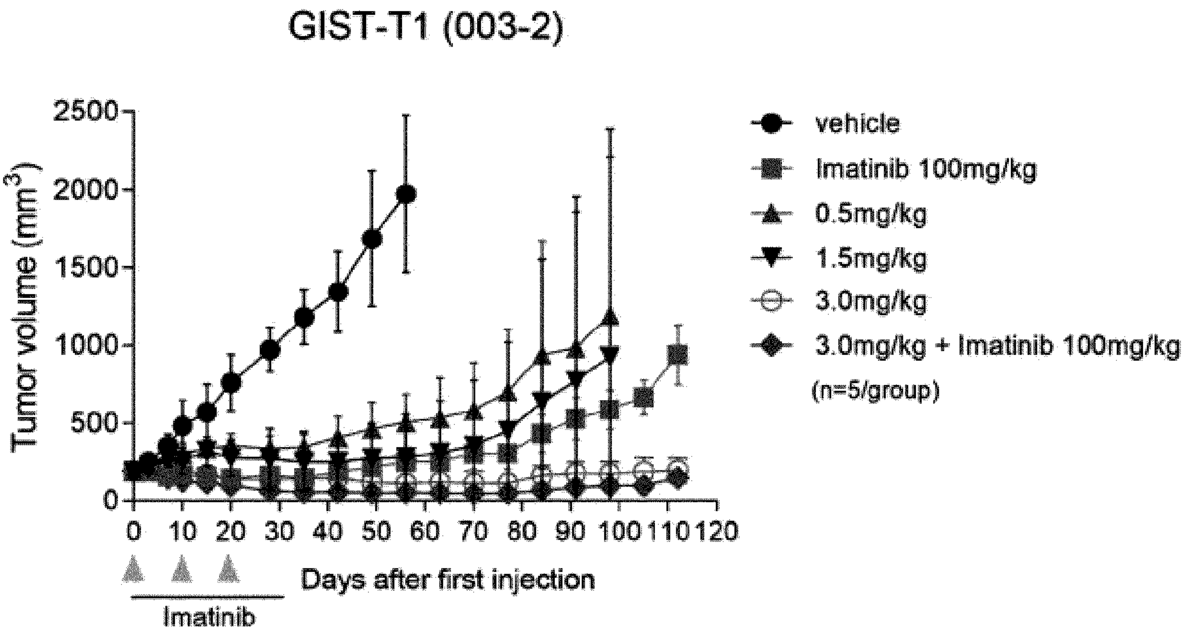
GIST-T1 (003-2)
Tumor volume (mm3)
Days after first injection
Imatinib
vehicle
Imatinib 100mg/kg
0.5mg/kg
1.5mg/kg
3.0mg/kg
3.0mg/kg + Imatinib 100mg/kg
(n=5/group)

Figure 4a

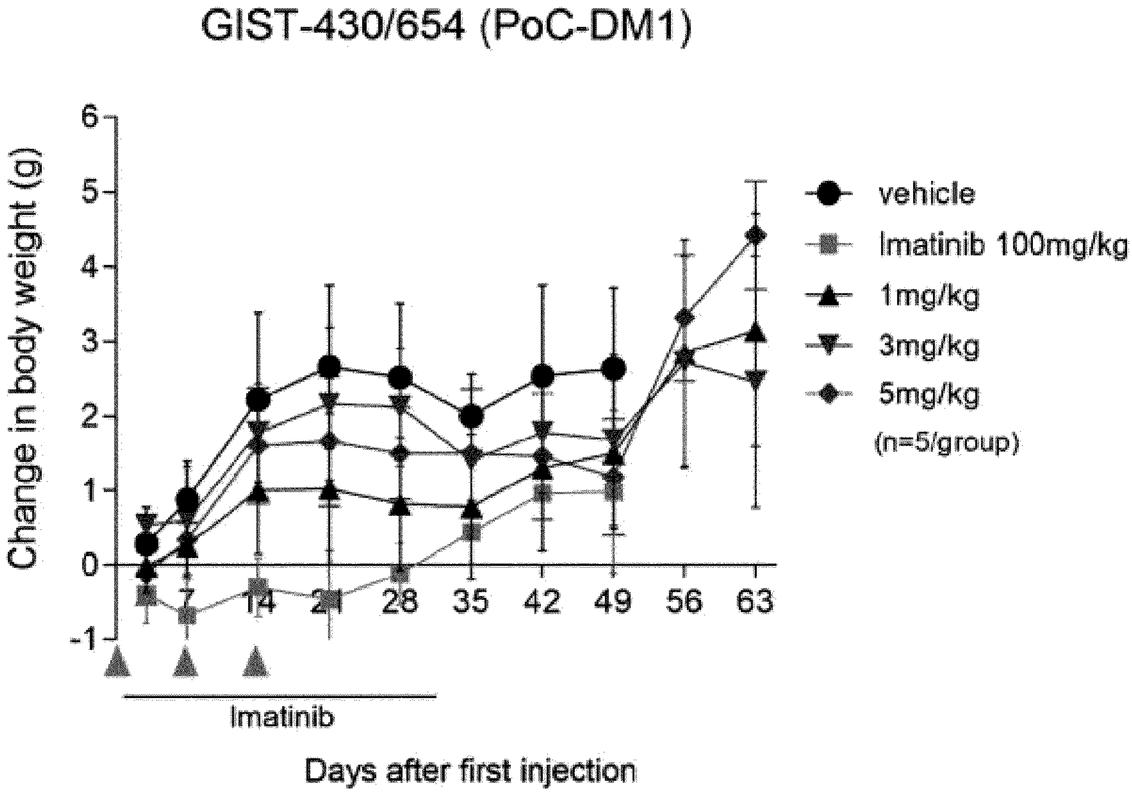
GIST-430/654 (PoC-DM1)
Change in body weight (g)
6
5
4
3
2
1
0
-1
7
14
21
28
35
42
49
56
63
Imatinib
Days after first injection
vehicle
Imatinib 100mg/kg
1mg/kg
3mg/kg
5mg/kg
(n=5/group)

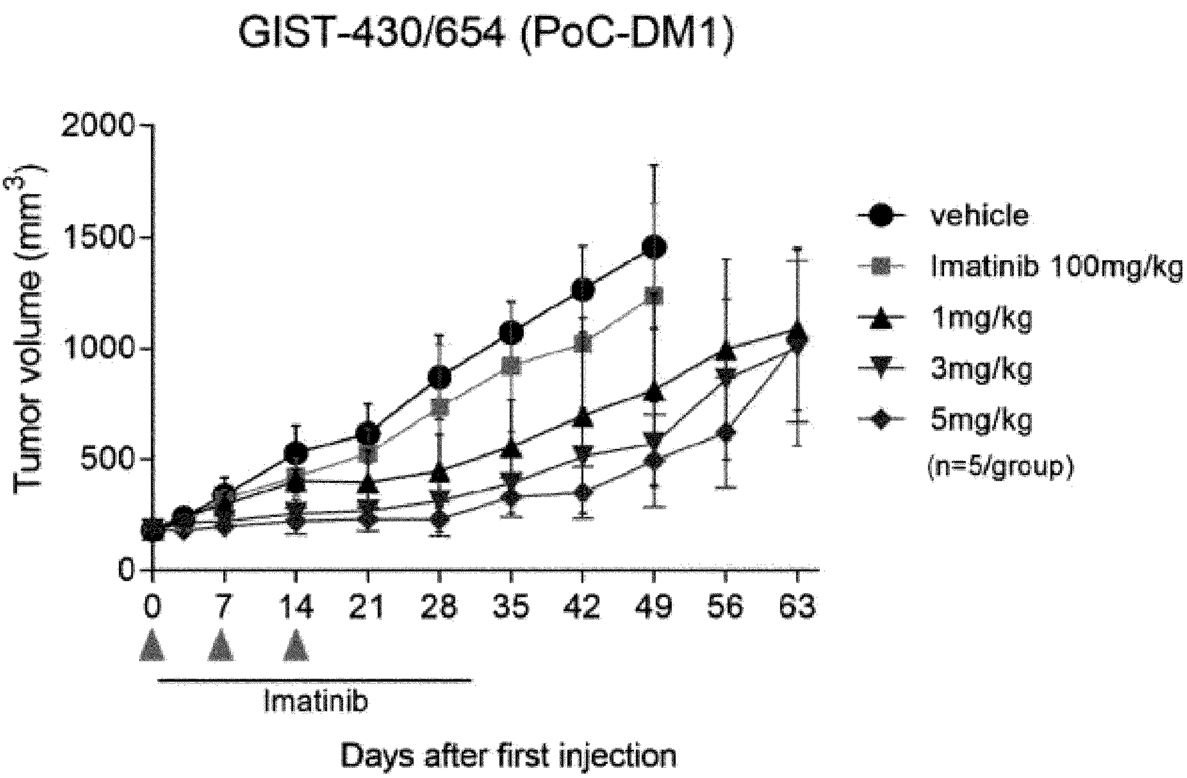
GIST-430/654 (PoC-DM1)
Tumor volume (mm3)
2000
1500
1000
500
0
0
7
14
21
28
35
42
49
56
63
Imatinib
Days after first injection
vehicle
Imatinib 100mg/kg
1mg/kg
3mg/kg
5mg/kg
(n=5/group)

Figure 4b

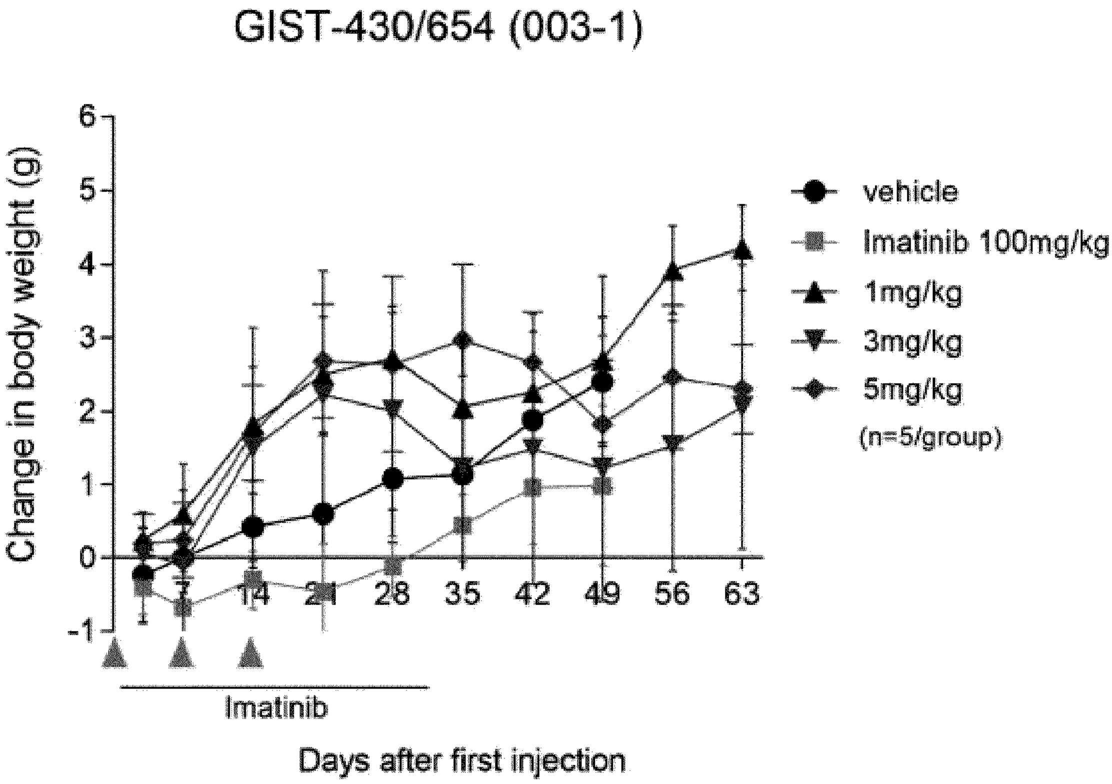
GIST-430/654 (003-1)
Change in body weight (g)
6
5
4
3
2
1
0
-1
7
14
21
28
35
42
49
56
63
Imatinib
Days after first injection
vehicle
Imatinib 100mg/kg
1mg/kg
3mg/kg
5mg/kg
(n=5/group)

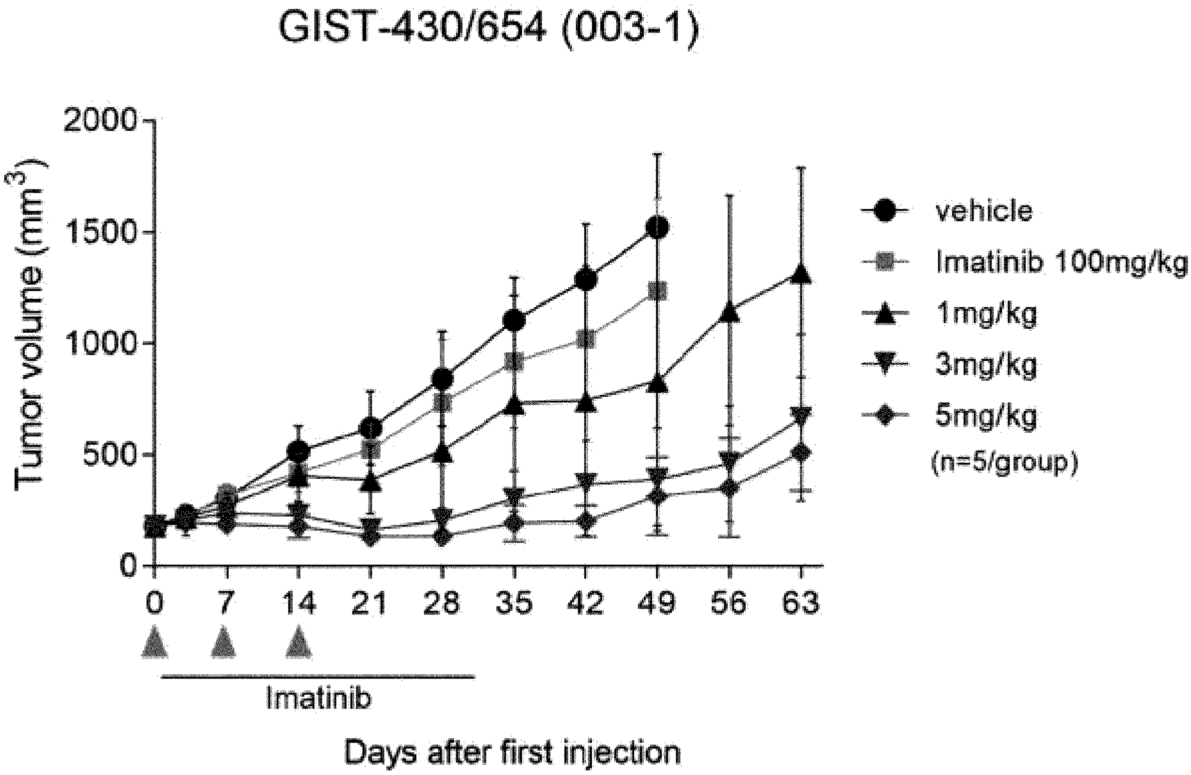
GIST-430/654 (003-1)
Tumor volume (mm3)
2000
1500
1000
500
0
0
7
14
21
28
35
42
49
56
63
Imatinib
Days after first injection
vehicle
Imatinib 100mg/kg
1mg/kg
3mg/kg
5mg/kg
(n=5/group)

Figure 4c

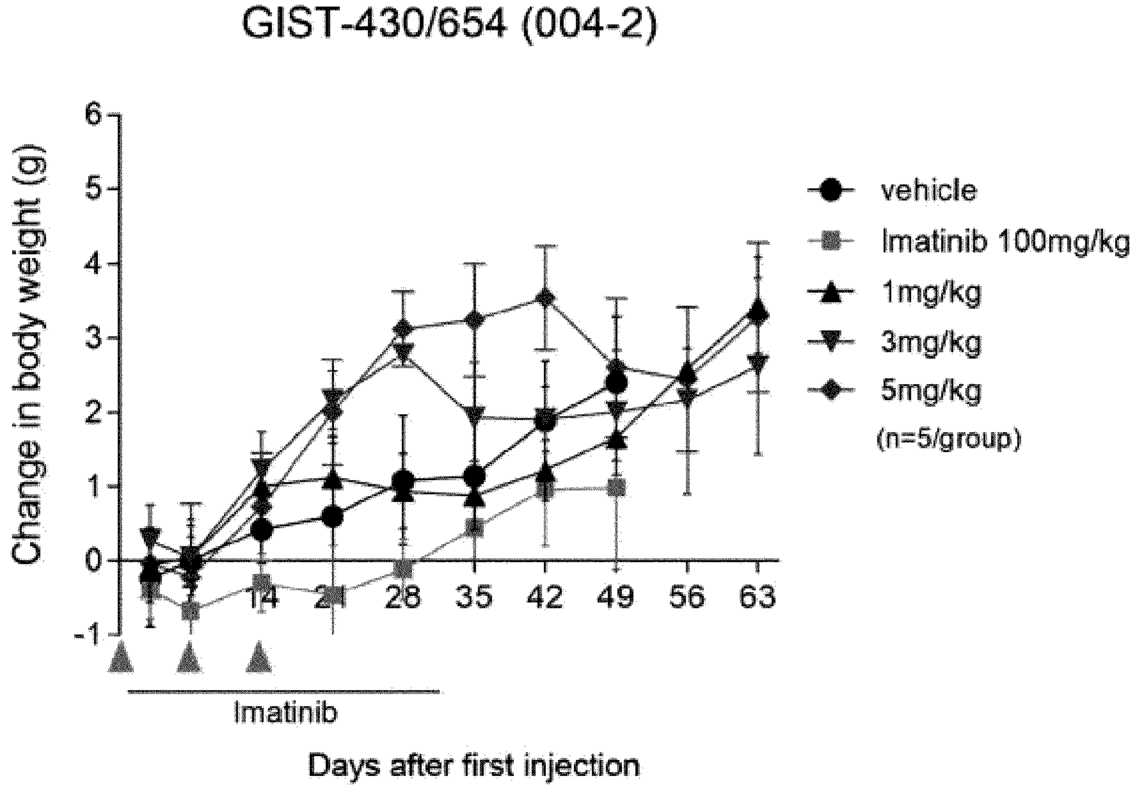
GIST-430/654 (004-2)
Change in body weight (g)
6
5
4
3
2
1
0
-1
14
21
28
35
42
49
56
63
Imatinib
Days after first injection
vehicle
Imatinib 100mg/kg
1mg/kg
3mg/kg
5mg/kg
(n=5/group)

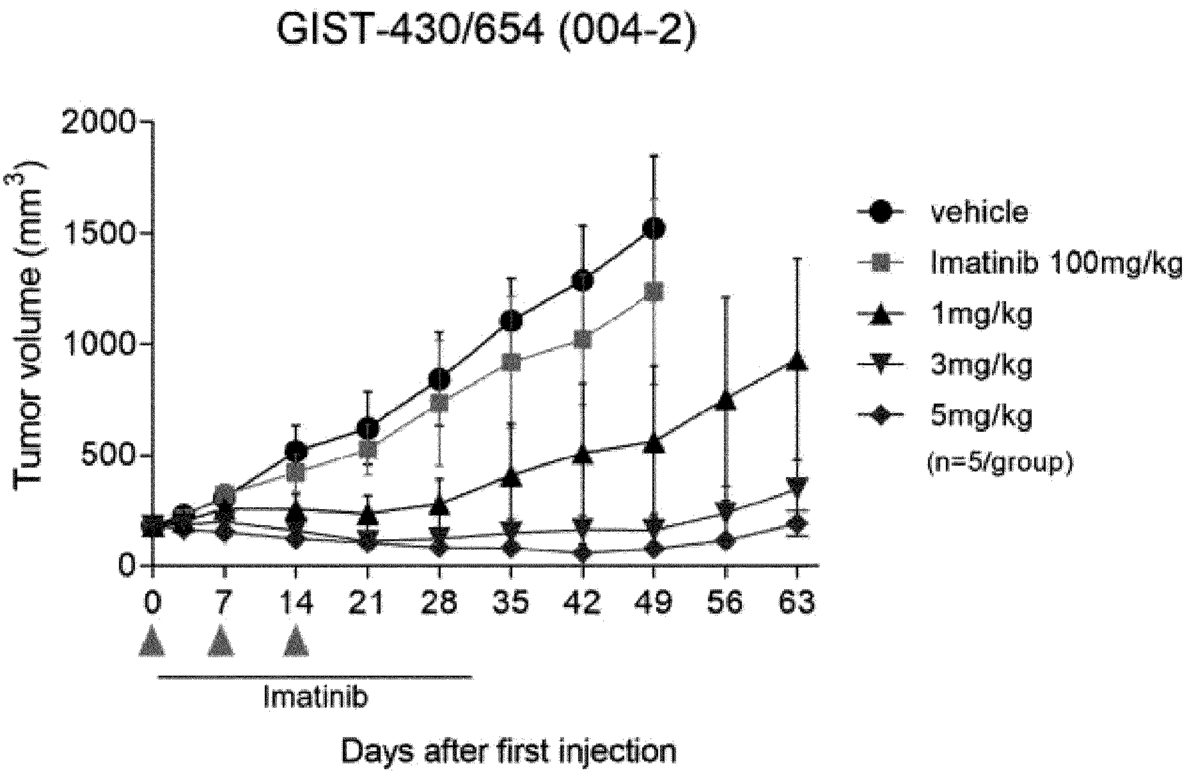
GIST-430/654 (004-2)
Tumor volume (mm3)
2000
1500
1000
500
0
0
7
14
21
28
35
42
49
56
63
Imatinib
Days after first injection
vehicle
Imatinib 100mg/kg
1mg/kg
3mg/kg
5mg/kg
(n=5/group)

Figure 4d

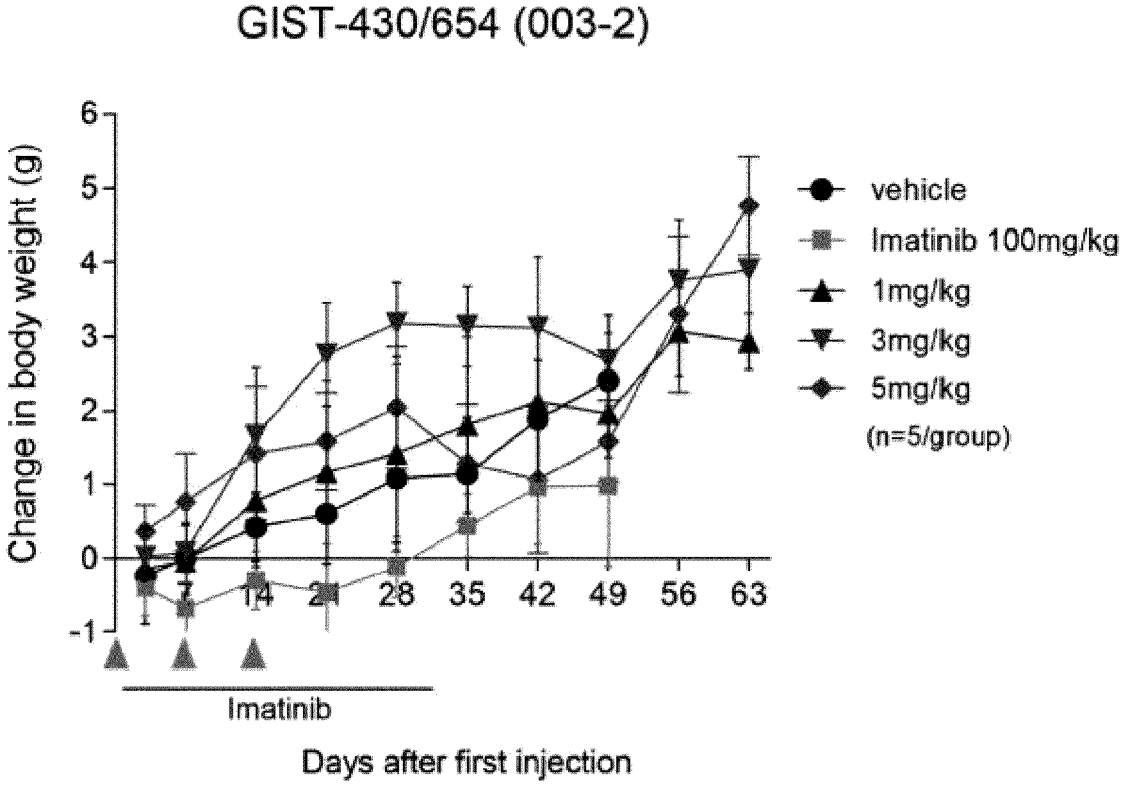
GIST-430/654 (003-2)
Change in body weight (g)
6
5
4
3
2
1
0
-1
7
14
21
28
35
42
49
56
63
Imatinib
Days after first injection
vehicle
Imatinib 100mg/kg
1mg/kg
3mg/kg
5mg/kg
(n=5/group)

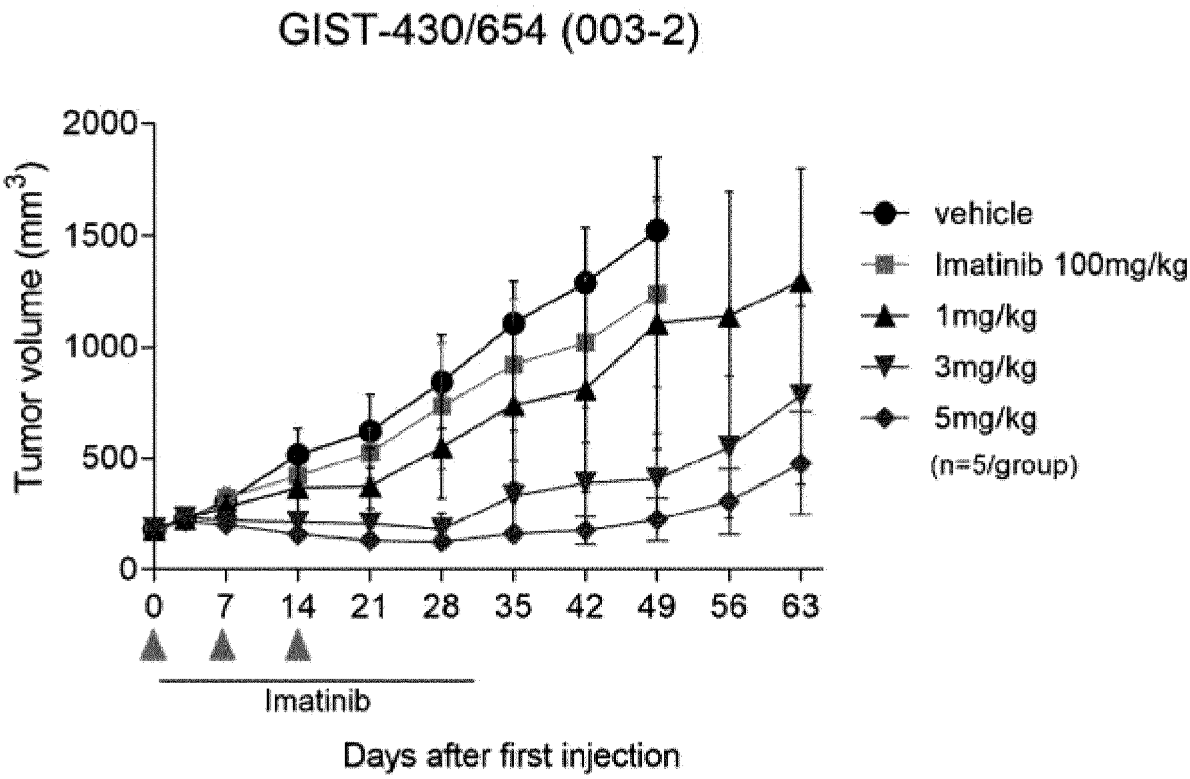
GIST-430/654 (003-2)
Tumor volume (mm³)
2000
1500
1000
500
0
0
7
14
21
28
35
42
49
56
63
Imatinib
Days after first injection
vehicle
Imatinib 100mg/kg
1mg/kg
3mg/kg
5mg/kg
(n=5/group)

Figure 4e

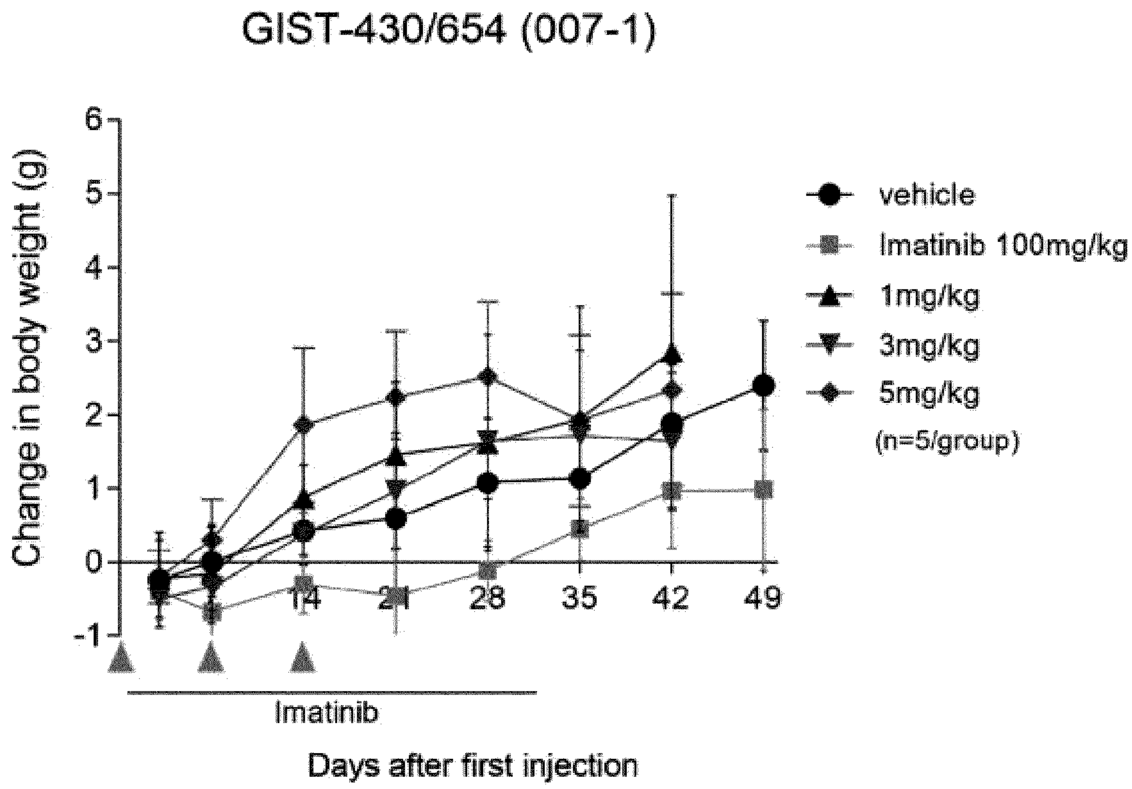
GIST-430/654 (007-1)
Change in body weight (g)
6
5
4
3
2
1
0
-1
14
21
28
35
42
49
vehicle
Imatinib 100mg/kg
1mg/kg
3mg/kg
5mg/kg
(n=5/group)
Imatinib
Days after first injection

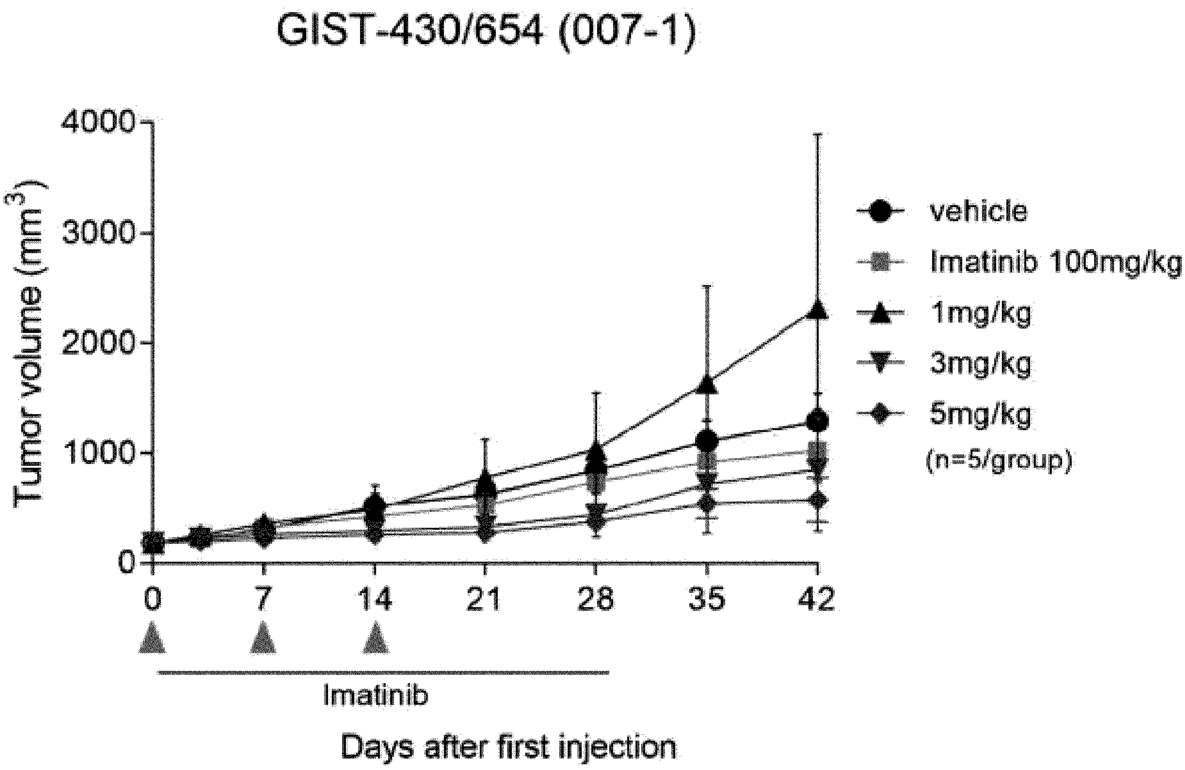
GIST-430/654 (007-1)
Tumor volume (mm3)
4000
3000
2000
1000
0
0
7
14
21
28
35
42
vehicle
Imatinib 100mg/kg
1mg/kg
3mg/kg
5mg/kg
(n=5/group)
Imatinib
Days after first injection

Figure 5a

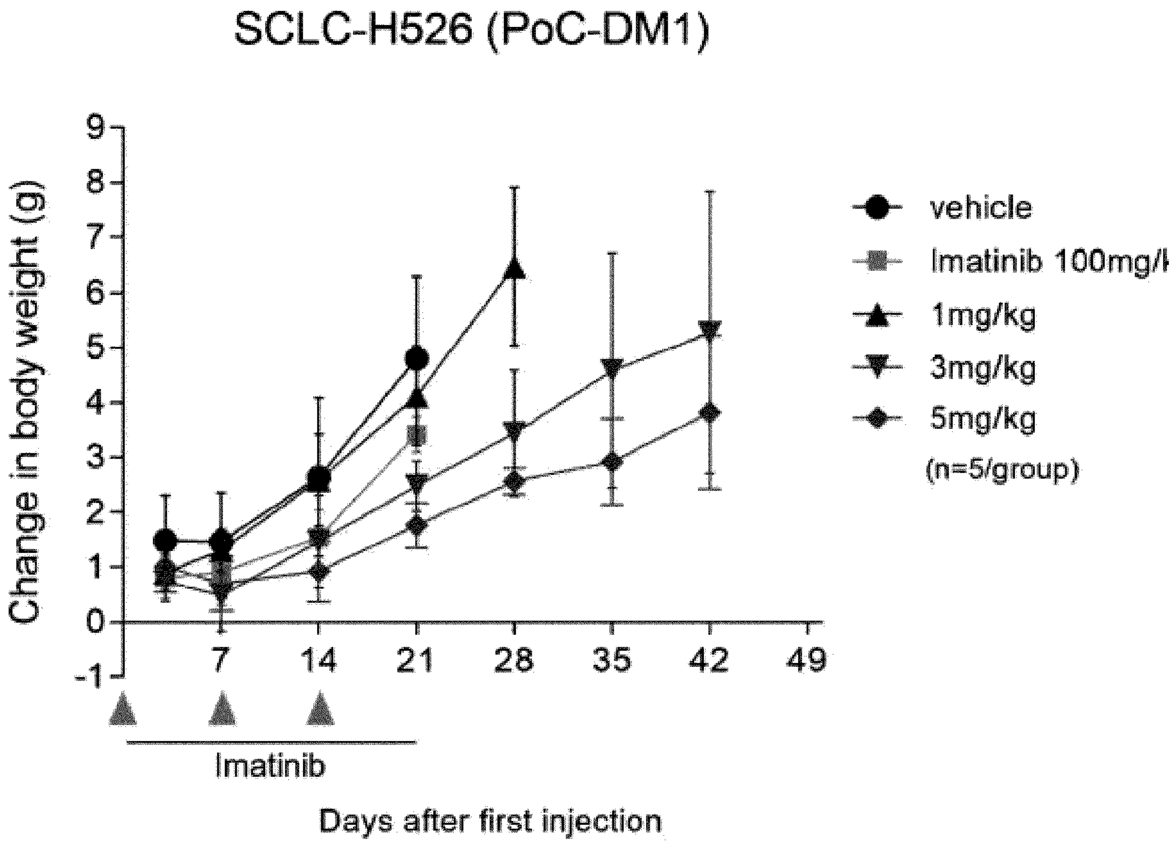
SCLC-H526 (PoC-DM1)
Change in body weight (g)
9
8
7
6
5
4
3
2
1
0
-1
7
14
21
28
35
42
49
vehicle
Imatinib 100mg/k
1mg/kg
3mg/kg
5mg/kg
(n=5/group)
Imatinib
Days after first injection

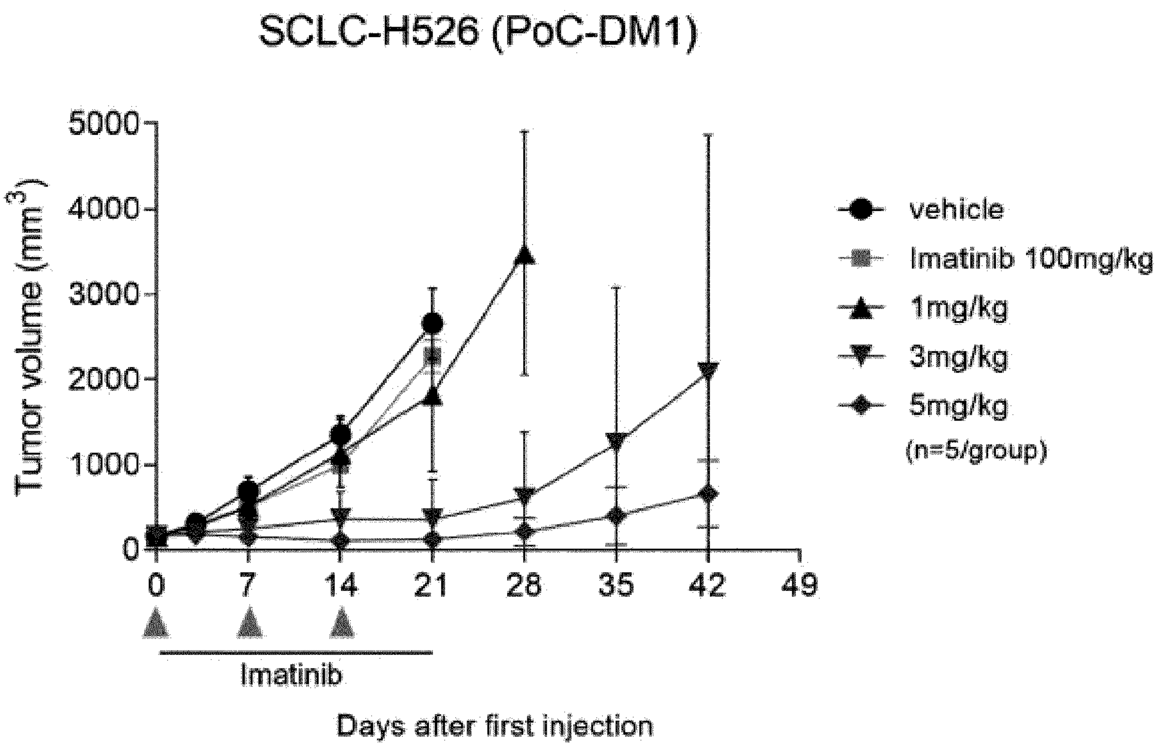
SCLC-H526 (PoC-DM1)
Tumor volume (mm3)
5000
4000
3000
2000
1000
0
0
7
14
21
28
35
42
49
vehicle
Imatinib 100mg/kg
1mg/kg
3mg/kg
5mg/kg
(n=5/group)
Imatinib
Days after first injection

Figure 5b

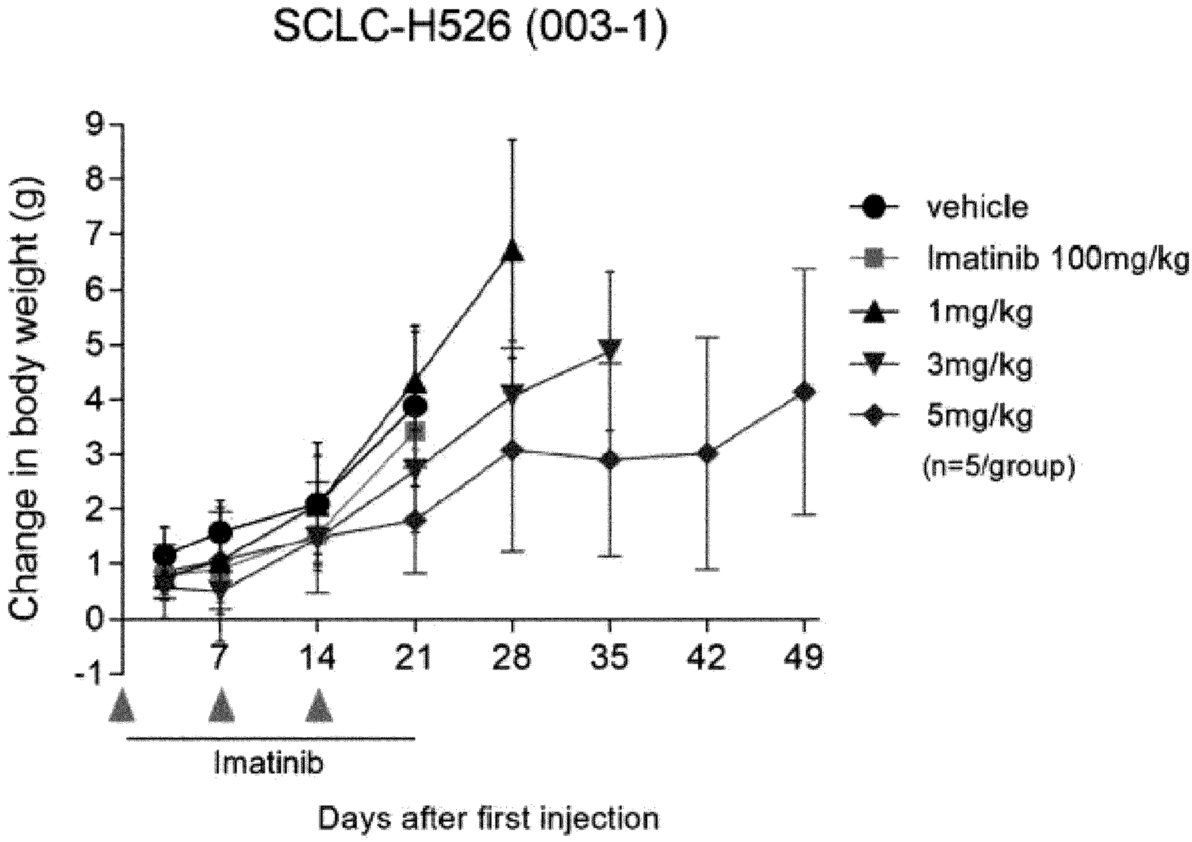
SCLC-H526 (003-1)
Change in body weight (g)
9
8
7
6
5
4
3
2
1
0
-1
7
14
21
28
35
42
49
Imatinib
Days after first injection
vehicle
Imatinib 100mg/kg
1mg/kg
3mg/kg
5mg/kg
(n=5/group)

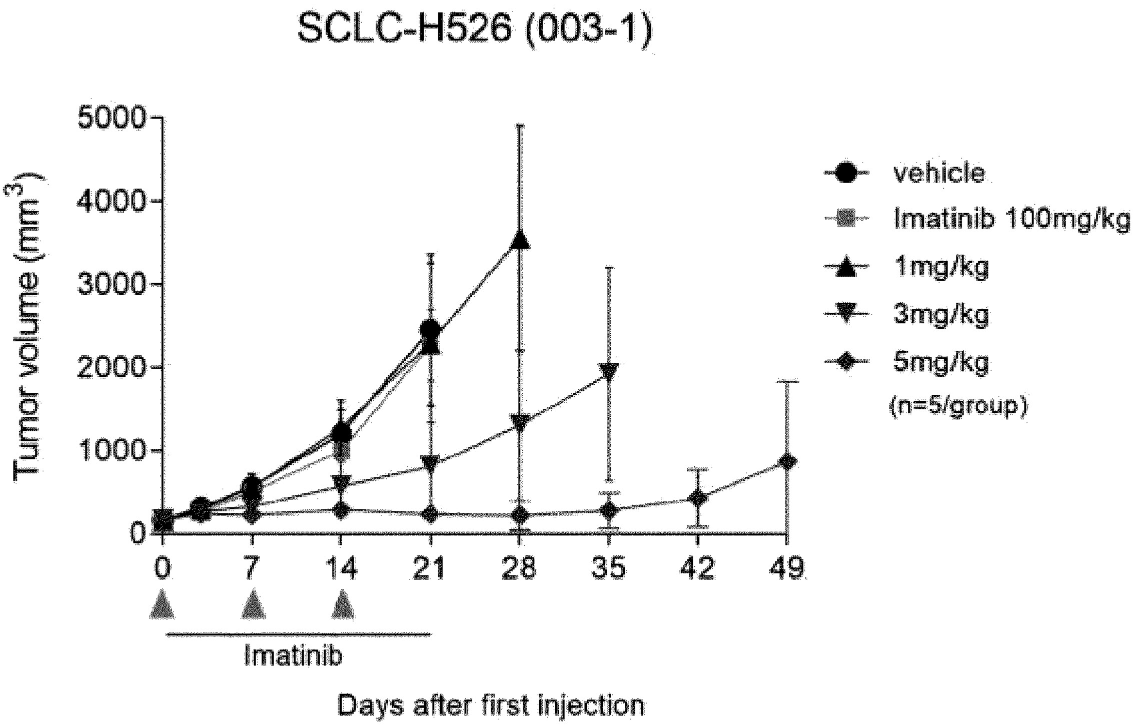
SCLC-H526 (003-1)
Tumor volume (mm3)
5000
4000
3000
2000
1000
0
0
7
14
21
28
35
42
49
Imatinib
Days after first injection
vehicle
Imatinib 100mg/kg
1mg/kg
3mg/kg
5mg/kg
(n=5/group)

Figure 5c

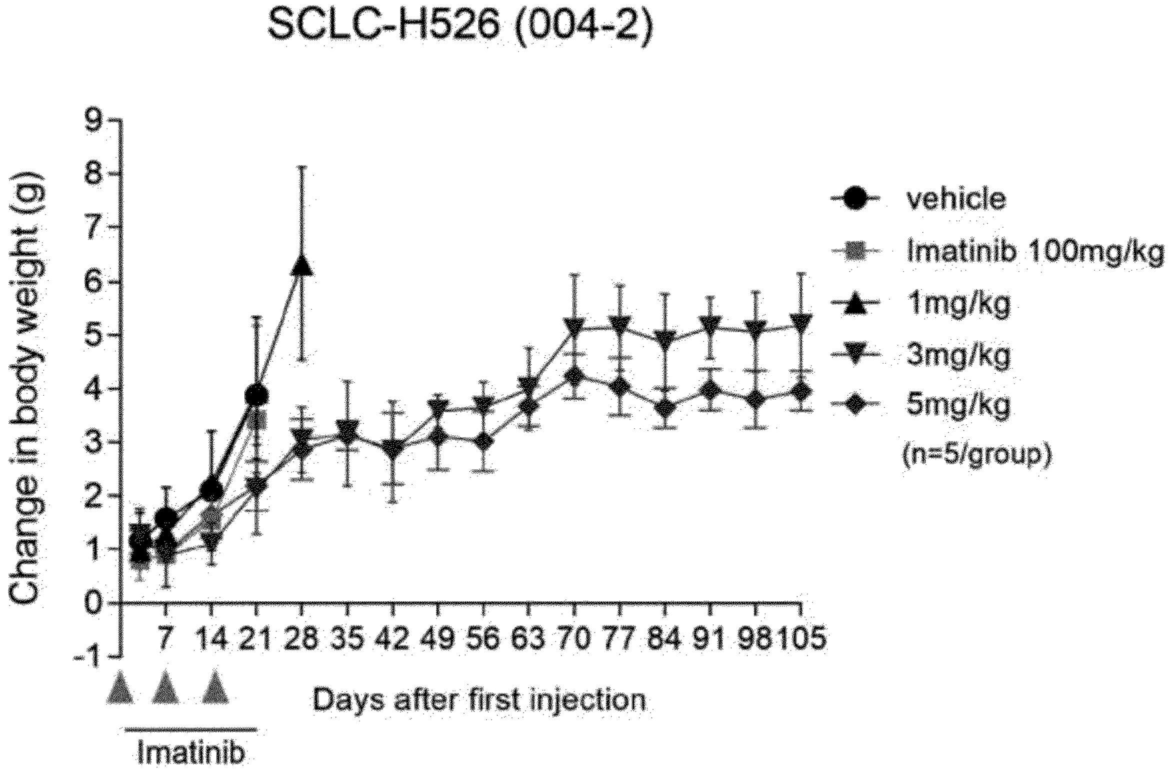
SCLC-H526 (004-2)
Change in body weight (g)
9
8
7
6
5
4
3
2
1
0
-1
7 14 21 28 35 42 49 56 63 70 77 84 91 98 105
Days after first injection
Imatinib
vehicle
Imatinib 100mg/kg
1mg/kg
3mg/kg
5mg/kg
(n=5/group)

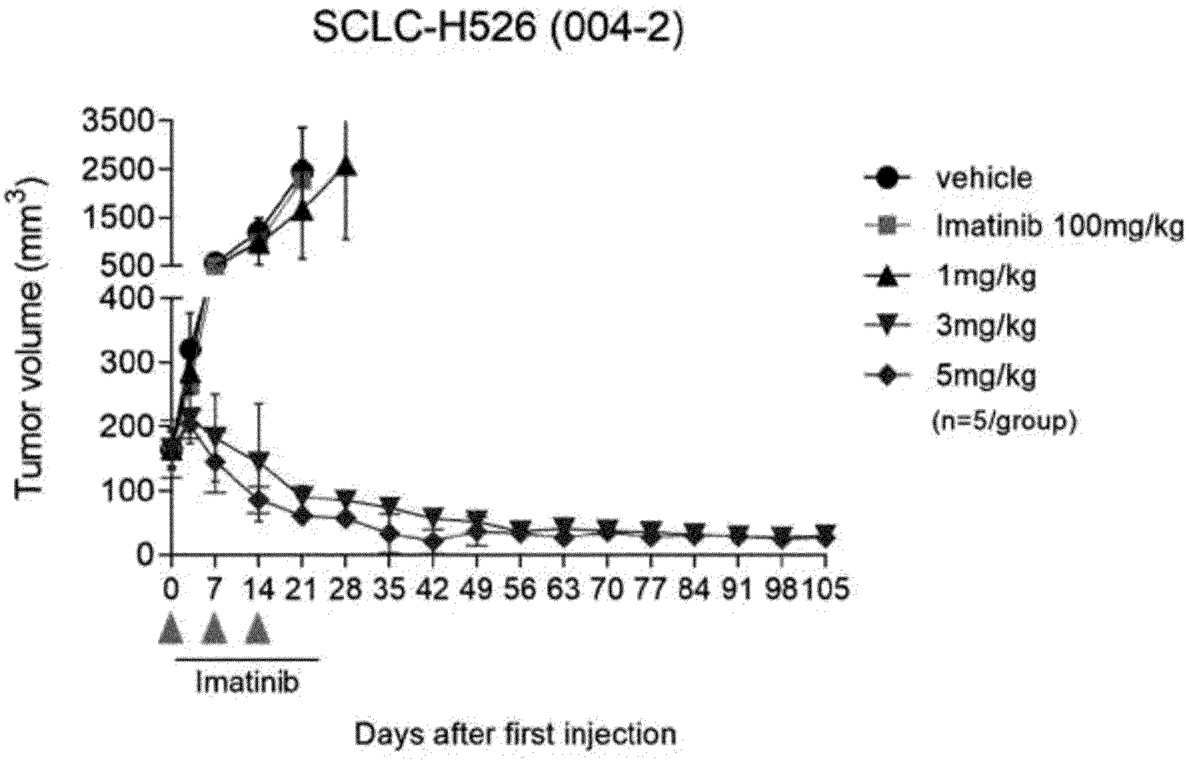
SCLC-H526 (004-2)
Tumor volume (mm³)
3500
2500
1500
500
400
300
200
100
0
0 7 14 21 28 35 42 49 56 63 70 77 84 91 98 105
Imatinib
Days after first injection
vehicle
Imatinib 100mg/kg
1mg/kg
3mg/kg
5mg/kg
(n=5/group)

Figure 5d

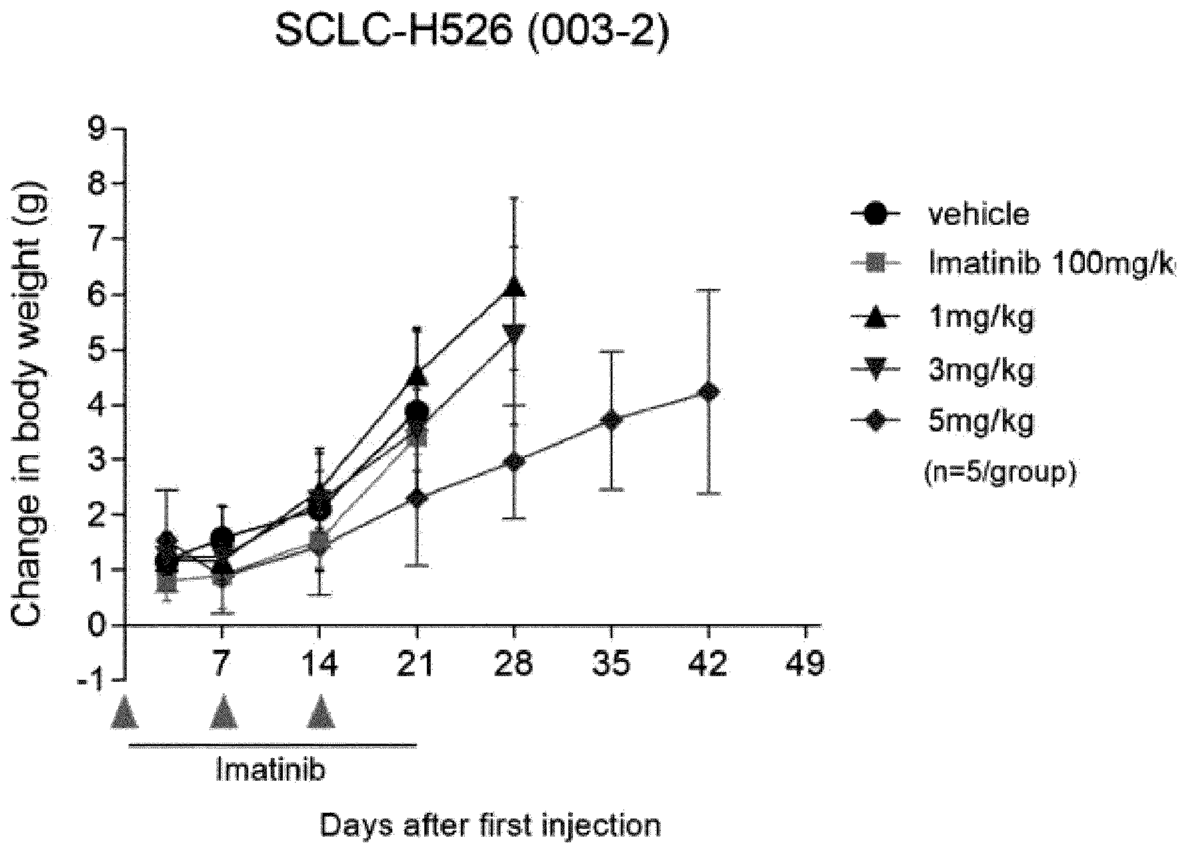
SCLC-H526 (003-2)
Change in body weight (g)
9
8
7
6
5
4
3
2
1
0
-1
7
14
21
28
35
42
49
Imatinib
Days after first injection
vehicle
Imatinib 100mg/k
1mg/kg
3mg/kg
5mg/kg
(n=5/group)

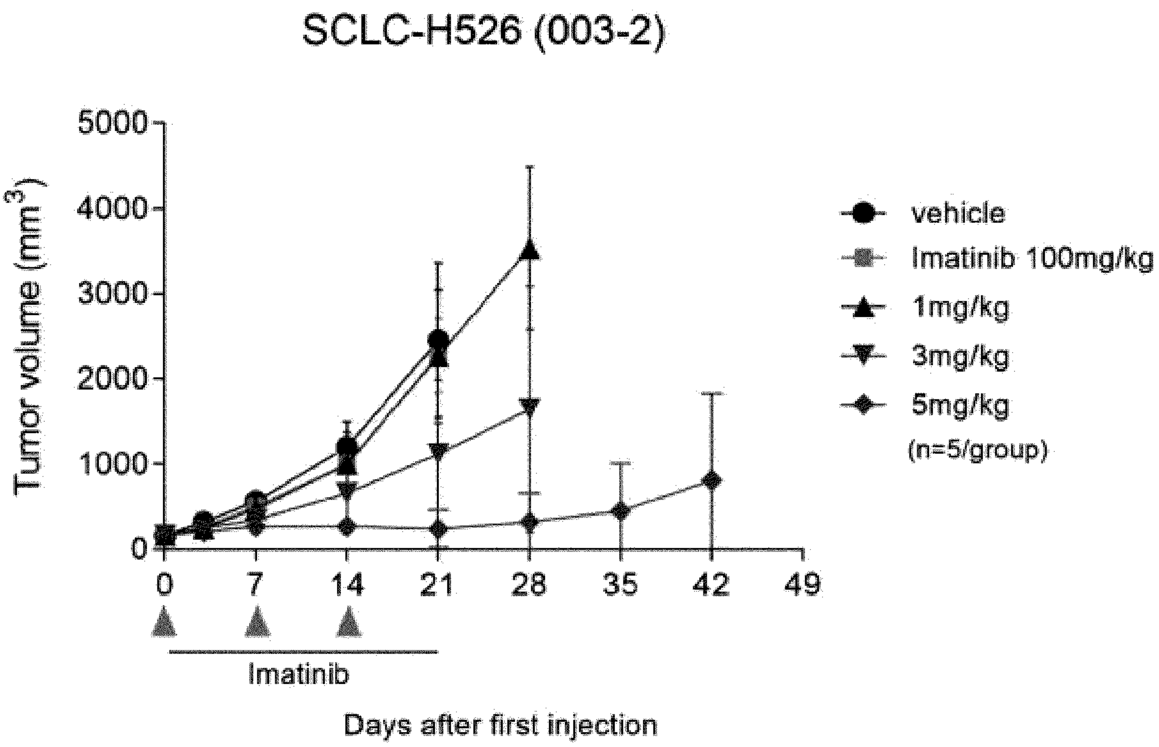
SCLC-H526 (003-2)
Tumor volume (mm3)
5000
4000
3000
2000
1000
0
0
7
14
21
28
35
42
49
Imatinib
Days after first injection
vehicle
Imatinib 100mg/kg
1mg/kg
3mg/kg
5mg/kg
(n=5/group)

Figure 6

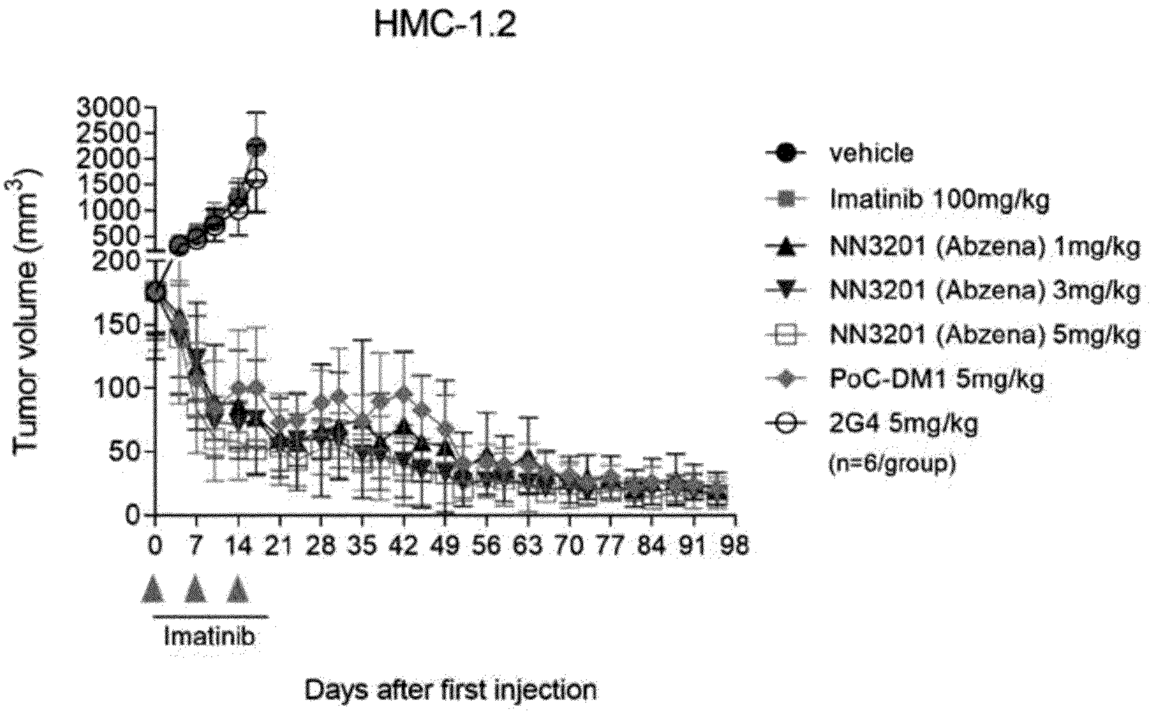
HMC-1.2
Tumor volume (mm$^3$)
3000
2500
2000
1500
1000
500
200
150
100
50
0
0 7 14 21 28 35 42 49 56 63 70 77 84 91 98
Imatinib
Days after first injection
vehicle
Imatinib 100mg/kg
NN3201 (Abzena) 1mg/kg
NN3201 (Abzena) 3mg/kg
NN3201 (Abzena) 5mg/kg
PoC-DM1 5mg/kg
2G4 5mg/kg
(n=6/group)

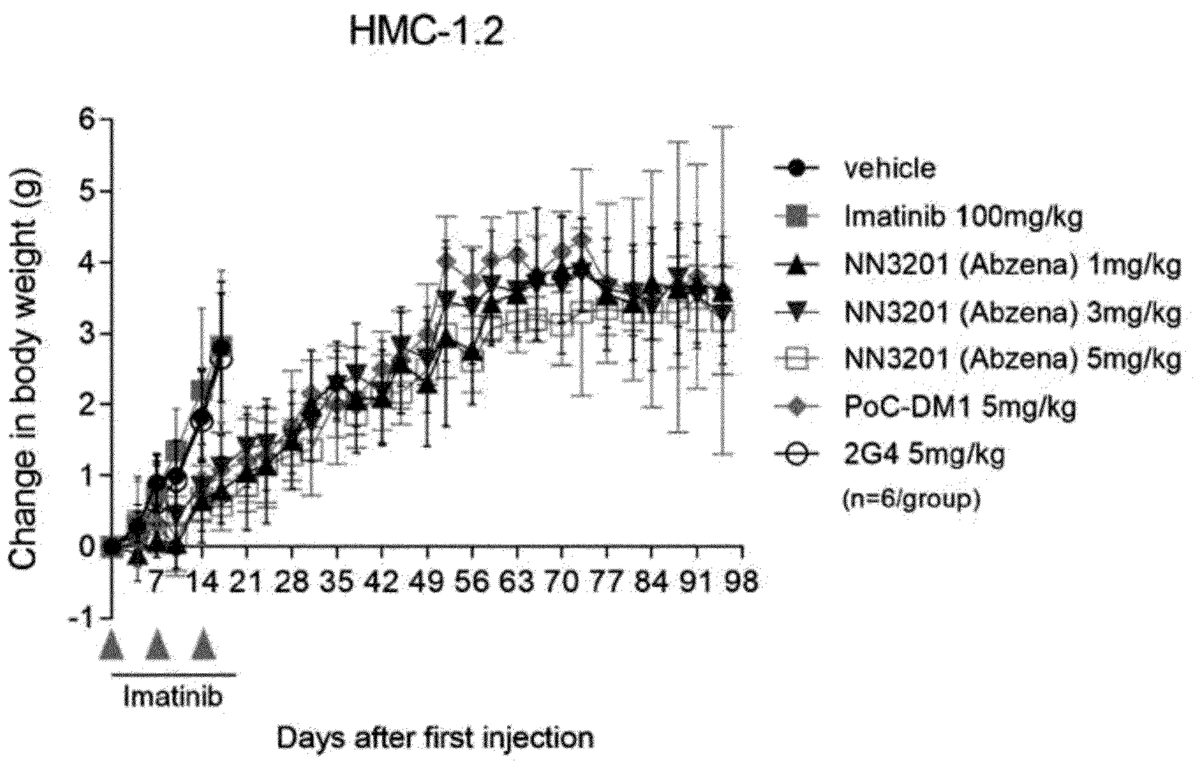
HMC-1.2
Change in body weight (g)
6
5
4
3
2
1
0
-1
7 14 21 28 35 42 49 56 63 70 77 84 91 98
Imatinib
Days after first injection
vehicle
Imatinib 100mg/kg
NN3201 (Abzena) 1mg/kg
NN3201 (Abzena) 3mg/kg
NN3201 (Abzena) 5mg/kg
PoC-DM1 5mg/kg
2G4 5mg/kg
(n=6/group)

Figure 7

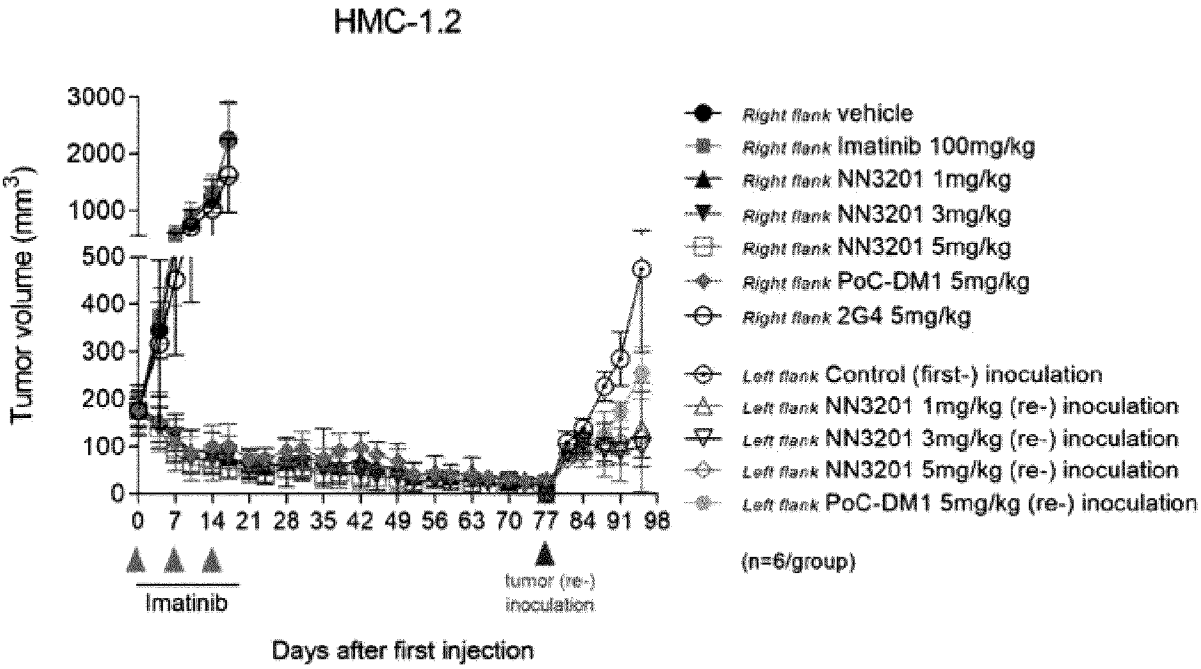
HMC-1.2
Tumor volume (mm3)
3000
2000
1000
500
400
300
200
100
0
0 7 14 21 28 35 42 49 56 63 70 77 84 91 98
Imatinib
tumor (re-) inoculation
Days after first injection
Right flank vehicle
Right flank Imatinib 100mg/kg
Right flank NN3201 1mg/kg
Right flank NN3201 3mg/kg
Right flank NN3201 5mg/kg
Right flank PoC-DM1 5mg/kg
Right flank 2G4 5mg/kg
Left flank Control (first-) inoculation
Left flank NN3201 1mg/kg (re-) inoculation
Left flank NN3201 3mg/kg (re-) inoculation
Left flank NN3201 5mg/kg (re-) inoculation
Left flank PoC-DM1 5mg/kg (re-) inoculation
(n=6/group)

Figure 8

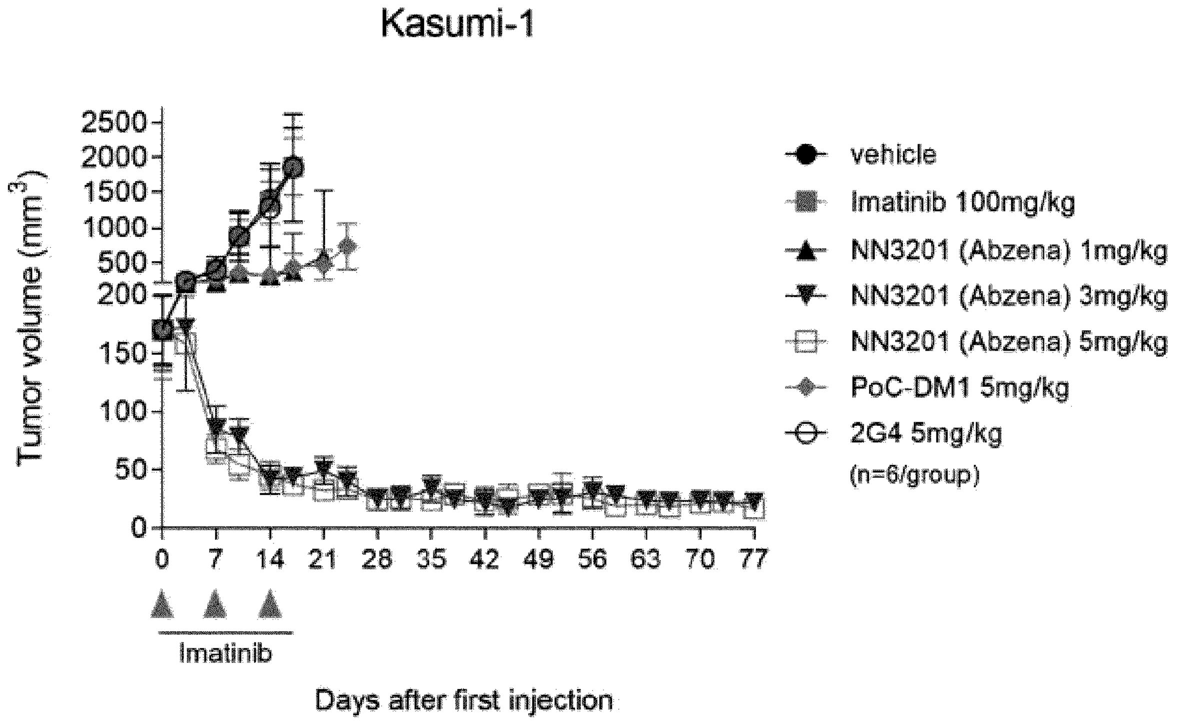
Kasumi-1
Tumor volume (mm3)
2500
2000
1500
1000
500
200
150
100
50
0
0 7 14 21 28 35 42 49 56 63 70 77
Imatinib
Days after first injection
vehicle
Imatinib 100mg/kg
NN3201 (Abzena) 1mg/kg
NN3201 (Abzena) 3mg/kg
NN3201 (Abzena) 5mg/kg
PoC-DM1 5mg/kg
2G4 5mg/kg
(n=6/group)

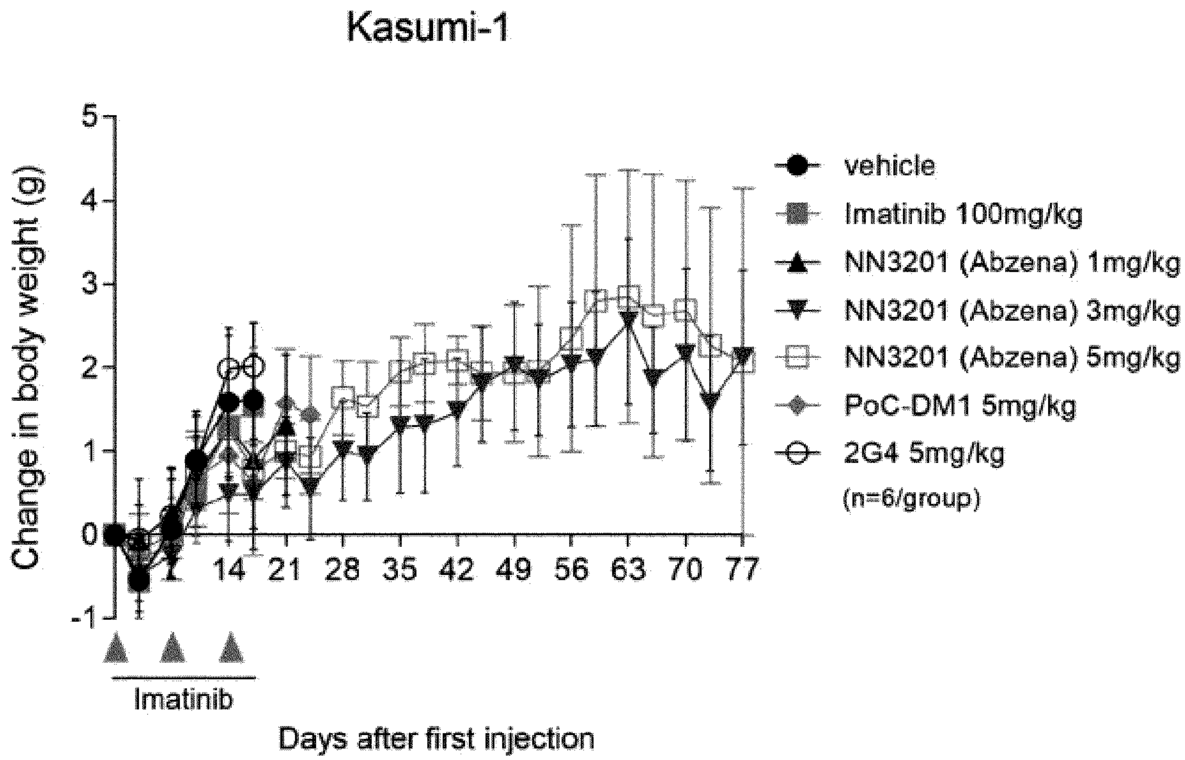
Kasumi-1
Change in body weight (g)
5
4
3
2
1
0
-1
14 21 28 35 42 49 56 63 70 77
Imatinib
Days after first injection
vehicle
Imatinib 100mg/kg
NN3201 (Abzena) 1mg/kg
NN3201 (Abzena) 3mg/kg
NN3201 (Abzena) 5mg/kg
PoC-DM1 5mg/kg
2G4 5mg/kg
(n=6/group)

Figure 9a

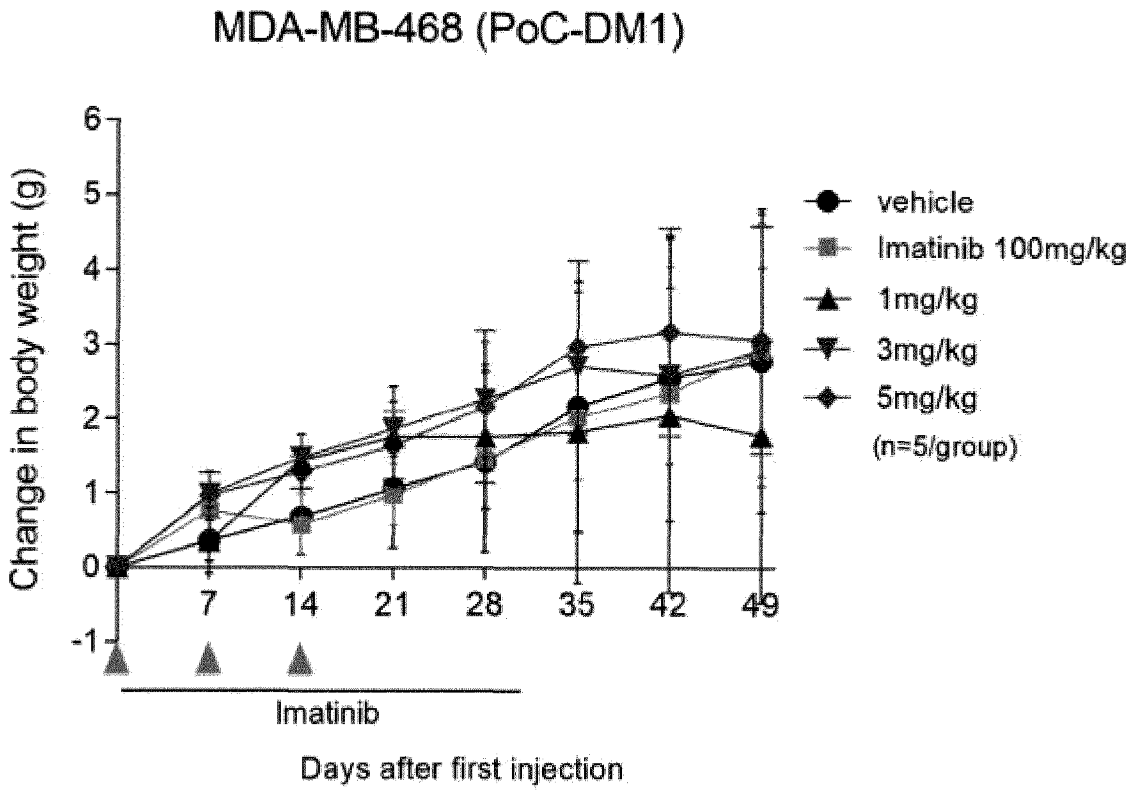
MDA-MB-468 (PoC-DM1)
Change in body weight (g)
6
5
4
3
2
1
0
-1
7
14
21
28
35
42
49
vehicle
Imatinib 100mg/kg
1mg/kg
3mg/kg
5mg/kg
(n=5/group)
Imatinib
Days after first injection

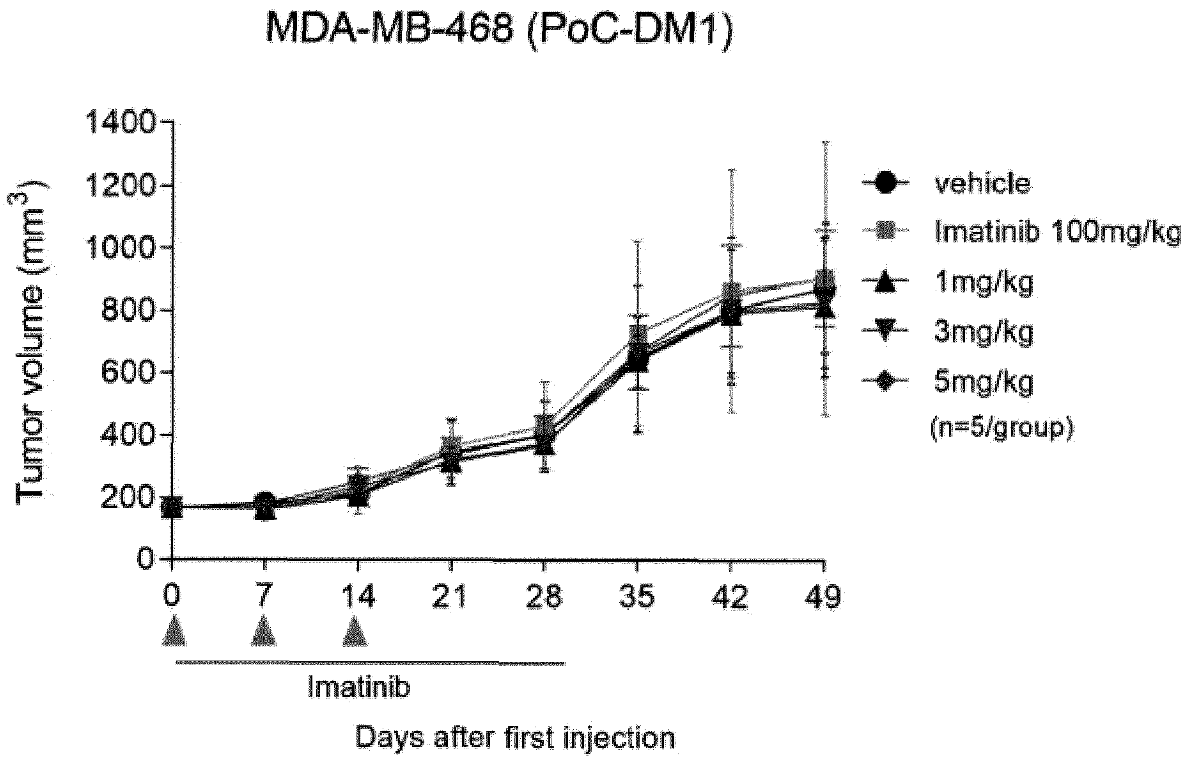
MDA-MB-468 (PoC-DM1)
Tumor volume (mm3)
1400
1200
1000
800
600
400
200
0
0
7
14
21
28
35
42
49
vehicle
Imatinib 100mg/kg
1mg/kg
3mg/kg
5mg/kg
(n=5/group)
Imatinib
Days after first injection

Figure 9b

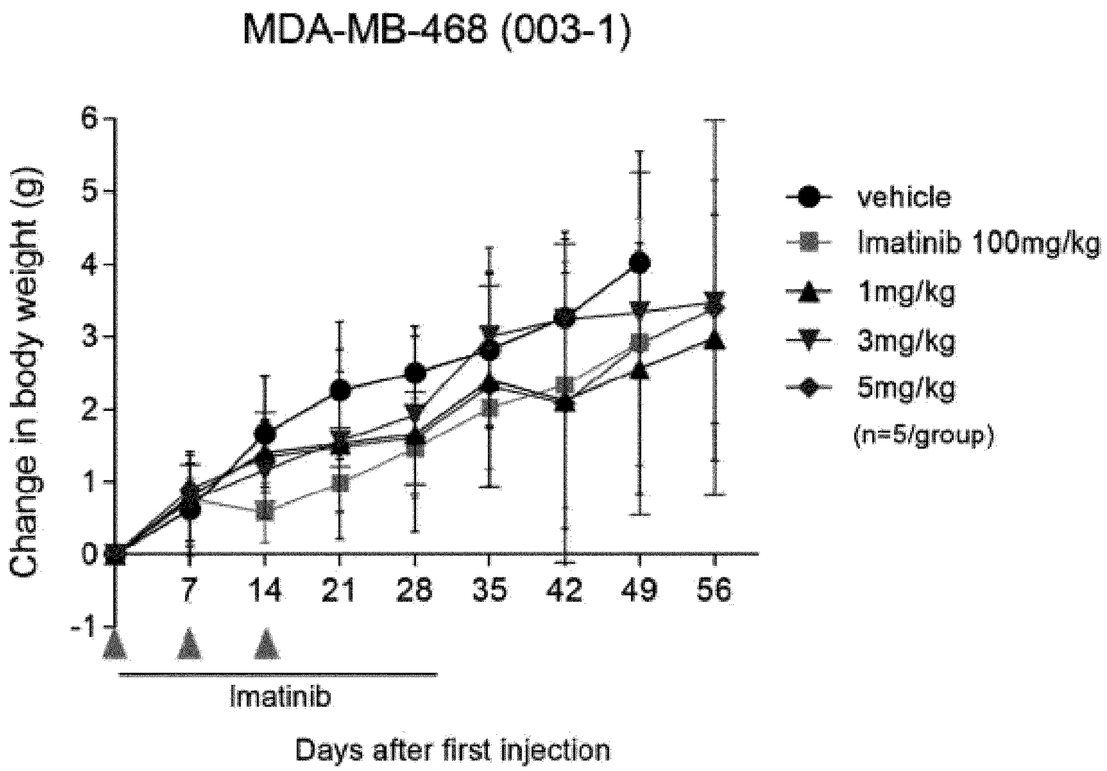
MDA-MB-468 (003-1)
Change in body weight (g)
6
5
4
3
2
1
0
-1
7
14
21
28
35
42
49
56
Imatinib
Days after first injection
vehicle
Imatinib 100mg/kg
1mg/kg
3mg/kg
5mg/kg
(n=5/group)

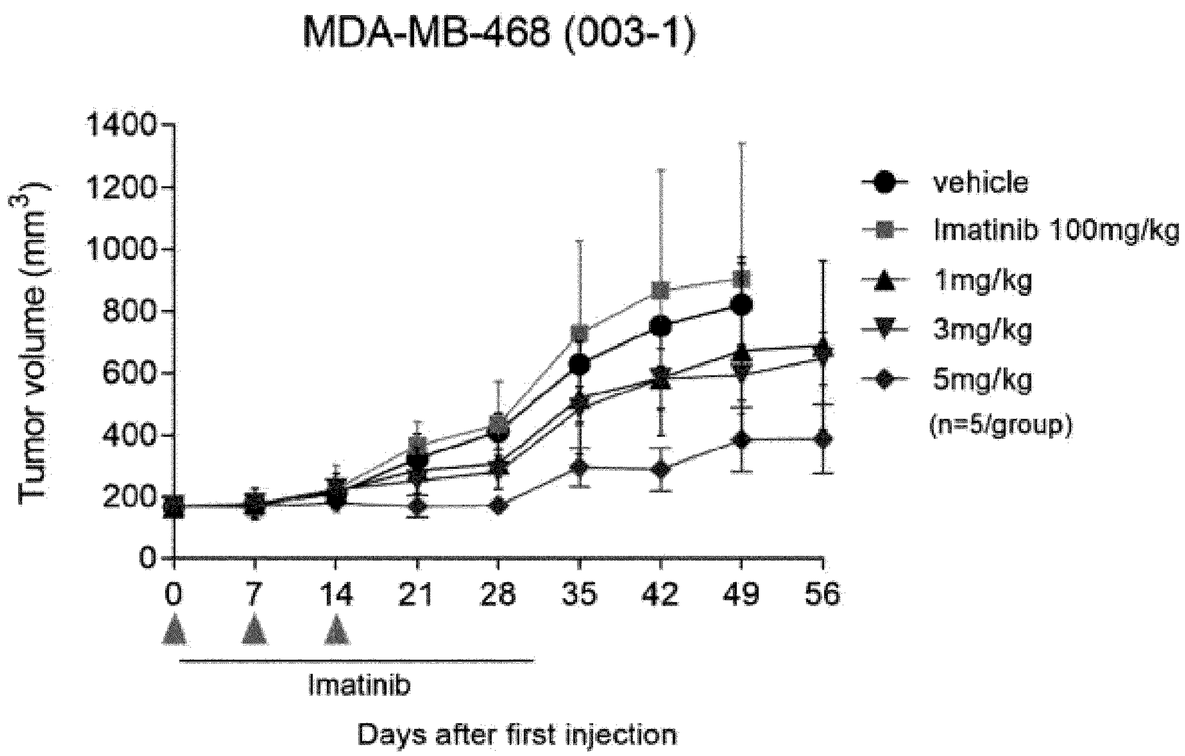
MDA-MB-468 (003-1)
Tumor volume (mm3)
1400
1200
1000
800
600
400
200
0
0
7
14
21
28
35
42
49
56
Imatinib
Days after first injection
vehicle
Imatinib 100mg/kg
1mg/kg
3mg/kg
5mg/kg
(n=5/group)

Figure 9c

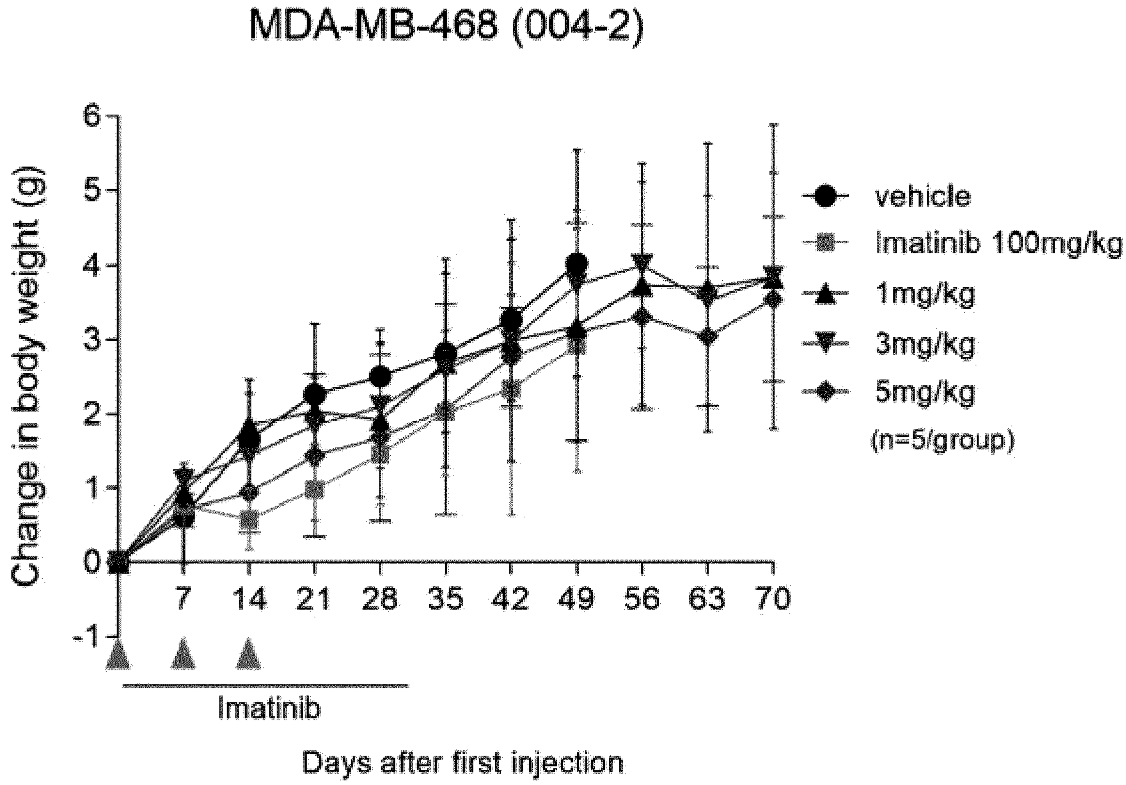
MDA-MB-468 (004-2)
Change in body weight (g)
6
5
4
3
2
1
0
-1
7 14 21 28 35 42 49 56 63 70
vehicle
Imatinib 100mg/kg
1mg/kg
3mg/kg
5mg/kg
(n=5/group)
Imatinib
Days after first injection

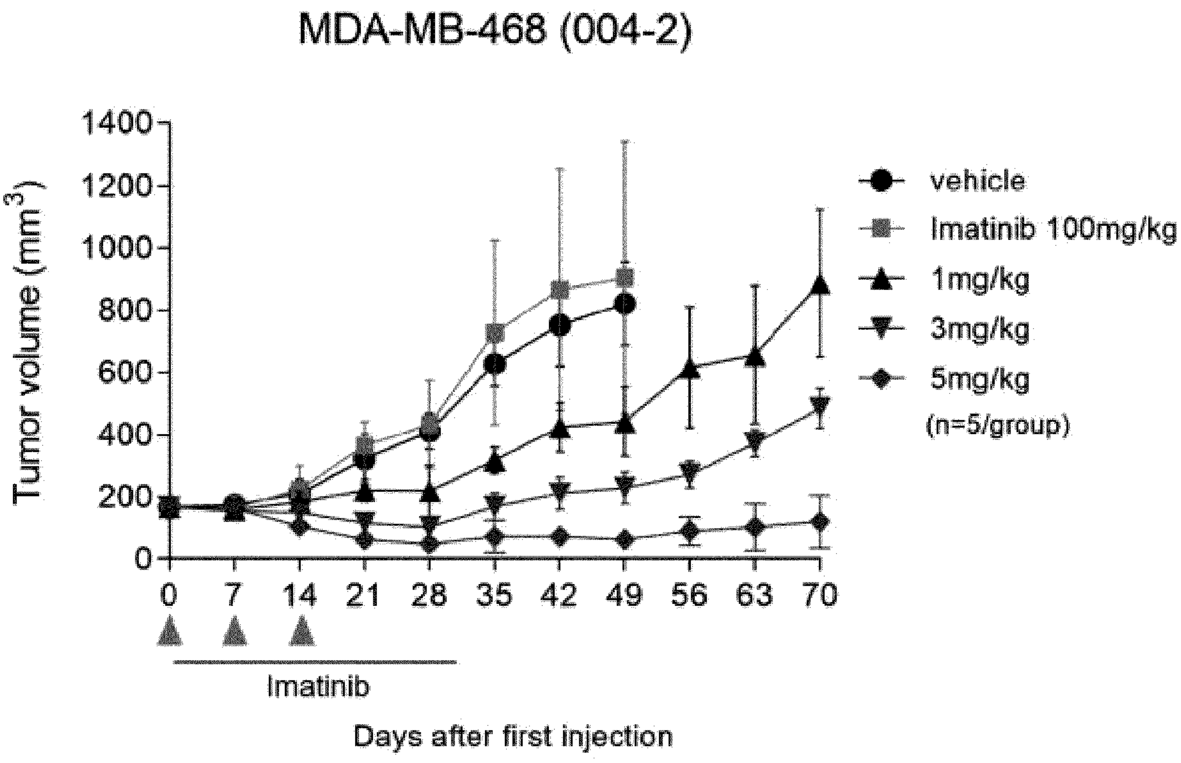
MDA-MB-468 (004-2)
Tumor volume ($mm^3$)
1400
1200
1000
800
600
400
200
0
0 7 14 21 28 35 42 49 56 63 70
vehicle
Imatinib 100mg/kg
1mg/kg
3mg/kg
5mg/kg
(n=5/group)
Imatinib
Days after first injection

Figure 9d

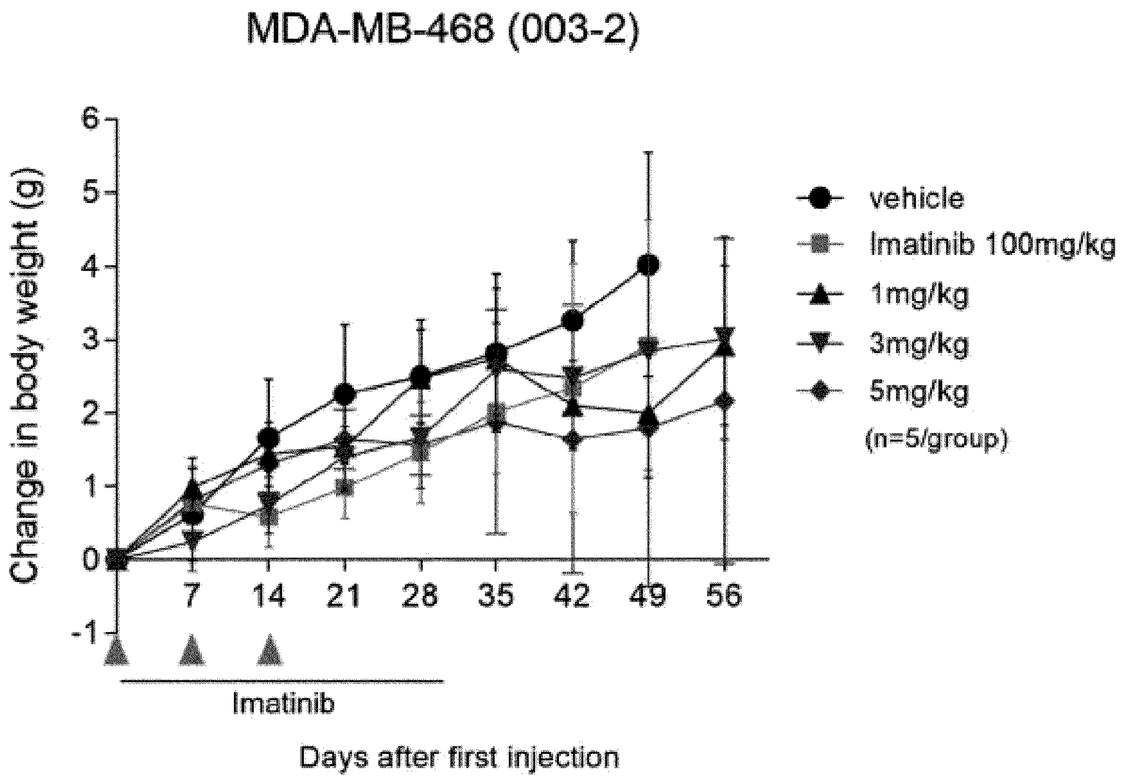
MDA-MB-468 (003-2)
Change in body weight (g)
6
5
4
3
2
1
0
-1
7
14
21
28
35
42
49
56
Imatinib
Days after first injection
vehicle
Imatinib 100mg/kg
1mg/kg
3mg/kg
5mg/kg
(n=5/group)

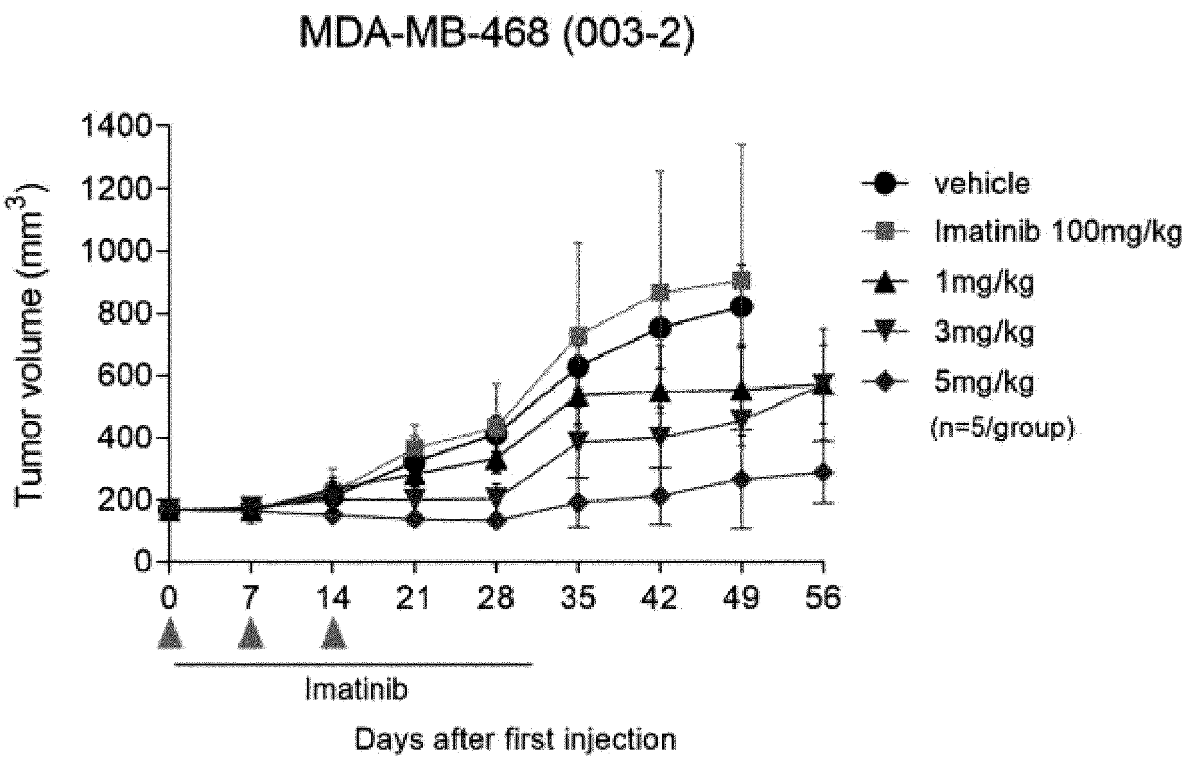
MDA-MB-468 (003-2)
Tumor volume (mm3)
1400
1200
1000
800
600
400
200
0
0
7
14
21
28
35
42
49
56
Imatinib
Days after first injection
vehicle
Imatinib 100mg/kg
1mg/kg
3mg/kg
5mg/kg
(n=5/group)

Figure 10

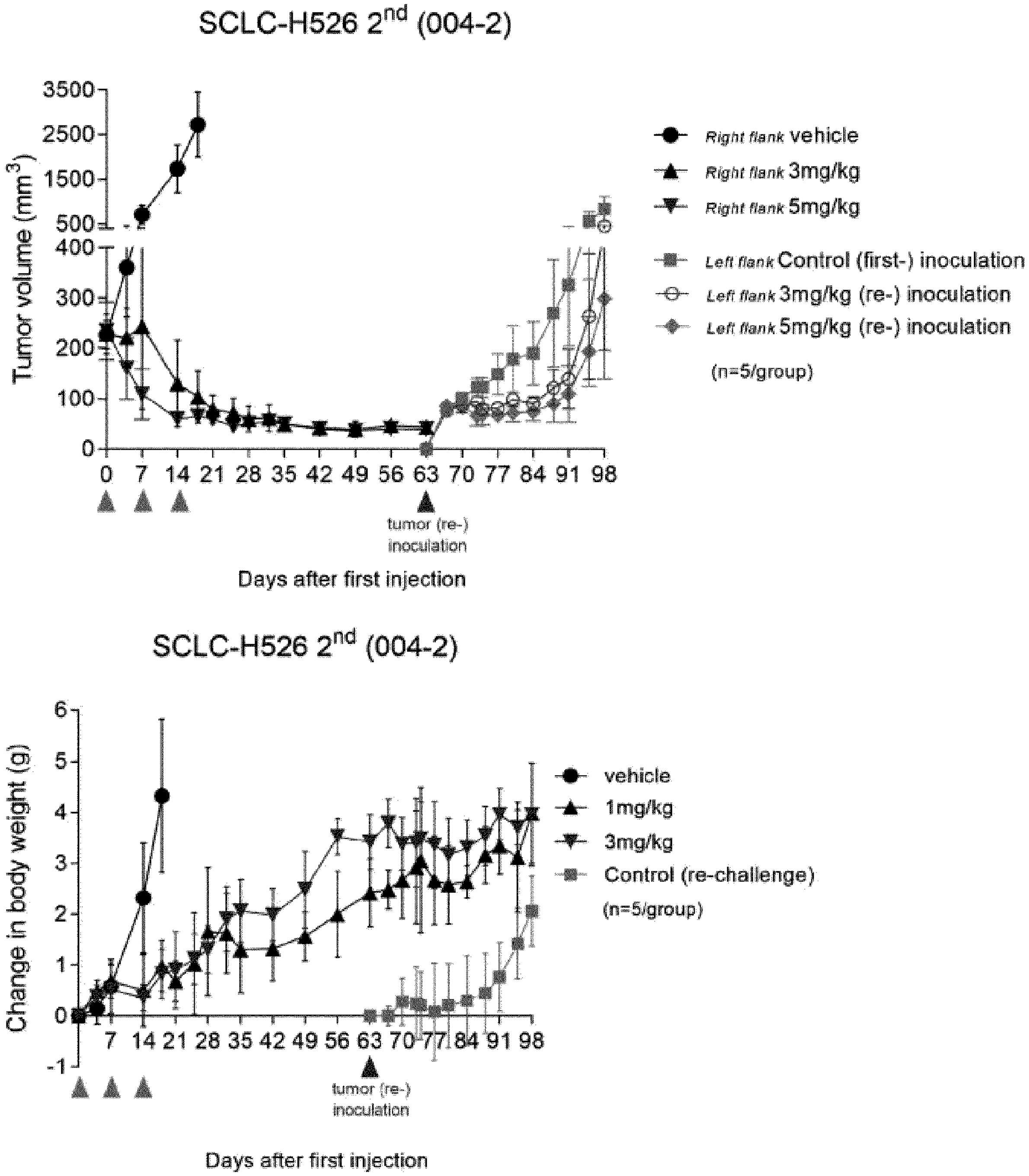
SCLC-H526 2nd (004-2)
Tumor volume (mm3)
3500
2500
1500
500
400
300
200
100
0
0 7 14 21 28 35 42 49 56 63 70 77 84 91 98
tumor (re-) inoculation
Days after first injection
Right flank vehicle
Right flank 3mg/kg
Right flank 5mg/kg
Left flank Control (first-) inoculation
Left flank 3mg/kg (re-) inoculation
Left flank 5mg/kg (re-) inoculation
(n=5/group)
SCLC-H526 2nd (004-2)
Change in body weight (g)
6
5
4
3
2
1
0
-1
7 14 21 28 35 42 49 56 63 70 77 84 91 98
tumor (re-) inoculation
Days after first injection
vehicle
1mg/kg
3mg/kg
Control (re-challenge)
(n=5/group)

Figure 11

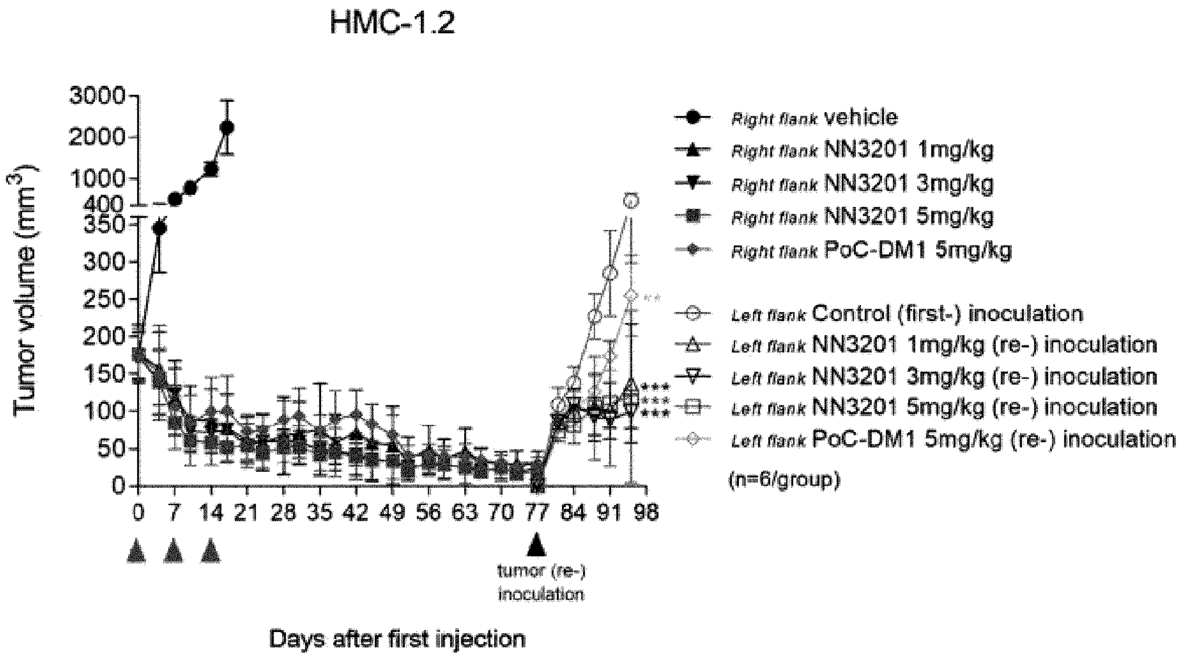
HMC-1.2
Tumor volume (mm$^3$)
3000
2000
1000
400
350
300
250
200
150
100
50
0
0 7 14 21 28 35 42 49 56 63 70 77 84 91 98
tumor (re-) inoculation
Days after first injection
Right flank vehicle
Right flank NN3201 1mg/kg
Right flank NN3201 3mg/kg
Right flank NN3201 5mg/kg
Right flank PoC-DM1 5mg/kg
Left flank Control (first-) inoculation
Left flank NN3201 1mg/kg (re-) inoculation
Left flank NN3201 3mg/kg (re-) inoculation
Left flank NN3201 5mg/kg (re-) inoculation
Left flank PoC-DM1 5mg/kg (re-) inoculation
(n=6/group)

Figure 12

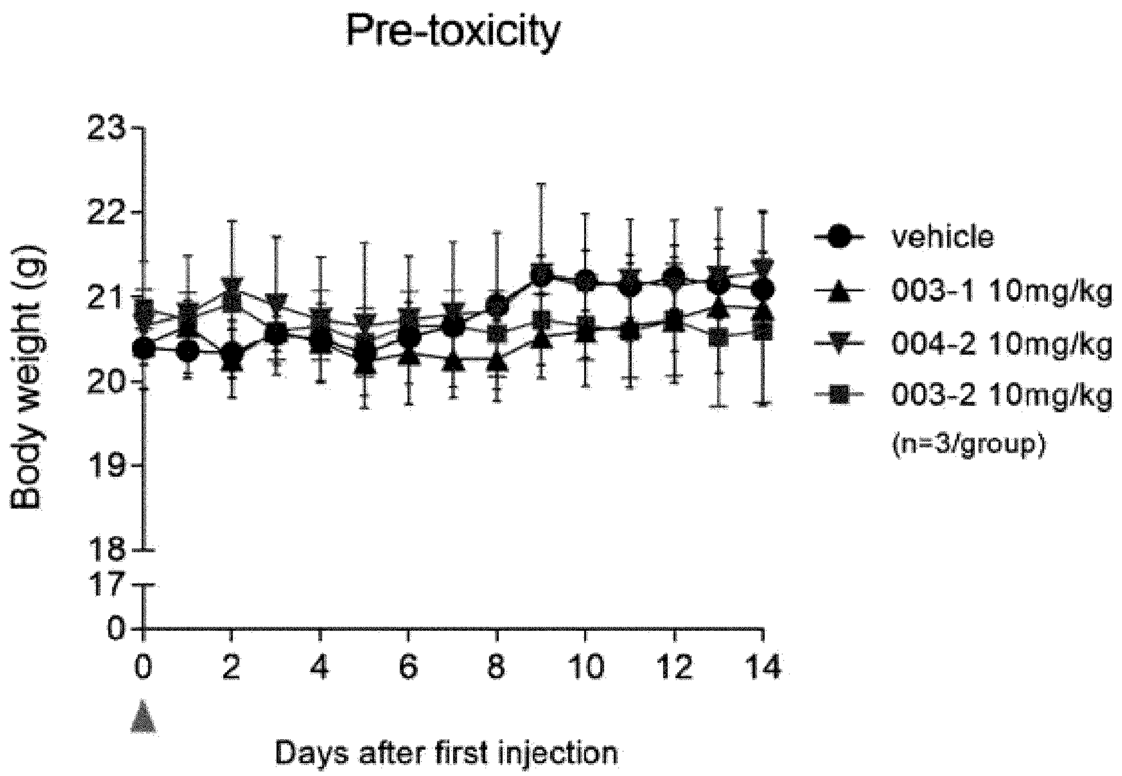
Pre-toxicity
Body weight (g)
23
22
21
20
19
18
17
0
0
2
4
6
8
10
12
14
Days after first injection
vehicle
003-1 10mg/kg
004-2 10mg/kg
003-2 10mg/kg
(n=3/group)

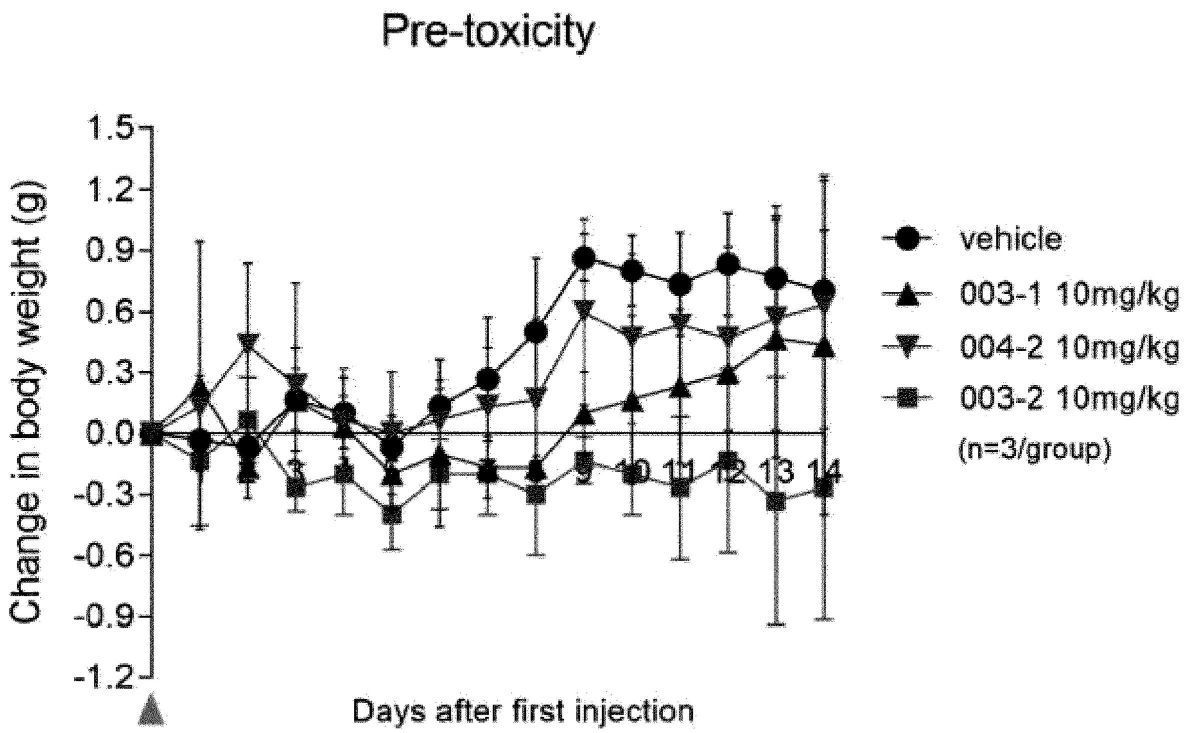
Pre-toxicity
Change in body weight (g)
1.5
1.2
0.9
0.6
0.3
0.0
-0.3
-0.6
-0.9
-1.2
Days after first injection
vehicle
003-1 10mg/kg
004-2 10mg/kg
003-2 10mg/kg
(n=3/group)

Figure 13a

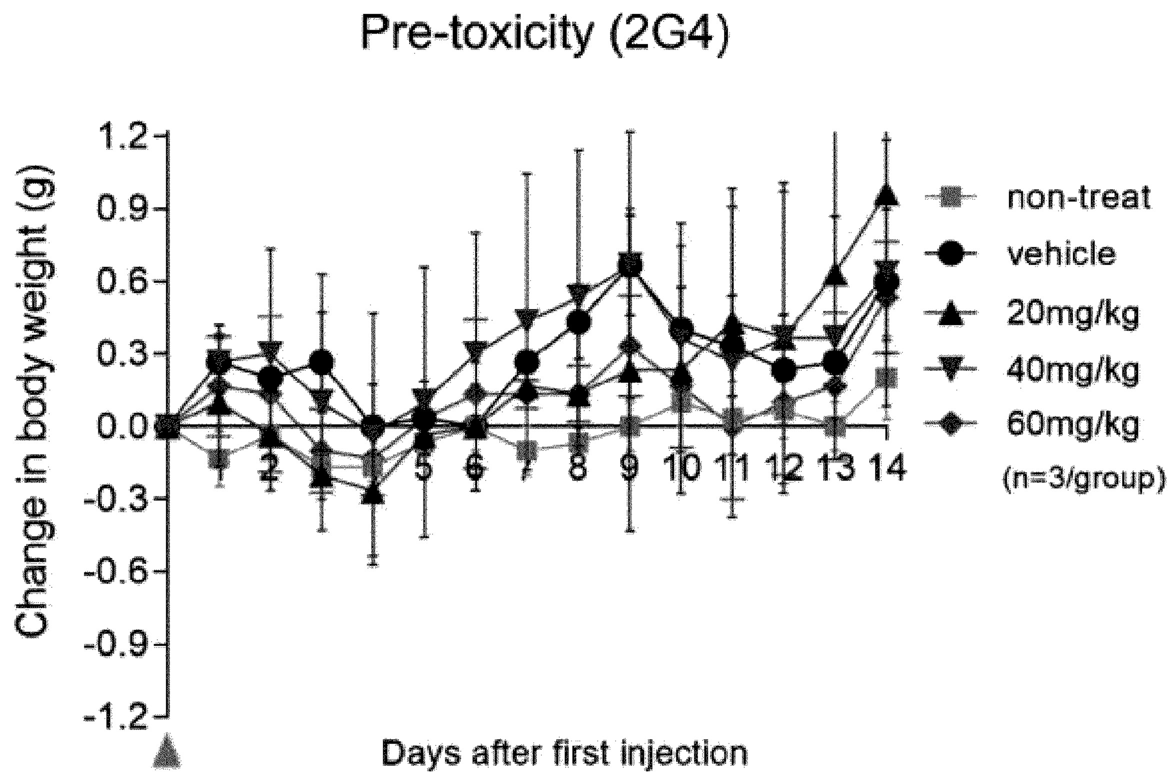


Figure 13b

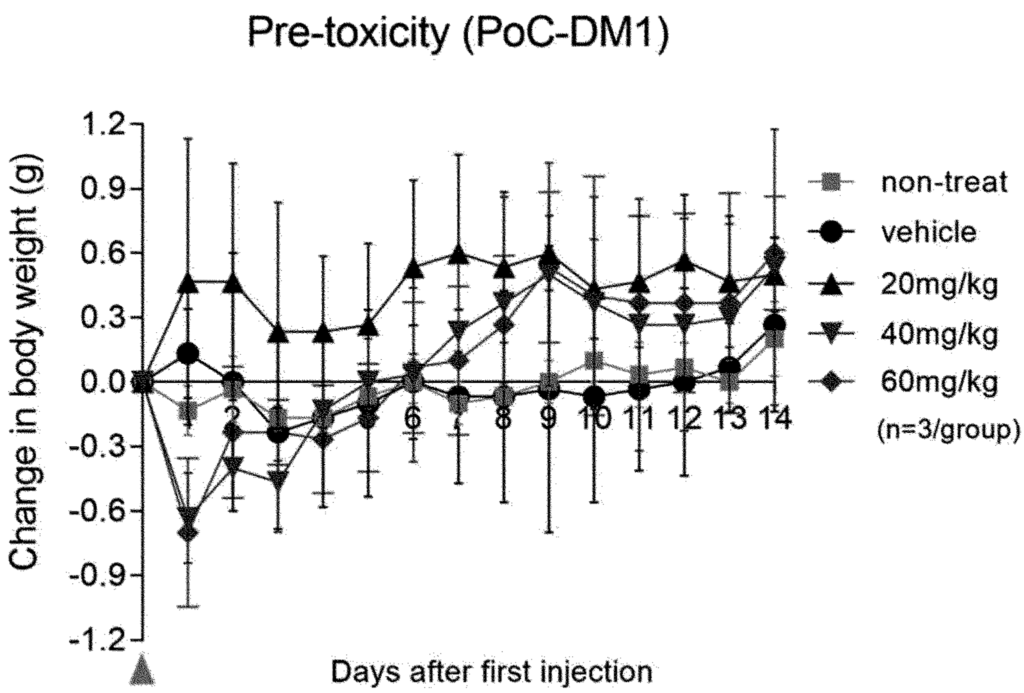

Figure 13c

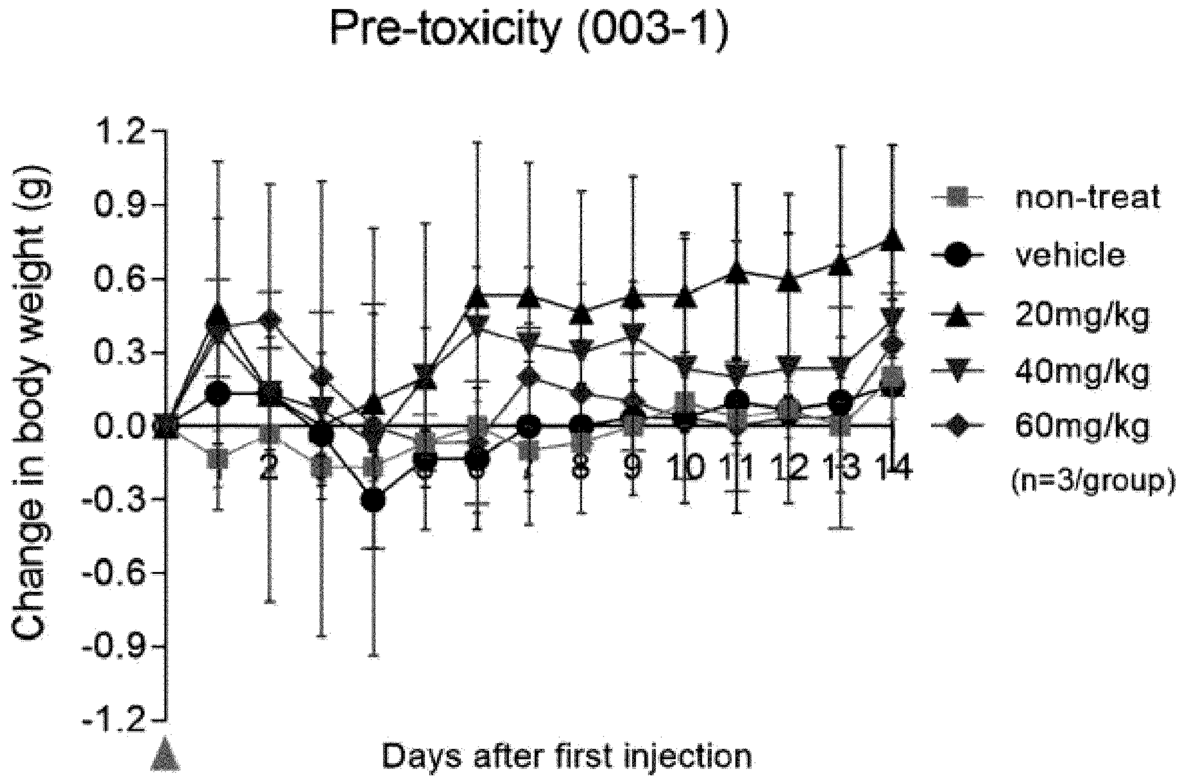


Figure 13d

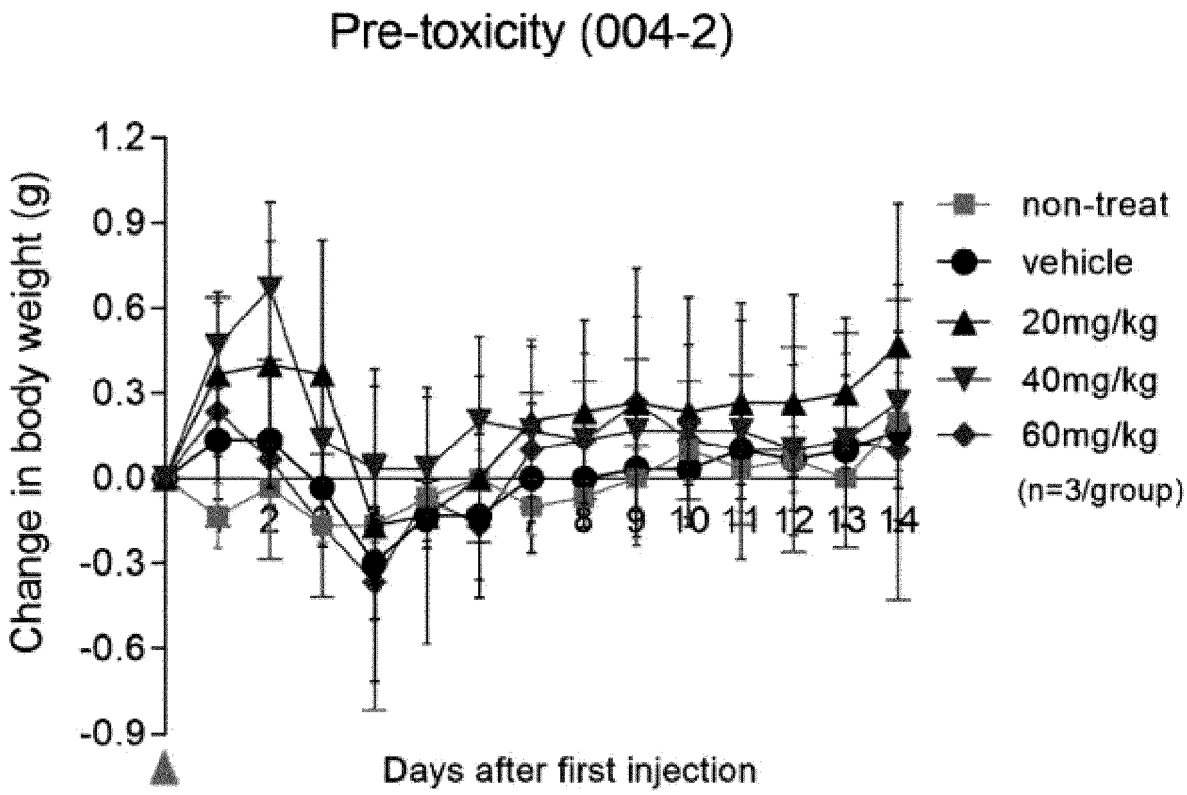

Figure 14a

| Name | Mutation | Domain |
|---|---|---|
| MT1 | R122A/Y125A | c-kit D2 |
| MT2 | R181A | |
| MT3 | K203A/R205A | |
| MT1/2 | R122A/Y125A/R181A | |
| MT1/2/3 | R122A/Y125A/R181A/K203A/R205A | |
| MT4 | S240A/S241A/Y243A | c-kit D3 |
| MT5 | N260A/W262A | |
| MT6 | S261A/H263A | |
| MT4/5 | S240A/S241A/Y243A/N260A/W262A | |
| MT4/6 | S240A/S241A/Y243A/S261A/H263A | |

Figure 14b

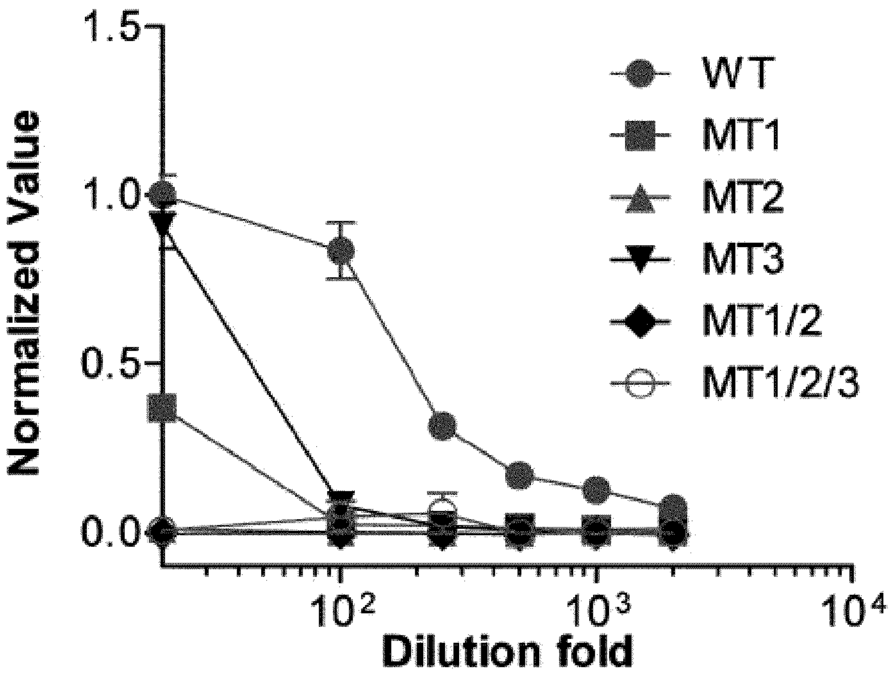

Figure 14c

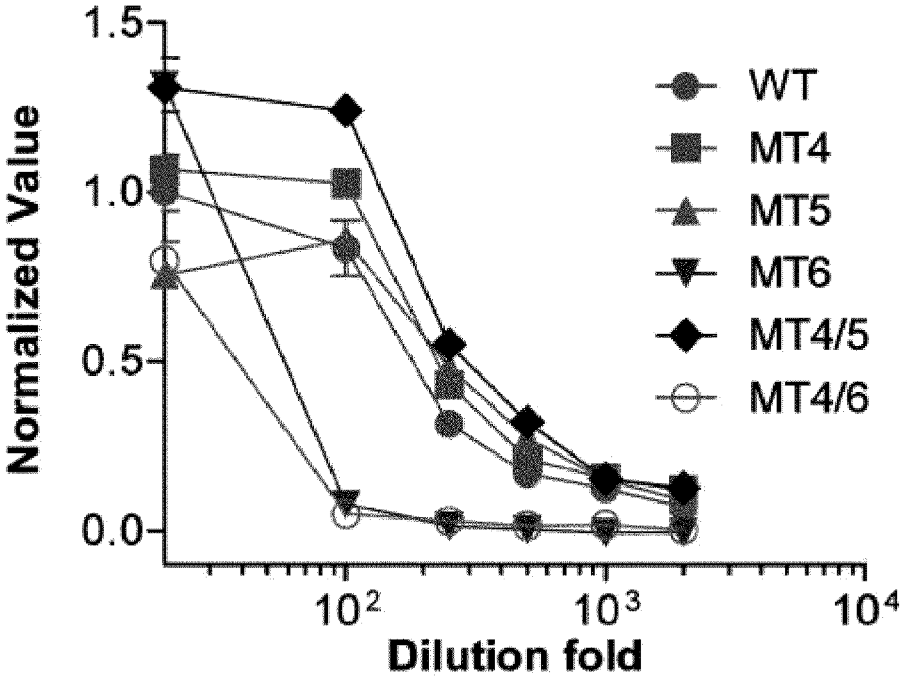


Figure 14d

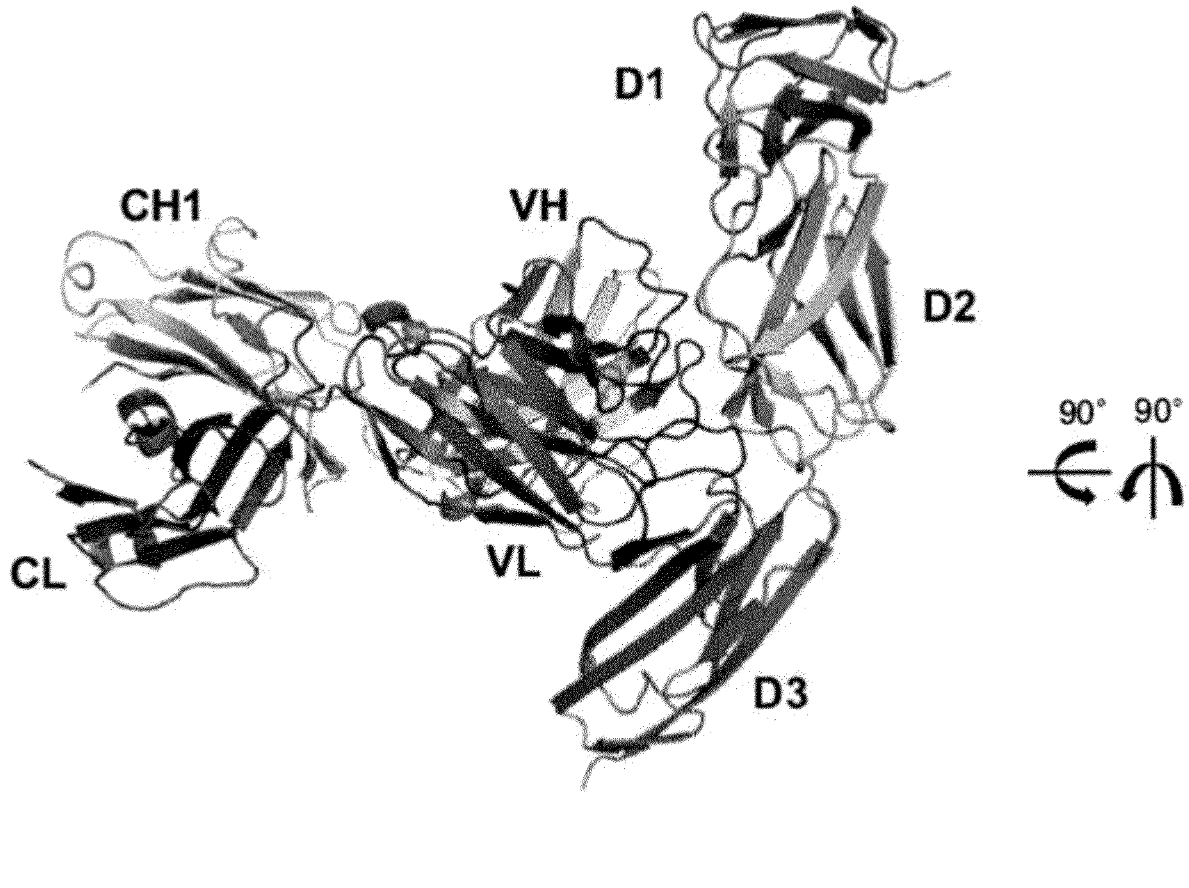

Figure 14e

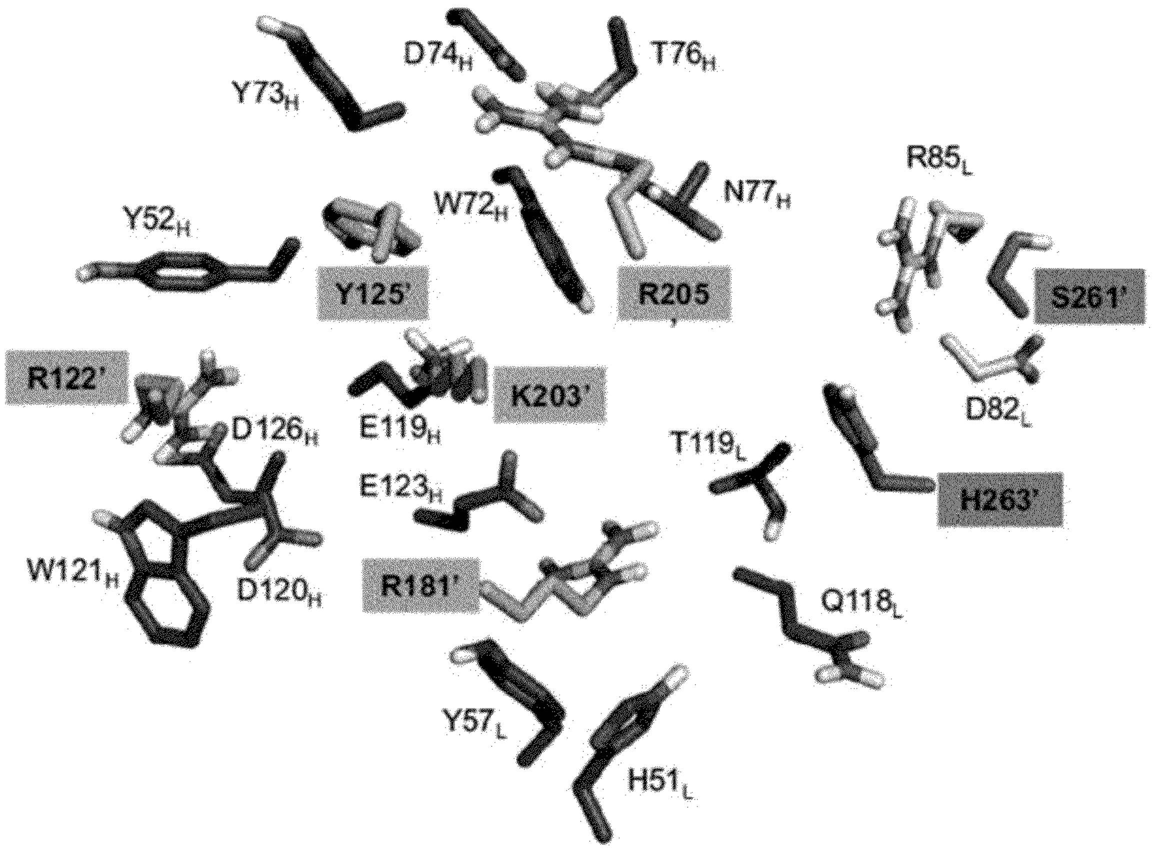
Y73H
D74H
T76H
R85L
N77H
W72H
Y52H
Y125'
R205
S261'
R122'
K203'
D82L
D126H
E119H
T119L
E123H
H263'
W121H
D120H
R181'
Q118L
Y57L
H51L

## INTERNATIONAL SEARCH REPORT

International application No.
**PCT/KR2022/002358**

**A. CLASSIFICATION OF SUBJECT MATTER**

**A61K 47/68**(2017.01)i; **A61K 38/05**(2006.01)i; **A61K 31/437**(2006.01)i; **A61P 35/00**(2006.01)i; **C07K 16/28**(2006.01)i

According to International Patent Classification (IPC) or to both national classification and IPC

**B. FIELDS SEARCHED**

Minimum documentation searched (classification system followed by classification symbols)

A61K 47/68(2017.01); A61K 31/537(2006.01); A61K 47/48(2006.01); A61K 47/65(2017.01); A61K 47/69(2017.01); A61K 49/00(2006.01); A61P 27/02(2006.01); C07K 16/28(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus), Google & keywords: 항체 약물 접합체 (antibody-drug conjugate), c-Kit, 암 (cancer)

**C. DOCUMENTS CONSIDERED TO BE RELEVANT**

| Category* | Citation of document, with indication, where appropriate, of the relevant passages | Relevant to claim No. |
|---|---|---|
| X<br>Y | KR 10-2015-0131173 A (NOVARTIS AG) 24 November 2015 (2015-11-24)<br>See claims 1-50; and table 2. | 1,2,8-15,18-20,23,25<br>3-6,16,17,21 |
| Y | KR 10-2020-0040407 A (NOVELTY NOBILITY INC.) 20 April 2020 (2020-04-20)<br>See claims 1-13. | 3-6,22 |
| Y | EP 3695852 A1 (REMEGEN, LTD.) 19 August 2020 (2020-08-19)<br>See claims 1-16. | 16,17,21,22 |
| Y | CN 106466485 A (TONGYI PHARMACEUTICAL CAYMAN CO., LTD.) 01 March 2017 (2017-03-01)<br>See pages 9-11. | 16,17,21,22 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **17 November 2022** | **17 November 2022** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2019)

**INTERNATIONAL SEARCH REPORT**

International application No.
**PCT/KR2022/002358**

**C. DOCUMENTS CONSIDERED TO BE RELEVANT**

| Category* | Citation of document, with indication, where appropriate, of the relevant passages | Relevant to claim No. |
|---|---|---|
| A | ABRAMS, T. et al. Preclinical antitumor activity of a novel anti–c-KIT antibody–drug conjugate against mutant and wild-type c-KIT–positive solid tumors. Clinical cancer research. 2018, vol. 24, no. 17, pp. 429 7-4308.<br>See entire document. | 1-6,8-23,25 |

Form PCT/ISA/210 (second sheet) (July 2019)

**INTERNATIONAL SEARCH REPORT**

International application No.
**PCT/KR2022/002358**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   - a. ☑ forming part of the international application as filed:
     - ☑ in the form of an Annex C/ST.25 text file.
     - ☐ on paper or in the form of an image file.
   - b. ☐ furnished together with the international application under PCT Rule 13*ter*.1(a) for the purposes of international search only in the form of an Annex C/ST.25 text file.
   - c. ☐ furnished subsequent to the international filing date for the purposes of international search only:
     - ☐ in the form of an Annex C/ST.25 text file (Rule 13*ter*.1(a)).
     - ☐ on paper or in the form of an image file (Rule 13*ter*.1(b) and Administrative Instructions, Section 713).
2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.
3. Additional comments:

Form PCT/ISA/210 (continuation of first sheet) (July 2019)

**INTERNATIONAL SEARCH REPORT**

International application No.
**PCT/KR2022/002358**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **24**
because they relate to subject matter not required to be searched by this Authority, namely:

Claim 24 pertains to a method for treatment of the human body by surgery or therapy (PCT Article 17(2)(a)(i) and PCT Rule 39.1(iv)).

2. ☐ Claims Nos.:
because they relate to parts of the international application that do not comply with the prescribed requirements to such an extent that no meaningful international search can be carried out, specifically:

3. ☑ Claims Nos.: **7**
because they are dependent claims and are not drafted in accordance with the second and third sentences of Rule 6.4(a).

Form PCT/ISA/210 (continuation of first sheet) (July 2019)

**INTERNATIONAL SEARCH REPORT**
**Information on patent family members**

International application No.
**PCT/KR2022/002358**

| Patent document cited in search report | Publication date (day/month/year) | Patent family member(s) | Publication date (day/month/year) |
|---|---|---|---|
| KR 10-2015-0131173 A | 24 November 2015 | AR 095666 A1 | 04 November 2015 |
| | | AU 2014-235474 A1 | 27 August 2015 |
| | | AU 2014-235474 B2 | 25 May 2017 |
| | | BR 112015021134 A2 | 10 October 2017 |
| | | CA 2903772 A1 | 25 September 2014 |
| | | CL 2015002246 A1 | 04 December 2015 |
| | | CN 105007950 A | 28 October 2015 |
| | | CN 105007950 B | 15 January 2019 |
| | | CR 20150488 A | 29 January 2016 |
| | | CU 20150128 A7 | 30 May 2016 |
| | | EA 035253 B1 | 21 May 2020 |
| | | EA 201591801 A1 | 31 March 2016 |
| | | EP 2968590 A1 | 20 January 2016 |
| | | EP 2968590 B1 | 05 September 2018 |
| | | EP 3514178 A1 | 24 July 2019 |
| | | ES 2701051 T3 | 20 February 2019 |
| | | IL 241277 A | 30 November 2015 |
| | | JP 2016-512832 A | 09 May 2016 |
| | | JP 2018-123141 A | 09 August 2018 |
| | | JP 6333943 B2 | 30 May 2018 |
| | | JP 6578400 B2 | 18 September 2019 |
| | | MA 38396 A1 | 30 April 2018 |
| | | MA 38396 B1 | 31 May 2019 |
| | | MX 2015013203 A | 11 December 2015 |
| | | MX 366978 B | 01 August 2019 |
| | | NZ 710929 A | 23 February 2018 |
| | | PH 12015501902 A1 | 04 January 2016 |
| | | SG 11201506395 A | 29 September 2015 |
| | | TN 2015000396 A1 | 03 January 2017 |
| | | TW 201446806 A | 16 December 2014 |
| | | US 10117953 B2 | 06 November 2018 |
| | | US 2014-0271688 A1 | 18 September 2014 |
| | | US 2015-0320880 A1 | 12 November 2015 |
| | | US 2016-0030594 A1 | 04 February 2016 |
| | | US 2017-0035905 A1 | 09 February 2017 |
| | | US 2018-0125996 A1 | 10 May 2018 |
| | | US 2020-0129632 A1 | 30 April 2020 |
| | | US 9498543 B2 | 22 November 2016 |
| | | US 9789203 B2 | 17 October 2017 |
| | | UY 35490 A | 31 October 2014 |
| | | WO 2014-150937 A1 | 25 September 2014 |
| | | ZA 201505656 B | 30 November 2016 |
| KR 10-2020-0040407 A | 20 April 2020 | AU 2019-358647 A1 | 03 June 2021 |
| | | BR 112021006924 A2 | 20 July 2021 |
| | | CA 3116154 A1 | 16 April 2020 |
| | | CN 113166256 A | 23 July 2021 |
| | | EA 202190983 A1 | 23 July 2021 |
| | | EP 3865512 A1 | 18 August 2021 |
| | | JP 2022-508747 A | 19 January 2022 |
| | | US 2021-0355212 A1 | 18 November 2021 |

Form PCT/ISA/210 (patent family annex) (July 2019)

INTERNATIONAL SEARCH REPORT
Information on patent family members

International application No.
PCT/KR2022/002358

| Patent document cited in search report | Publication date (day/month/year) | Patent family member(s) | Publication date (day/month/year) |
|---|---|---|---|
| | | WO 2020-076105 A1 | 16 April 2020 |
| EP 3695852 A1 | 19 August 2020 | AU 2019-268215 A1 | 19 December 2019 |
| | | AU 2019-268215 B2 | 18 March 2021 |
| | | AU 2019-272250 A1 | 19 March 2020 |
| | | AU 2019-272250 B2 | 13 January 2022 |
| | | BR 112020005596 A2 | 29 September 2020 |
| | | BR 112020014380 A2 | 29 December 2020 |
| | | CA 3062265 A1 | 21 November 2019 |
| | | CA 3062265 C | 22 March 2022 |
| | | CA 3082160 A1 | 28 November 2019 |
| | | CN 110507824 A | 29 November 2019 |
| | | CN 110740753 A | 31 January 2020 |
| | | CN 110740754 A | 31 January 2020 |
| | | EP 3797795 A1 | 31 March 2021 |
| | | JP 2020-533398 A | 19 November 2020 |
| | | JP 2021-523874 A | 09 September 2021 |
| | | JP 6949206 B2 | 13 October 2021 |
| | | KR 10-2020-0098626 A | 20 August 2020 |
| | | KR 10-2020-0120728 A | 21 October 2020 |
| | | KR 10-2454638 B1 | 14 October 2022 |
| | | RU 2724436 C1 | 23 June 2020 |
| | | RU 2747995 C1 | 18 May 2021 |
| | | SG 11202006515 A | 28 August 2020 |
| | | SG 11202011159 A | 30 December 2020 |
| | | TW 202003040 A | 16 January 2020 |
| | | TW I714092 B | 21 December 2020 |
| | | US 2020-0138968 A1 | 07 May 2020 |
| | | US 2022-0056147 A1 | 24 February 2022 |
| | | WO 2019-223579 A1 | 28 November 2019 |
| | | WO 2019-223653 A1 | 28 November 2019 |
| CN 106466485 A | 01 March 2017 | AU 2016-305703 A1 | 23 November 2017 |
| | | AU 2016-305703 B2 | 25 July 2019 |
| | | AU 2019-240611 A1 | 17 October 2019 |
| | | AU 2019-240611 B2 | 09 September 2021 |
| | | CA 2987322 A1 | 16 February 2017 |
| | | CA 2987322 C | 11 February 2020 |
| | | CN 106466484 A | 01 March 2017 |
| | | CN 106466484 B | 04 May 2021 |
| | | CN 106466485 B | 04 May 2021 |
| | | CN 108135881 A | 08 June 2018 |
| | | CN 108135881 B | 13 November 2020 |
| | | CN 111617250 A | 04 September 2020 |
| | | CN 112263683 A | 26 January 2021 |
| | | DK 3334500 T3 | 21 June 2021 |
| | | EP 3334500 A1 | 20 June 2018 |
| | | EP 3334500 B1 | 07 April 2021 |
| | | ES 2877409 T3 | 16 November 2021 |
| | | HK 1250634 A1 | 11 January 2019 |
| | | JP 2018-529632 A | 11 October 2018 |
| | | JP 2021-006549 A | 21 January 2021 |

Form PCT/ISA/210 (patent family annex) (July 2019)

**INTERNATIONAL SEARCH REPORT**
**Information on patent family members**

International application No.
**PCT/KR2022/002358**

| Patent document cited in search report | Publication date (day/month/year) | Patent family member(s) | Publication date (day/month/year) |
|---|---|---|---|
| | | JP 6772199 B2 | 21 October 2020 |
| | | KR 10-2018-0033513 A | 03 April 2018 |
| | | KR 10-2020-0071152 A | 18 June 2020 |
| | | KR 10-2301596 B1 | 14 September 2021 |
| | | MX 2018001723 A | 17 May 2018 |
| | | RU 2018104266 A | 12 September 2019 |
| | | RU 2018104266 A3 | 12 September 2019 |
| | | RU 2722449 C2 | 01 June 2020 |
| | | US 2018-0200377 A1 | 19 July 2018 |
| | | WO 2017-025057 A1 | 16 February 2017 |
| | | ZA 201707464 B | 26 May 2021 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20090274713
- WO 0138318 A
- US 0302675 W
- US 5475092 A
- US 6340701 B
- US 6372738 B
- US 6436931 B
- US 20010036923 A1
- US 024290
- US 10116053 B
- WO 0149698 A
- US 20090304721 A
- US 6316652 B
- US 6274552 B
- US 6271359 B
- US 6253872 B
- US 6139865 A
- US 6131570 A
- US 6120751 A
- US 6071495 A
- US 6060082 A
- US 6048736 A
- US 6039975 A
- US 6004534 A
- US 5985307 A
- US 5972366 A
- US 5900252 A
- US 5840674 A
- US 5759542 A
- US 5709874 A
- US 7811572 B
- US 6411163 B
- US 7368565 B
- US 8163888 B
- US 20110003969
- US 20110166319
- US 20120253021
- US 20120259100


### Non-patent literature cited in the description


- **YASUDA A et al.** *Dig Dis Sci*, 2007, vol. 52, 2292-2300
- **SUN J** ; **PEDERSEN M** ; **RONNSTRAND L**. *J Biol Chem*, 2009, vol. 284, 11039-11047
- **LENNARTSSON J** ; **RONNSTRAND L**. *Physiol Rev*, 2012, vol. 92, 1619-1649
- **ABBASPOUR BABAEI M et al.** *Drug Des Devel Ther*, 2016, vol. 10, 2443-2459
- **KANTARJIAN HM et al.** *Blood*, 2012, vol. 119, 1123-1129
- **LEUNG D et al.** *Antibodies*, 2020, vol. 9, 2
- **HARMEN M. M.** ; **HAARD H. J.** *Appl Microbiol Biotechnol.*, 2007, vol. 77 (1), 13-22
- **WARD et al.** *Nature*, 1989, vol. 341, 544-546
- **BIRD et al.** *Science*, 1988, vol. 242, 423-426
- **HUSTON et al.** *Proc. Natl. Acad. Sci. USA*, 1988, vol. 85, 5879-5883
- **ROUX et al.** *J. Immunol.*, 1998, vol. 161, 4083
- **KABAT EA et al.** Sequences of Proteins of Immunological Interest. U.S. Department of Health and Human Services, 1991
- **VIDARSSON G. et al.** *Front Immunol.*, 20 October 2014, vol. 5, 520
- **JEFFERIS et al.** *mAbs*, 2009, vol. 1, 1
- **LEFRANC MP**. *mAbs*, 2009, vol. 1 (4), 1-7
- Epitope Mapping Protocols in Methods in Molecular Biology. 1996, vol. 66
- Cold Spring Harb Protoc. 2006
- Using Antibodies. Cold Spring Harbor Laboratory Press, 1999
- **STAHLI et al.** *Methods in Enzymology*, 1983, vol. 9, 242
- **KIRKLAND et al.** *J. Immunol.*, 1986, vol. 137, 3614
- **HARLOW** ; **LANE**. Antibodies: A Laboratory Manual. Cold Spring Harbor Press, 1988
- **MOREL et al.** *Mol. Immunol.*, 1988, vol. 25 (1), 7
- **CHEUNG et al.** *Virology*, 1990, vol. 176, 546
- **MOLDENHAUER et al.** *Scand. J. Immunol.*, 1990, vol. 32, 77
- **J.-O. KIM et al.** *International Journal of Biological Macromolecules*, 2020, vol. 159, 66-78
- **LONBERG**. *Nature Biotech.*, 2005, vol. 23 (9), 1117-1125
- **BRUMMELL et al.** *Biochem.*, 1993, vol. 32, 1180-1187
- **KOBAYASHI et al.** *Protein Eng.*, 1999, vol. 12 (10), 879-884
- **BURKS et al.** *Proc. Natl. Acad. Sci. USA*, 1997, vol. 94, 412-417

- **NEEDLEMAN** ; **WUNSCH**. *J. Mol. Biol.*, 1970, vol. 48, 444-453
- **ALTSCHUL et al.** *J. Mol. Biol.*, 1990, vol. 215, 403-10
- **ALTSCHUL et al.** *Nucleic Acids Res.*, 1997, vol. 25 (17), 3389-3402
- **SASSE et al.** *J. Antibiot.*, 2000, vol. 53, 879-85
- **SUZAWA et al.** *Bioorg. Med. Chem.,*, 2000, vol. 8, 2175-84
- **ICHIMURA et al.** *J. Antibiot.*, 1991, vol. 44, 1045-53
- **FRANCISCO et al.** *Blood*, 2003, vol. 102 (4), 1458-65
- **SAITO et al.** *Adv. Drug Deliv. Rev.*, 2003, vol. 55, 199-215
- **TRAIL et al.** *Cancer Immunol. Immunother.*, 2003, vol. 52, 328-337
- **PAYNE**. *Cancer Cell*, 2003, vol. 3, 207-212
- **ALLEN**. *Nat. Rev. Cancer*, 2002, vol. 2, 750-763
- **PASTAN** ; **KREITMAN**. *Curr. Opin. Investig. Drugs*, 2002, vol. 3, 1089-1091
- **SENTER** ; **SPRINGER**. *Adv. Drug Deliv. Rev.*, 2001, vol. 53, 247-264
- **DENARDO et al.** *Clin Cancer Res.*, 1998, vol. 4 (10), 2483-90
- **PETERSON et al.** *Bioconjug. Chem.*, 1999, vol. 10 (4), 553-7
- **ZIMMERMAN et al.** *Nucl. Med. Biol.*, 1999, vol. 26 (8), 943-50
- Remington’s Pharmaceutical Sciences. Mack Publishing Co., 1990